(19) Europäisches Patentamt
European Patent Office
Office européen des brevets

(11) EP 4 263 558 B1

(12) **EUROPEAN PATENT SPECIFICATION**

(45) Date of publication and mention of the grant of the patent:
**12.02.2025 Bulletin 2025/07**

(21) Application number: **21830695.9**

(22) Date of filing: **16.12.2021**

(51) International Patent Classification (IPC):
***C07D 513/20*** $^{(2006.01)}$ ***C07D 513/22*** $^{(2006.01)}$
***A61P 35/00*** $^{(2006.01)}$ ***A61K 31/554*** $^{(2006.01)}$

(52) Cooperative Patent Classification (CPC):
**C07D 513/22; A61P 35/00; C07D 513/20**

(86) International application number:
**PCT/EP2021/086192**

(87) International publication number:
**WO 2022/129331 (23.06.2022 Gazette 2022/25)**

# (54) MACROCYCLIC BRANCHED 3-FLUORO-BUT-3-ENAMIDES AS INHIBITORS OF MCL-1

SPIROCYCLISCHE MAKROCYCLISCHE VERBINDUNGEN ALS MCL-1-INHIBITOREN

COMPOSÉS SPIROCYCLIQUES MACROCYCLIQUES EN TANT QU'INHIBITEURS DE MCL-1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.12.2020 EP 20214807**

(43) Date of publication of application:
**25.10.2023 Bulletin 2023/43**

(73) Proprietor: **JANSSEN Pharmaceutica NV**
**2340 Beerse (BE)**

(72) Inventors:
- **HSIAO, Meng-Yang**
**2340 Beerse (BE)**
- **JERHAOUI, Soufyan**
**2340 Beerse (BE)**
- **ROMBOUTS, Frederik, Jan, Rita**
**2340 Beerse (BE)**
- **SURKYN, Michel**
**2340 Beerse (BE)**
- **JOUFFROY, Matthieu, Dominique**
**2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2019/036575 WO-A1-2019/046150**
**WO-A1-2021/005043**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 263 558 B1

## Description

FIELD OF THE INVENTION

The present invention relates to pharmaceutical agents useful for therapy and/or prophylaxis in a subject, pharmaceutical composition comprising such compounds, and their use as MCL-1 inhibitors, useful for treating or preventing diseases such as cancer.

BACKGROUND OF THE INVENTION

Cellular apoptosis or programmed cell death is critical to the development and homeostasis of many organs including the hematopoietic system. Apoptosis can be initiated via the extrinsic pathway, which is mediated by death receptors, or by the intrinsic pathway using the B cell lymphoma (BCL-2) family of proteins. Myeloid cell leukemia-1 (MCL-1) is a member of the BCL-2 family of cell survival regulators and is a critical mediator of the intrinsic apoptosis pathway. MCL-1 is one of five principal anti-apoptotic BCL-2 proteins (MCL-1, BCL-2, BCL-XL, BCL-w, and BFL1/A1) responsible for maintaining cell survival. MCL-1 continuously and directly represses the activity of the pro-apoptotic BCL-2 family proteins Bak and Bax and indirectly blocks apoptosis by sequestering BH3 only apoptotic sensitizer proteins such as Bim and Noxa. The activation of Bak/Bax following various types of cellular stress leads to aggregation on the mitochondrial outer membrane and this aggregation facilitates pore formation, loss of mitochondrial outer membrane potential, and subsequent release of cytochrome C into the cytosol. Cytosolic cytochrome C binds Apaf-1 and initiates recruitment of procaspase 9 to form apoptosome structures (Cheng et al. eLife 2016; 5: e17755). The assembly of apoptosomes activates the executioner cysteine proteases 3/7 and these effector caspases then cleave a variety of cytoplasmic and nuclear proteins to induce cell death (Julian et al. Cell Death and Differentiation 2017; 24, 1380-1389).

Avoiding apoptosis is an established hallmark of cancer development and facilitates the survival of tumor cells that would otherwise be eliminated due to oncogenic stresses, growth factor deprivation, or DNA damage (Hanahan and Weinberg. Cell 2011;1-44). Thus, unsurprisingly, MCL-1 is highly upregulated in many solid and hematologic cancers relative to normal non-transformed tissue counterparts. The overexpression of MCL-1 has been implicated in the pathogenesis of several cancers where it correlated with poor outcome, relapse, and aggressive disease. Additionally, overexpression of MCL-1 has been implicated in the pathogenesis of the following cancers: prostate, lung, pancreatic, breast, ovarian, cervical, melanoma, B-cell chronic lymphocytic leukemia (CLL), acute myeloid leukemia (AML), and acute lymphoblastic leukemia (ALL). The human MCL-1 genetic locus (1q21) is frequently amplified in tumors and quantitatively increases total MCL-1 protein levels (Beroukhim et al. Nature 2010;463 (7283) 899-905). MCL-1 also mediates resistance to conventional cancer therapeutics and is transcriptionally upregulated in response to inhibition of BCL-2 function (Yecies et al. Blood 2010;115 (16)3304-3313).

A small molecule BH3 inhibitor of BCL-2 has demonstrated clinical efficacy in patients with chronic lymphocytic leukemia and is FDA approved for patients with CLL or AML (Roberts et al. NEJM 2016;374:311-322). The clinical success of BCL-2 antagonism led to the development of several MCL-1 BH3 mimetics that show efficacy in preclinical models of both hematologic malignancies and solid tumors (Kotschy et al. Nature 2016;538 477-486, Merino et al. Sci. Transl. Med;2017 (9)).

MCL-1 regulates several cellular processes in addition to its canonical role in mediating cell survival including mitochondrial integrity and non-homologous end joining following DNA damage (Chen et al. JCI 2018;128(1):500-516). The genetic loss of MCL-1 shows a range of phenotypes depending on the developmental timing and tissue deletion. MCL-1 knockout models reveal there are multiple roles for MCL-1 and loss of function impacts a wide range of phenotypes. Global MCL-1-deficient mice display embryonic lethality and studies using conditional genetic deletion have reported mitochondrial dysfunction, impaired activation of autophagy, reductions in B and T lymphocytes, increased B and T cell apoptosis, and the development of heart failure/ cardiomyopathy (Wang et al. Genes and Dev 2013;27 1351-1364, Steimer et al. Blood 2009;(113) 2805-2815).

WO2019046150 discloses macrocyclic compounds that inhibit mcl-1 protein.

WO2019173181 discloses alpha-hydroxy phenylacetic acid pharmacophore or bioisostere mcl-1 protein antagonists.WO2016033486 discloses tetrahydronaphthalene derivatives that inhibit mcl-1 protein.

WO2019036575, WO2017147410, and WO2018183418 disclose compounds that inhibit mcl-1 protein.

WO2019222112 discloses MCL-1 inhibitors for treating cancer.

WO2020097577 discloses spiro-sulfonamide derivatives as inhibitors of myeloid cell leukemia-1 (MCL-1) protein.

WO2021211922 discloses spiro-sulfonimidamide derivatives as inhibitors of myeloid cell leukemia-1 (mcl-1) protein.

There remains a need for MCL-1 inhibitors, useful for the treatment or prevention of cancers such as prostate, lung, pancreatic, breast, ovarian, cervical, melanoma, B-cell chronic lymphocytic leukemia (CLL), acute myeloid leukemia

(AML), and acute lymphoblastic leukemia (ALL).

SUMMARY OF THE INVENTION

The present invention concerns compounds of Formula (I):

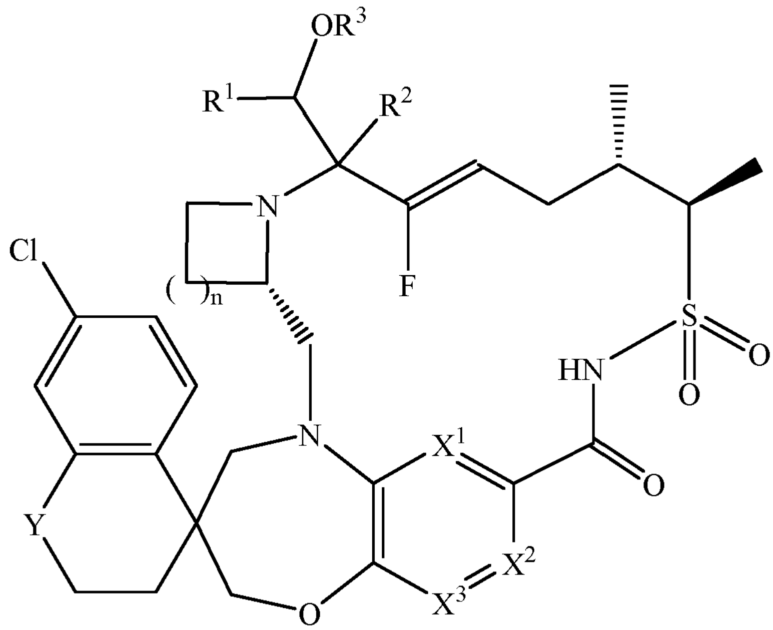

(I)

wherein

$R^1$ represents $C_{1-4}$alkyl, $-C_{1-4}$alkyl-$NR^{4a}R^{4b}$, or $-C_{1-4}$alkyl-$OR^5$;

$R^2$ represents hydrogen, methyl, $-CH_2-NR^{4c}R^{4d}$, or $-CH_2-OR^6$; and

$R^3$ represents hydrogen, $C_{1-4}$alkyl, or $-C_{2-4}$alkyl-O-$C_{1-4}$alkyl;
or

$R^1$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and

$R^2$ represents hydrogen, methyl, $-CH_2-NR^{4c}R^{4d}$, or $-CH_2-OR^6$;
or

$R^2$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and

$R^1$ represents hydrogen, $C_{1-4}$alkyl, $-C_{1-4}$alkyl-$NR^{4a}R^{4b}$, or $-C_{1-4}$alkyl-$OR^5$;
or

$R^1$ and $R^2$ are taken together to form together with the atoms to which they are attached a 4- to 7-membered

monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and

$R^3$ represents hydrogen, $C_{1-4}$alkyl, or -$C_{2-4}$alkyl-O-$C_{1-4}$alkyl;

$R^{4a}$ and $R^{4b}$ are each independently selected from the group consisting of $C_{1-4}$alkyl, -$C_{1-4}$alkyl-Het$^{1b}$, -$C_{2-4}$alkyl-O-$C_{1-4}$alkyl, and -$C_{2-4}$alkyl-O-$C_{1-4}$alkyl-O-$C_{1-4}$alkyl;

or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^{4c}$ and $R^{4d}$ are each independently selected from the group consisting of $C_{1-4}$alkyl and -$C_{2-4}$alkyl-O-$C_{1-4}$alkyl;

or $R^{4c}$ and $R^{4d}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4c}$ and $R^{4d}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^5$ represents hydrogen, $C_{1-4}$alkyl, Het$^{1a}$, -$C_{2-4}$alkyl-NR$^{7a}$R$^{7b}$, -$C_{1-4}$alkyl-Het$^{1b}$, or $C_{1-4}$alkyl substituted with one or two -O-$C_{1-4}$alkyl;

$R^{7a}$ and $R^{7b}$ are each independently selected from the group consisting of hydrogen and $C_{1-4}$alkyl;

Het$^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

Het$^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

The present invention also relates to a pharmaceutical composition comprising a therapeutically effective amount of a compound of Formula (I), a pharmaceutically acceptable salt, or a solvate thereof, and a pharmaceutically acceptable carrier or excipient.

Additionally, the invention relates to a compound of Formula (I), a pharmaceutically acceptable salt, or a solvate

thereof, for use as a medicament, and to a compound of Formula (I), a pharmaceutically acceptable salt, or a solvate thereof, for use in the treatment or in the prevention of cancer.

In a particular embodiment, the invention relates to a compound of Formula (I), a pharmaceutically acceptable salt, or a solvate thereof, for use in the treatment or in the prevention of cancer.

Disclosed herein is the use of a compound of Formula (I), a pharmaceutically acceptable salt, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable salt, or a solvate thereof.

Disclosed herein is a product comprising a compound of Formula (I), a pharmaceutically acceptable salt, or a solvate thereof, and an additional pharmaceutical agent, as a combined preparation for simultaneous, separate or sequential use in the treatment or prevention of cancer.

DETAILED DESCRIPTION OF THE INVENTION

The term 'halo' or 'halogen' as used herein represents fluoro, chloro, bromo and iodo.

The prefix '$C_{x-y}$' (where x and y are integers) as used herein refers to the number of carbon atoms in a given group. Thus, a $C_{1-4}$alkyl group contains from 1 to 4 carbon atoms, and so on.

The term '$C_{1-4}$alkyl' as used herein as a group or part of a group represents a straight or branched chain fully saturated hydrocarbon radical having from 1 to 4 carbon atoms, such as methyl, ethyl, n-propyl, isopropyl, n-butyl, s-butyl, t-butyl and the like.

It will be clear for the skilled person that $S(=O)_2$ or $SO_2$ represents a sulfonyl moiety.

It will be clear for the skilled person that CO or C(=O) represents a carbonyl moiety.

Non-limiting examples of two R groups taken together to form together with the N-atom to which they are attached a 4- to 7-membered monocyclic fully saturated heterocyclyl containing one N-atom and optionally one additional heteroatom selected from O, S, and N, include, but are not limited to N-linked azetidinyl, N-linked pyrrolidinyl, N-linked morpholinyl and N-linked piperidinyl.

Non-limiting examples of 4- to 7-membered monocyclic fully saturated heterocyclyl containing one or two heteroatoms each independently selected from O, S, and N, include, but are not limited to tetrahydropyranyl, morpholinyl and azetidinyl.

Non-limiting examples of 4- to 7-membered monocyclic fully saturated heterocyclyl containing one O-atom and optionally one additional heteroatom selected from O, S, and N, include, but are not limited to morpholinyl, tetrahydropyranyl, tetrahydrofuranyl, oxathianyl and oxepanyl.

Non-limiting examples of 5- to 6-membered monocyclic aromatic ring containing one N-atom and optionally one or two additional heteroatoms each independently selected from O, S, and N, include, but are not limited to pyrrolyl, pyridinyl, pyrimidinyl, thiazolyl, oxazolyl.

Unless otherwise specified or clear from the context, heterocyclyl goups (e.g. $Het^{1b}$) can be attached to the remainder of the molecule of Formula (I) through any available ring carbon atom (C-linked) or nitrogen atom (N-linked) if available.

In general, whenever the term 'substituted' is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture.

Combinations of substituents and/or variables are permissible only if such combinations result in chemically stable compounds. 'Stable compound' is meant to indicate a compound that is sufficiently robust to survive isolation to a useful degree of purity from a reaction mixture.

When two or more substituents are present on a moiety they may, where possible and unless otherwise indicated or clear from the context, replace hydrogens on the same atom or they may replace hydrogen atoms on different atoms in the moiety.

When any variable occurs more than one time in any constituent, each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, or subject (e.g., human) that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R*, *S*) around one or more atoms, contemplates each possible stereoisomer, or mixture of two or more stereoisomers. Where the stereochemistry of any particular chiral atom is not specified in the structures shown herein, then all stereoisomers are contemplated and included as the compounds of the invention, either as a pure stereoisomer or as a mixture of two or more stereoisomers.

Hereinbefore and hereinafter, the term "compound of Formula (I)" is meant to include the stereoisomers thereof and the tautomeric forms thereof. However where stereochemistry, as mentioned in the previous paragraph, is specified by bonds which are shown as solid wedged or hashed wedged bonds, or are otherwise indicated as having a particular configuration (e.g. *R*, *S*), or when the stereochemistry around a double bond is shown (e.g. in Formula (I)), then that stereoisomer is so specified and defined. It will be clear this also applies to subgroups of Formula (I).

It follows that a single compound may, where possible, exist in both stereoisomeric and tautomeric form.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Diastereomers (or diastereoisomers) are stereoisomers that are not enantiomers, i.e. they are not related as mirror images. If a compound contains a double bond, the substituents may be in the *E* or the *Z* configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, diastereomers, racemates, *E* isomers, *Z* isomers, cis isomers, trans isomers and mixtures thereof, unless the context indicates otherwise and whenever chemically possible.

The meaning of all those terms, i.e. enantiomers, diastereomers, racemates, *E* isomers, *Z* isomers, cis isomers, trans isomers and mixtures thereof are known to the skilled person.

The absolute configuration is specified according to the Cahn-Ingold-Prelog system. The configuration at an asymmetric atom is specified by either *R* or *S*. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (*R*), this means that the compound is substantially free of the (*S*) isomer; when a compound of Formula (I) is for instance specified as *E*, this means that the compound is substantially free of the *Z* isomer; when a compound of Formula (I) is for instance specified as cis, this means that the compound is substantially free of the trans isomer.

Pharmaceutically acceptable salts, in particular pharmaceutically acceptable additions salts, include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I), and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic,

hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, N-oxides and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$ , $^{13}N$, $^{15}O$, $^{17}O$, $^{18}O$, $^{32}P$, $^{33}P$, $^{35}S$, $^{18}F$, $^{36}Cl$, $^{122}I$, $^{123}I$, $^{125}I$, $^{131}I$, $^{75}Br$, $^{76}Br$, $^{77}Br$ and $^{82}Br$. Preferably, the isotope is selected from the group of $^{2}H$, $^{3}H$, $^{11}C$ and $^{18}F$. More preferably, the isotope is $^{2}H$. In particular, deuterated compounds are intended to be included within the scope of the present invention.

Certain isotopically-labeled compounds of the present invention (e.g., those labeled with $^{3}H$ and $^{14}C$) may be useful for example in substrate tissue distribution assays. Tritiated ($^{3}H$) and carbon-14 ($^{14}C$) isotopes are useful for their ease of preparation and detectability. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies. PET imaging in cancer finds utility in helping locate and identify tumours, stage the disease and determine suitable treatment. Human cancer cells overexpress many receptors or proteins that are potential diseasespecific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

In an embodiment, the present invention concerns novel compounds of Formula (I), wherein

$R^1$ represents $C_{1-4}$alkyl, $-C_{1-4}$alkyl-$NR^{4a}R^{4b}$, or $-C_{1-4}$alkyl-$OR^5$;

$R^2$ represents hydrogen, methyl, $-CH_2-NR^{4c}R^{4d}$, or $-CH_2-OR^6$; and

$R^3$ represents hydrogen, $C_{1-4}$alkyl, or $-C_{2-4}$alkyl-O-$C_{1-4}$alkyl;

$R^{4a}$ and $R^{4b}$ are each independently selected from the group consisting of $C_{1-4}$alkyl, -$C_{1-4}$alkyl-Het$^{1b}$, -$C_{2-4}$alkyl-O-$C_{1-4}$alkyl, and -$C_{2-4}$alkyl-O-$C_{1-4}$alkyl-O-$C_{1-4}$alkyl;

or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^{4c}$ and $R^{4d}$ are each independently selected from the group consisting of $C_{1-4}$alkyl and -$C_{2-4}$alkyl-O-$C_{1-4}$alkyl;

or $R^{4c}$ and $R^{4d}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4c}$ and $R^{4d}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^5$ represents hydrogen, $C_{1-4}$alkyl, Het$^{1a}$, -$C_{2-4}$alkyl-NR$^{7a}$R$^{7b}$, -$C_{1-4}$alkyl-Het$^{1b}$, or $C_{1-4}$alkyl substituted with one or two -O-$C_{1-4}$alkyl;

$R^{7a}$ and $R^{7b}$ are each independently selected from the group consisting of hydrogen and $C_{1-4}$alkyl;

Het$^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

Het$^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and

$R^2$ represents hydrogen, methyl, -$CH_2$-NR$^{4c}$R$^{4d}$, or -$CH_2$-OR$^6$;

$R^{4a}$ and $R^{4b}$ are each independently selected from the group consisting of $C_{1-4}$alkyl, -$C_{1-4}$alkyl-Het$^{1b}$, -$C_{2-4}$alkyl-O-

$C_{1-4}$alkyl, and $-C_{2-4}$alkyl-O-$C_{1-4}$alkyl-O-$C_{1-4}$alkyl;

or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^{4c}$ and $R^{4d}$ are each independently selected from the group consisting of $C_{1-4}$alkyl and $-C_{2-4}$alkyl-O-$C_{1-4}$alkyl;

or $R^{4c}$ and $R^{4d}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4c}$ and $R^{4d}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^5$ represents hydrogen, $C_{1-4}$alkyl, $Het^{1a}$, $-C_{2-4}$alkyl-$NR^{7a}R^{7b}$, $-C_{1-4}$alkyl-$Het^{1b}$, or $C_{1-4}$alkyl substituted with one or two -O-$C_{1-4}$alkyl;

$R^{7a}$ and $R^{7b}$ are each independently selected from the group consisting of hydrogen and $C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^2$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and

$R^1$ represents hydrogen, $C_{1-4}$alkyl, $-C_{1-4}$alkyl-$NR^{4a}R^{4b}$, or $-C_{1-4}$alkyl-$OR^5$;

$R^{4a}$ and $R^{4b}$ are each independently selected from the group consisting of $C_{1-4}$alkyl, $-C_{1-4}$alkyl-$Het^{1b}$, $-C_{2-4}$alkyl-O-$C_{1-4}$alkyl, and $-C_{2-4}$alkyl-O-$C_{1-4}$alkyl-O-$C_{1-4}$alkyl;

or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^{4c}$ and $R^{4d}$ are each independently selected from the group consisting of $C_{1-4}$alkyl and -$C_{2-4}$alkyl-O-$C_{1-4}$alkyl;

or $R^{4c}$ and $R^{4d}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4c}$ and $R^{4d}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^5$ represents hydrogen, $C_{1-4}$alkyl, $Het^{1a}$, -$C_{2-4}$alkyl-$NR^{7a}R^{7b}$, -$C_{1-4}$alkyl-$Het^{1b}$, or $C_{1-4}$alkyl substituted with one or two -O-$C_{1-4}$alkyl;

$R^{7a}$ and $R^{7b}$ are each independently selected from the group consisting of hydrogen and $C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ and $R^2$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and
$R^3$ represents hydrogen, $C_{1-4}$alkyl, or -$C_{2-4}$alkyl-O-$C_{1-4}$alkyl;
$R^{4a}$ and $R^{4b}$ are each independently selected from the group consisting of $C_{1-4}$alkyl, -$C_{1-4}$alkyl-$Het^{1b}$, -$C_{2-4}$alkyl-O-$C_{1-4}$alkyl, and -$C_{2-4}$alkyl-O-$C_{1-4}$alkyl-O-$C_{1-4}$alkyl;
or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;
or $R^{4a}$ and $R^{4b}$ are taken together to form together with the N-atom to which they are attached a 5- to 6-membered

monocyclic aromatic ring containing one N-atom and optionally one or two additional heteroatoms each independently selected from O, S, and N, wherein said S-atom might be substituted to form S(=O) or $S(=O)_2$;
$R^{4c}$ and $R^{4d}$ are each independently selected from the group consisting of $C_{1-4}$alkyl and -$C_{2-4}$alkyl-O-$C_{1-4}$alkyl;
or $R^{4c}$ and $R^{4d}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;
or $R^{4c}$ and $R^{4d}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;
$R^5$ represents hydrogen, $C_{1-4}$alkyl, $Het^{1a}$, -$C_{2-4}$alkyl-$NR^{7a}R^{7b}$, -$C_{1-4}$alkyl-$Het^{1b}$, or $C_{1-4}$alkyl substituted with one or two -O-$C_{1-4}$alkyl;
$R^{7a}$ and $R^{7b}$ are each independently selected from the group consisting of hydrogen and $C_{1-4}$alkyl;
$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;
$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;
$R^6$ represents hydrogen or $C_{1-4}$alkyl;
n is 1 or 2;
Y represents O or $CH_2$;
$X^1$ represents CH;
$X^2$ represents CH;
$X^3$ represents CH;
and the pharmaceutically acceptable salts and the solvates thereof.

In an embodiment, the present invention concerns novel compounds of Formula (I), wherein

$R^1$ represents $C_{1-4}$alkyl, -$C_{1-4}$alkyl-$NR^{4a}R^{4b}$, or -$C_{1-4}$alkyl-$OR^5$;

$R^2$ represents hydrogen, or -$CH_2$-$OR^6$; and

$R^3$ represents hydrogen, or $C_{1-4}$alkyl;
or

$R^1$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and

$R^2$ represents -$CH_2$-$OR^6$;
or

$R^2$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and

$R^1$ represents hydrogen;
or

$R^1$ and $R^2$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and

$R^3$ represents hydrogen or $C_{1-4}$alkyl;

$R^{4a}$ and $R^{4b}$ are each independently selected from the group consisting of $C_{1-4}$alkyl, -$C_{1-4}$alkyl-$Het^{1b}$, -$C_{2-4}$alkyl-O-$C_{1-4}$alkyl, and -$C_{2-4}$alkyl-O-$C_{1-4}$alkyl-O-$C_{1-4}$alkyl;

or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^5$ represents hydrogen, $C_{1-4}$alkyl, $Het^{1a}$, -$C_{1-4}$alkyl-$Het^{1b}$, or

$C_{1-4}$alkyl substituted with one or two -O-$C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents $C_{1-4}$alkyl, -$C_{1-4}$alkyl-$NR^{4a}R^{4b}$, or -$C_{1-4}$alkyl-$OR^5$;

$R^2$ represents hydrogen, or -$CH_2$-$OR^6$; and

$R^3$ represents hydrogen, or $C_{1-4}$alkyl;

$R^{4a}$ and $R^{4b}$ are each independently selected from the group consisting of $C_{1-4}$alkyl, -$C_{1-4}$alkyl-$Het^{1b}$, -$C_{2-4}$alkyl-O-$C_{1-4}$alkyl, and -$C_{2-4}$alkyl-O-$C_{1-4}$alkyl-O-$C_{1-4}$alkyl;

or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;

or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;

$R^5$ represents hydrogen, $C_{1-4}$alkyl, $Het^{1a}$, -$C_{1-4}$alkyl-$Het^{1b}$, or

$C_{1-4}$alkyl substituted with one or two -O-$C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and

$R^2$ represents $-CH_2-OR^6$;

$R^5$ represents hydrogen, $C_{1-4}$alkyl, $Het^{1a}$, $-C_{1-4}$alkyl-$Het^{1b}$, or

$C_{1-4}$alkyl substituted with one or two $-O-C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^2$ and $R^3$ are taken together to form together with the atoms to which they are attached a 4- to 7-membered monocyclic fully saturated heterocyclyl containing one O-atom and optionally one additional heteroatom selected

from O, S, and N, wherein said S-atom might be substituted to form S(=O) or $S(=O)_2$, and wherein said heterocyclyl is optionally substituted with one, two or three $C_{1-4}$alkyl substituents;
and

$R^1$ represents hydrogen;

$R^5$ represents hydrogen, $C_{1-4}$alkyl, $Het^{1a}$, -$C_{1-4}$alkyl-$Het^{1b}$, or

$C_{1-4}$alkyl substituted with one or two -O-$C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ and $R^2$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and

$R^3$ represents hydrogen or $C_{1-4}$alkyl;

$R^5$ represents hydrogen, $C_{1-4}$alkyl, $Het^{1a}$, -$C_{1-4}$alkyl-$Het^{1b}$, or

$C_{1-4}$alkyl substituted with one or two -O-$C_{1-4}$alkyl;

$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;

$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;

$R^6$ represents hydrogen or $C_{1-4}$alkyl;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention relates to those compounds of Formula (I) and the pharmaceutically acceptable salts, and the solvates thereof, or any subgroup thereof as mentioned in any of the other embodiments, wherein Y represents O.

In an embodiment, the present invention relates to those compounds of Formula (I) and the pharmaceutically acceptable salts, and the solvates thereof, or any subgroup thereof as mentioned in any of the other embodiments, wherein Y represents $CH_2$.

In an embodiment, the present invention relates to those compounds of Formula (I) and the pharmaceutically acceptable salts, and the solvates thereof, or any subgroup thereof as mentioned in any of the other embodiments, wherein n is 1.

In an embodiment, the present invention relates to those compounds of Formula (I) and the pharmaceutically acceptable salts, and the solvates thereof, or any subgroup thereof as mentioned in any of the other embodiments, wherein n is 2.

In an embodiment, the present invention relates to those compounds of Formula (I) and the pharmaceutically acceptable salts, and the solvates thereof, or any subgroup thereof as mentioned in any of the other embodiments, wherein

$R^{4a}$and $R^{4b}$ are each independently selected from the group consisting of $C_{1-4}$alkyl, -$C_{1-4}$alkyl-Het$^{1b}$, -$C_{2-4}$alkyl-O-$C_{1-4}$alkyl, and -$C_{2-4}$alkyl-O-$C_{1-4}$alkyl-O-$C_{1-4}$alkyl.

In an embodiment, the present invention relates to those compounds of Formula (I) and the pharmaceutically acceptable salts, and the solvates thereof, or any subgroup thereof as mentioned in any of the other embodiments, wherein

$R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$.

In an embodiment, the present invention relates to those compounds of Formula (I) and the pharmaceutically acceptable salts, and the solvates thereof, or any subgroup thereof as mentioned in any of the other embodiments, wherein

$R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-a):

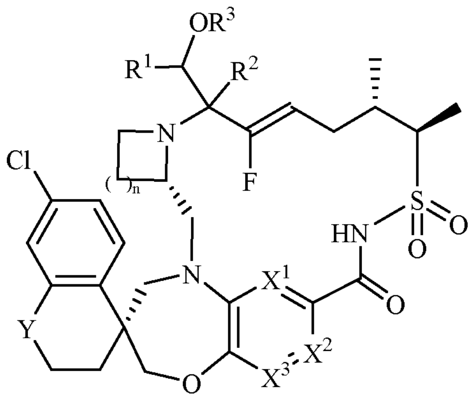

(I-a)

It will be clear that all variables in the structure of Formula (I-a), are defined as defined for the compounds of Formula (I) or any subgroup thereof as mentioned in any of the other embodiments.

In an embodiment, the present invention relates to those compounds of Formula (I) and the pharmaceutically

acceptable salts, and the solvates thereof, or any subgroup thereof as mentioned in any of the other embodiments, wherein the compounds of Formula (I) are restricted to compounds of Formula (I-b):

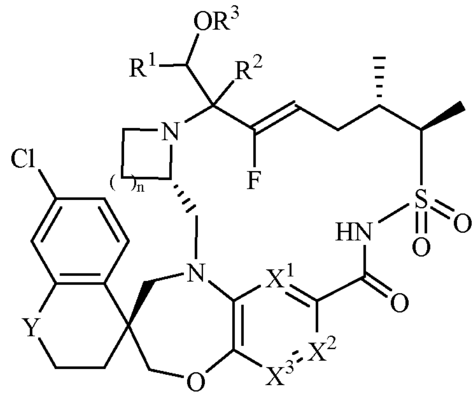

(I-b)

It will be clear that all variables in the structure of Formula (I-b), are defined as defined for the compounds of Formula (I) or any subgroup thereof as mentioned in any of the other embodiments.

In an embodiment, the present invention relates to a subgroup of Formula (I) as defined in the general reaction schemes.

In an embodiment the compound of Formula (I) is selected from the group consisting of any of the exemplified compounds,
and the free bases, the pharmaceutically acceptable salts, and the solvates thereof.

All possible combinations of the above indicated embodiments are considered to be embraced within the scope of the invention.

METHODS FOR THE PREPARATION OF COMPOUNDS

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or can be prepared by published methods. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art including also analogous reaction protocols as described in WO2016033486, WO2017147410 and WO2018183418.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under $N_2$-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described

herein may be synthesized in the form of mixtures of tautomers and stereoisomeric forms that can be separated from one another following art-known resolution procedures.

The meaning of the chemical abbreviations used in the schemes below are as defined in Table 1.

Compounds of Formula (I), wherein $X^1$, $X^2$, $X^3$, Y, n, $R^1$, $R^2$, and $R^3$ are as defined in Formula (I), can be prepared according to Scheme 1,

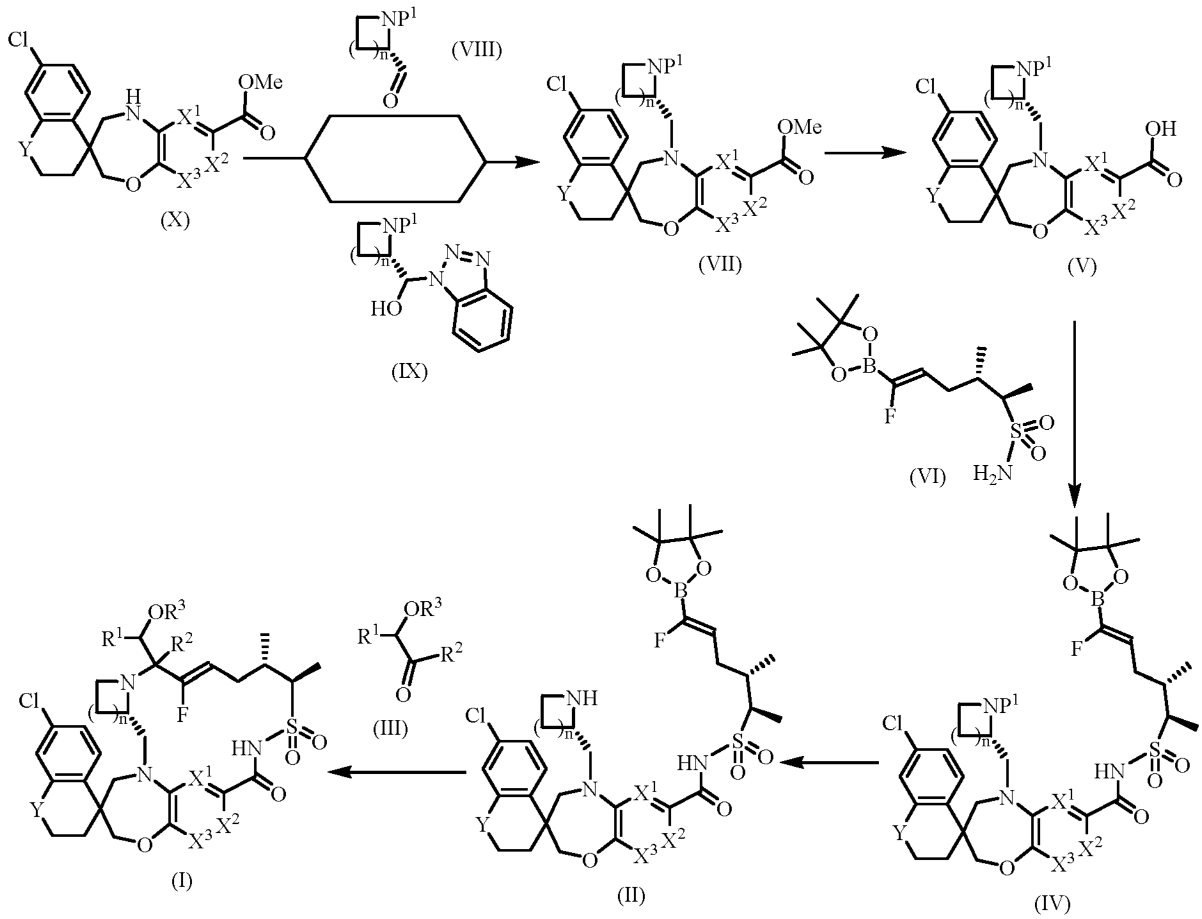

Scheme 1

- By reacting an intermediate of Formula (II) with an intermediate of Formula (III) in a suitable solvent such as, for example, MeOH, at a suitable temperature such as, for example, 60 °C.
- Intermediates of Formula (II) can be prepared by reacting an intermediate of Formula (IV), wherein $X^1$, $X^2$, $X^3$, Y, and n, are as defined in Formula (I), and $P^1$ is a suitable protecting group such as, for example, Boc, with a suitable deprotecting agent such as, for example, TFA or a solution of HCl in dioxane, in a suitable solvent such as, for example, DCM or dioxane, at a suitable temperature such as, for example, room temperature.
- Intermediates of Formula (IV) can be prepared by reacting an intermediate of Formula (V), wherein $X^1$, $X^2$, $X^3$, Y, and n are as defined in Formula (I), and $P^1$ is a suitable protecting group such as, for example, Boc with an intermediate of Formula (VI), in a suitable solvent such as, for example, DCM, in the presence of a suitable base such as, for example, DMAP, and in the presence of a suitable coupling agent such as, for example, EDC.HCl, at a suitable temperature such as, for example, room temperature.
- Intermediates of Formula (V) can be prepared by reacting an intermediate of Formula (VII), wherein $X^1$, $X^2$, $X^3$, Y, and n are as defined in Formula (I), with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 50 °C.
- Intermediates of Formula (VII) can be prepared by reacting an intermediate of Formula (X), wherein $X^1$, $X^2$, $X^3$, and Y are as defined in Formula (I), with a suitable intermediate of Formula (VIII), or a suitable activated form of this intermediate such as a compound of Formula (IX), in the presence of a suitable acid such as, for example, AcOH, and in the presence of a suitable reducing agent such as, for example, $NaBH(OAc)_3$, in a suitable solvent such as, for

example, DCM, at a suitable temperature such as, for example, 0 °C or room temperature.

- Intermediates of Formula (IX) can be prepared by reacting an intermediate of Formula (VIII) with benzotriazole, in a suitable solvent such as, for example, methyltertbutyl ether, at a suitable temperature such as, for example, room temperature.

Alternatively, compounds of Formula (I), wherein $R^1$ and $R^3$ taken together can form a monocyclic fully saturated ring, can be prepared by reacting compounds of Formula (I), wherein $R^3$ is defined as H, and $R^1$ is defined as $-CH_2OH$, with a suitable ketone such as, for example, acetone, in the presence of a suitable acid such as, for example, PTSA, at a suitable temperature such as, for example, room temperature.

Alternatively, compounds of Formula (I), wherein $R^1$ and $R^2$ taken together can form a monocyclic fully saturated ring, can be prepared by reacting compounds of Formula (I), wherein $R^3$ is defined as H, and $R^1$ and $R^2$ are defined as $-CH_2OH$, with a suitable ketone such as, for example, acetone, in the presence of a suitable acid such as, for example, PTSA, at a suitable temperature such as, for example, room temperature.

A skilled person will understand that using appropriate protection-deprotection strategies, rings between $R^1$, $R^2$ or $R^3$ could be formed selectively. A skilled person will also understand that art known conditions could provide alternative ring systems between $R^1$, $R^2$ or $R^3$ than the ones described hereinabove.

Intermediates of Formula (X) have been described in literature but can also be prepared according to Scheme 2,

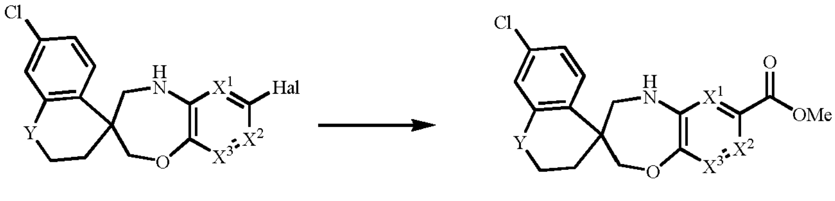

(XI) (X)

Scheme 2

by reacting an intermediate of Formula (XI), wherein $X^1$, $X^2$, $X^3$, and Y, are as defined in Formula (I), and Hal is defined as an halogen such as, for example, I or Br, in particular I, with MeOH, in the presence of a suitable pressure of CO such as, for example, 50 atm, in the presence of a suitable catalyst such as, for example, [1,1'-bis(diphenylphosphino)ferrocene] dichloropalladium(II) ($PdCl_2$(dppf)), at a suitable temperature such as, for example, 100 °C.

Alternatively, Intermediates of Formula (VII) can be prepared according to Scheme 3,

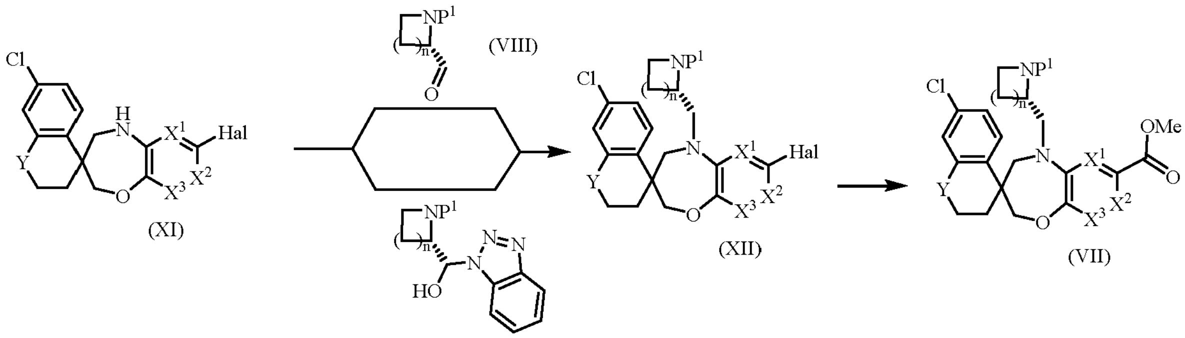

(VIII) (XI) (XII) (VII) (IX)

Scheme 3

- by reacting an intermediate of Formula (XII), wherein $X^1$, $X^2$, $X^3$, and n, are as defined in Formula (I), and Hal is defined as an halogen such as, for example, I or Br, in particular I, with MeOH, in the presence of a suitable base such as, for example, $Et_3N$, in the presence of a suitable pressure of CO such as, for example, 30 bar, in the presence of a suitable catalyst such as, for example, [1,1'-bis(diphenylphosphino)ferrocene]dichloropalladium(II) ($PdCl_2$(dppf)), at a suitable temperature such as, for example, 100 °C.
- Intermediates of Formula (XII) can be prepared by reacting an intermediate of Formula (XI), wherein $X^1$, $X^2$, $X^3$, and Y are as defined in Formula (I), and Hal is defined as an halogen such as, for example, I or Br, in particular I, with a suitable intermediate of Formula (VIII), or a suitable activated form of this intermediate such as a compound of

Formula (IX), in the presence of a suitable acid such as, for example, AcOH, and in the presence of a suitable reducing agent such as, for example, $NaBH(OAc)_3$, in a suitable solvent such as, for example, DCM, at a suitable temperature such as, for example, 0 °C or room temperature.

Intermediates of Formula (VI) can be prepared according to Scheme 4,

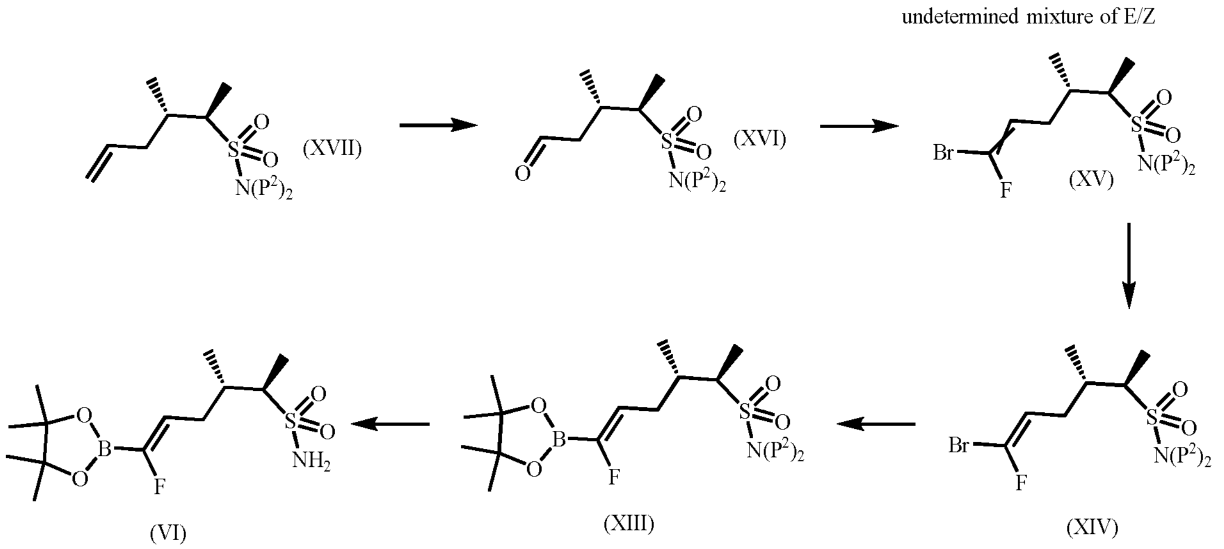


(XVII) (XVI) (XV) (XIV) (XIII) (VI)

Scheme 4

- by reacting an intermediate of Formula (XIII), wherein $P^2$ is a suitable protecting group such as, for example, PMB (p-methoxybenzyl), with a suitable deprotecting agent such as, for example, TFA, in a suitable solvent such as, for example, DCM, at a suitable temperature such as, for example, room temperature.
- Intermediates of Formula (XIII) can be prepared by reacting an intermediate of Formula (XIV) with bis(pinacolato) diboron, in the presence of a suitable base such as, for example, potassium acetate, in the presence of a suitable catalyst such as, for example, $PdCl_2(dppf).CH_2Cl_2$, in a suitable solvent such as, for example, 1,4-dioxane, at a suitable temperature such as, for example, 95 °C.
- Intermediates of Formula (XIV) can be prepared by reacting an intermediate of Formula (XV) with a suitable base such as, for example, LiHMDS, in a suitable solvent such as, for example, THF, at a suitable temperature such as, for example 0 °C.
- Intermediates of Formula (XV) can be prepared by reacting an intermediate of Formula (XVI) with fluorotribromomethane and triphenylphosphine, in the presence of a suitable dialkylzinc derivative such as, for example, diethylzinc, in a suitable solvent such as, for example, THF, at a suitable temperature such as, for example, room temperature.
- Intermediates of Formula (XVI) can be prepared by reacting an intermediate of Formula (XVII) with ozone, in the presence of a suitable reductant such as, for example, triphenylphosphine, in a suitable solvent such as, for example, DCM or MeOH or a mixture thereof, at a suitable temperature such as, for example, -78 °C.
- Intermediates of Formula (XVII) are described in literature or can be prepared in analogy with published procedures.

Alternatively Intermediates of Formula (XV), wherein $P^2$ is a suitable protective group such as, for example, p-methoxybenzyl, can be prepared according to Scheme 5,

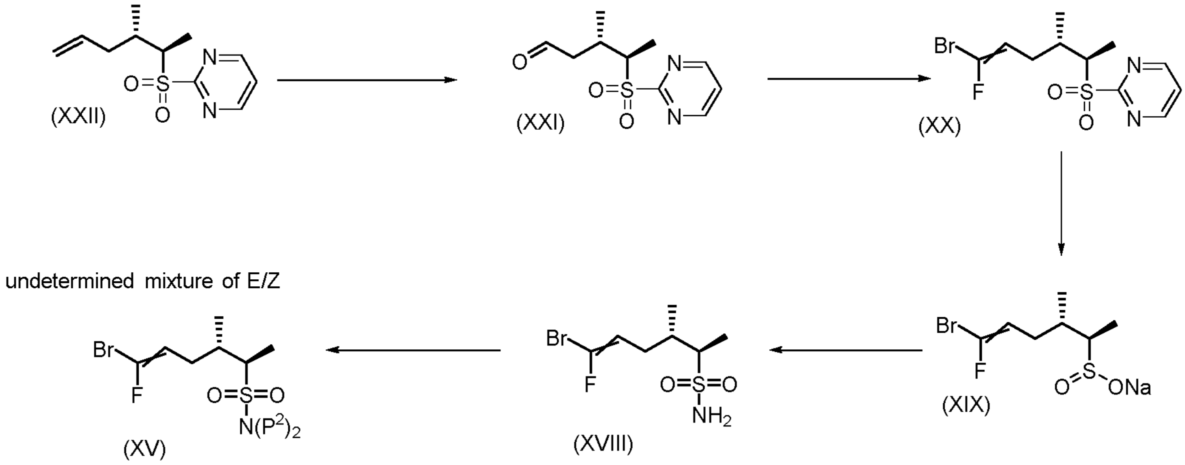


(XXII) (XXI) (XX) (XIX) (XVIII) (XV)

Scheme 5

- By protecting the intermediate of Formula (XVIII) with a suitable protecting group such as, for example, p-methoxybenzyl chloride, in presence of a suitable base such as, for example, potassium carbonate, in a suitable solvent such as, for example, THF.
- Intermediate of Formula (XVIII) can be prepared by treatment of the intermediate of Formula (XIX) with sodium acetate and hydroxylamine-O-sulfonic acid in a suitable solvent such as, for example, water, at a suitable temperature such as, for example, room temperature.
- Intermediate of Formula (XIX) can be prepared by treatment of the intermediate of Formula (XX) with an appropriate base such as, for example, sodium methoxide, in a suitable solvent such as, for example, methanol, at a suitable temperature such as, for example, 0 °C or room temperature.
- Intermediate of Formula (XX) can be prepared by reacting the intermediate of Formula (XXI) with fluorotribromomethane and triphenylphosphine, in the presence of a suitable dialkylzinc derivative such as, for example, diethylzinc, in a suitable solvent such as, for example, THF, at a suitable temperature such as, for example, room temperature.
- Intermediate of Formula (XXI) can be prepared either by ozonolysis of the intermediate of Formula (XXII) in a suitable solvent such as, for example, dichloromethane or methanol, at a suitable temperature such as, for example, - 78 °C, or by oxidation of the intermediate of Formula (XXII) with suitable reagents such as, for example, a catalytic amount of osmium tetroxide with sodium periodate, in a suitable solvent such as, for example, tetrahydrofuran and water mixture, at a suitable temperature such as, for example, room temperature.

Intermediate (XXII) corresponds with (CAS [1638587-10-6]).

Alternatively Intermediates of Formula (VI), wherein $P^2$ is a suitable protective group such as, for example, p-methoxybenzyl, can be prepared according to Scheme 6,

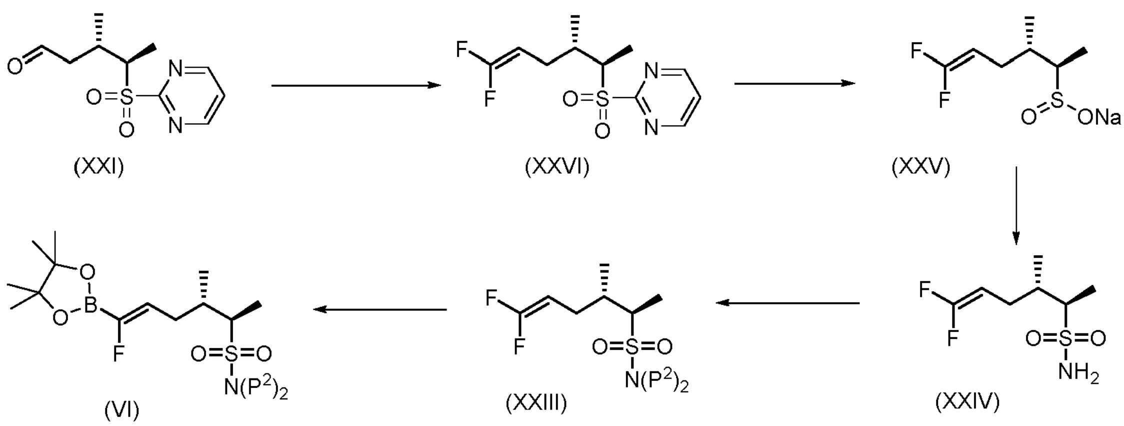

(XXI) (XXVI) (XXV) (XXIV) (XXIII) (VI)

Scheme 6

- By reacting an intermediate of Formula (XXIII) with bis(pinacolato)diboron and a copper catalyst such as, for example,

copper chloride mixed with a phosphine ligand such as, for example, XantPhos, in presence of a base such as, for example, potassium tert-butoxide, and an alcohol such as, for example, methanol, in a suitable solvent such as, for example, DME, at a suitable temperature such as, for example, 40 °C.

- Intermediates of Formula (XXIII) can be prepared by protecting the intermediate of Formula (XXIV) with a suitable protecting group such as, for example, p-methoxybenzyl chloride, in presence of a suitable base such as, for example, potassium carbonate, in a suitable solvent such as, for example, THF.
- Intermediate of Formula (XXIV) can be prepared by treatment of the intermediate of Formula (XXV) with sodium acetate and hydroxylamine-O-sulfonic acid in a suitable solvent such as, for example, water, at a suitable temperature such as, for example, room temperature.
- Intermediate of Formula (XXV) can be prepared by treatment of the intermediate of Formula (XXVI) with an appropriate base such as, for example, sodium methoxide, in a suitable solvent such as, for example, methanol, at a suitable temperature such as, for example, 0 °C or room temperature.
- Intermediate of Formula (XXVI) can be prepared by reacting the intermediate of Formula (XXI) with sodium chlorodifluoroacetate and triphenylphosphine, in a suitable solvent such as, for example, DMF, at a suitable temperature such as, for example, room temperature.

Intermediates of Formula (III), wherein $R^2$ and $R^3$ are defined as H (hydrogen) and $R^1$ is defined as $-CH_2-NR^{4a}R^{4b}$, or - $CH_2-OR^5$, can be prepared according to Scheme 7,

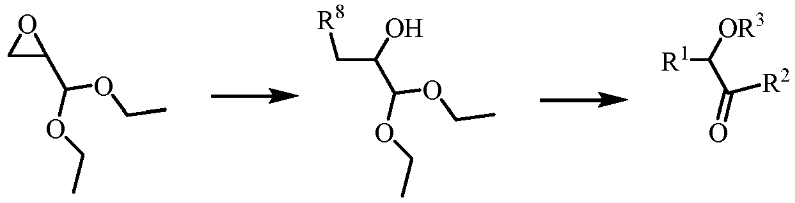

(XXVIII) (XXVII) (III)

Scheme 7

- by reacting an intermediate of Formula (XXVII), wherein $R^8$ is defined as $-NR^{4a}R^{4b}$ or $-O-C_{1-4}$alkyl , with a suitable deprotecting agent such as, for example, 1 M aqueous HCl or a suitable solid-supported source of protons, such as, for example, Amberlyst®-15 (H form), in a suitable solvent such as, for example, water or acetone, or a mixture thereof, at a suitable temperature such as, for example, room temperature or 60 °C.
- Intermediates of Formula (XXVII) can be prepared by reacting an intermediate of Formula (XVIII) with a suitable source of $R^8$, such as, for example, an amine $H-NR^{1a}R^{1b}$, an alcohol such as, for example, tetrahydro-2H-pyran-4-ol , or a heterocycle nucleophile such as, for example, pyrazole, in the presence of a suitable base such as, for example, KOH or $Cs_2CO_3$, neat or in a suitable solvent such as, for example, DMF, at a suitable temperature such as, for example, 70 °C.
- Alternatively, intermediates of Formula (XXVII) can be prepared by reacting an intermediate of Formula (XVIII) with a suitable precursor for $R^8$, such as, for example, an azide, with an appropriate reagent such as $NaN_3$, in a suitable solvent such as, for example, MeOH, at a suitable temperature such as, for example, 70 °C. Said precursor can further be converted to $R^8$ under skilled art conditions. For instance, in case said precursor is an azide, a reduction and alkylation can provide an amine of formula $NR^{4a}R^{4b}$, or alternatively a cycloaddition with a suitable alkyne can provide an amine of formula $NR^{4a}R^{4b}$ wherein $R^{4a}$ and $R^{4b}$ are taken together to form together with the N-atom to which they are attached a 5- to 6-membered monocyclic aromatic ring.

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically

isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

PHARMACOLOGY OF COMPOUNDS

It has been found that the compounds of the present invention inhibit one of more MCL-1 activities, such as MCL-1 antiapoptotic activity.

An MCL-1 inhibitor is a compound that blocks one or more MCL-1 functions, such as the ability to bind and repress proapoptotic effectors Bak and Bax or BH3 only sensitizers such as Bim, Noxa or Puma.

The compounds of the present invention can inhibit the MCL-1 pro-survival functions. Therefore, the compounds of the present invention may be useful in treating and / or preventing, in particular treating, diseases that are susceptible to the effects of the immune system such as cancer.

In another embodiment of the present invention, the compounds of the present invention exhibit anti-tumoral properties, for example, through immune modulation.

In an embodiment, the present invention is directed to a compound of formula (I) for use in methods for treating and / or preventing a cancer, wherein the cancer is selected from those described herein, comprising administering to a subject in need thereof (preferably a human), a therapeutically effective amount of a compound of Formula (I), or pharmaceutically acceptable salt, or a solvate thereof.

In an embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of acute lymphoblastic leukemia (ALL), acute myeloid leukemia (AML), B cells acute lymphoblastic leukemia, B-cell chronic lymphocytic leukemia (CLL), bladder cancer, breast cancer, chronic lymphocytic leukemia, chronic myeloid leukemia, colon adenocarcinoma, diffuse large B cell lymphoma, esophageal cancer, follicular lymphoma, gastric cancer, head and neck cancer (including, but not limited to head and neck squamous cell carcinoma), hematopoietic cancer, hepatocellular carcinoma, Hodgkin lymphoma, liver cancer, lung cancer (including but not limited to lung adenocarcinoma), lymphoma, medulloblastoma, melanoma, monoclonal gammopathy of undetermined significance, multiple myeloma, myelodysplastic syndromes, myelofibrosis, myeloproliferative neoplasms, ovarian cancer, ovarian clear cell carcinoma, ovarian serous cystadenoma, pancreatic cancer, polycythemia vera, prostate cancer, rectum adenocarcinoma, renal cell carcinoma, smoldering multiple myeloma, T cell acute lymphoblastic leukemia, T cell lymphoma, and Waldenstroms macroglobulinemia.

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is preferably selected from the group consisting of acute lymphoblastic leukemia (ALL), acute myeloid leukemia (AML), B cells acute lymphoblastic leukemia, B-cell chronic lymphocytic leukemia (CLL), breast cancer, chronic lymphocytic leukemia, chronic myeloid leukemia, diffuse large B cell lymphoma, follicular lymphoma, hematopoietic cancer, Hodgkin lymphoma, lung cancer (including, but not limited to lung adenocarcinoma) lymphoma, monoclonal gammopathy of undetermined significance, multiple myeloma, myelodysplastic syndromes, myelofibrosis, myeloproliferative neoplasms, smoldering multiple myeloma, T cell acute lymphoblastic leukemia, T cell lymphoma and Waldenstroms macroglobulinemia.

In another embodiment, the present invention is directed to compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of adenocarcinoma, benign monoclonal gammopathy, biliary cancer (including, but not limited to, cholangiocarcinoma), bladder cancer, breast cancer (including, but not limited to, adenocarcinoma of the breast, papillary carcinoma of the breast, mammary cancer, medullary carcinoma of the breast), brain cancer (including, but not limited to, meningioma), glioma (including, but not limited to, astrocytoma, oligodendroglioma; medulloblastoma), bronchus cancer, cervical cancer (including, but not limited to, cervical adenocarcinoma), chordoma, choriocarcinoma, colorectal cancer (including, but not limited to, colon cancer, rectal cancer, colorectal adenocarcinoma), epithelial carcinoma, endothelial sarcoma (including, but not limited to, Kaposi's sarcoma, multiple

idiopathic hemorrhagic sarcoma), endometrial cancer (including, but not limited to, uterine cancer, uterine sarcoma), esophageal cancer (including, but not limited to, adenocarcinoma of the esophagus, Barrett's adenocarinoma), Ewing sarcoma, gastric cancer (including, but not limited to, stomach adenocarcinoma), gastrointestinal stromal tumor (GIST), head and neck cancer (including, but not limited to, head and neck squamous cell carcinoma), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, kidney cancer (including, but not limited to, nephroblastoma a.k.a. Wilms' tumor, renal cell carcinoma), liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, *Lewis lung carcinoma,* lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES), ovarian cancer (including, but not limited to, cystadenocarcinoma, ovarian embryonal carcinoma, ovarian adenocarcinoma), pancreatic cancer (including, but not limited to, pancreatic andenocarcinoma, intraductal papillary mucinous neoplasm (IPMN), Islet cell tumors), prostate cancer (including, but not limited to, prostate adenocarcinoma), skin cancer (including, but not limited to, squamous cell carcinoma (SCC), keratoacanthoma (KA), melanoma, basal cell carcinoma (BCC)) and soft tissue sarcoma (e.g. malignant fibrous histiocytoma (MFH), liposarcoma, malignant peripheral nerve sheath tumor (MPNST), chondrosarcoma, fibrosarcoma, myxosarcoma).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of benign monoclonal gammopathy, breast cancer (including, but not limited to, adenocarcinoma of the breast, papillary carcinoma of the breast, mammary cancer, medullary carcinoma of the breast), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, Lewis lung carcinoma, lung neuroendocrine tumors, typical carcinoid, atypical carcinoid, small cell lung cancer (SCLC), and large cell neuroendocrine carcinoma), myelodysplastic syndromes (MDS), myeloproliferative disorder

(MPD), and prostate cancer (including, but not limited to, prostate adenocarcinoma).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of prostate, lung, pancreatic, breast, ovarian, cervical, melanoma, B-cell chronic lymphocytic leukemia (CLL), acute myeloid leukemia (AML), and acute lymphoblastic leukemia (ALL).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is multiple myeloma.

The compounds according to the present invention or pharmaceutical compositions comprising said compounds, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1 and or CTLA-4 or engineered chimeric antigen receptor T cells (CART) targeting tumor associated antigens.

The compounds according to the present invention or pharmaceutical compositions comprising said compounds, may also be combined with radiotherapy or chemotherapeutic agents (including, but not limited to, anti-cancer agents) or any other pharmaceutical agent which is administered to a subject having cancer for the treatment of said subject's cancer or for the treatment or prevention of side effects associated with the treatment of said subject's cancer.

The compounds according to the present invention or pharmaceutical compositions comprising said compounds, may also be combined with other agents that stimulate or enhance the immune response, such as vaccines.

Therefore also disclosed herein are the methods for treating and / or preventing a cancer (wherein the cancer is selected from those described herein) comprising administering to a subject in need thereof (preferably a human), a therapeutically effective amount of co-therapy or combination therapy; wherein the co-therapy or combination therapy comprises a compound of Formula (I) of the present invention and one or more anti-cancer agent(s) selected from the group consisting of (a) immune modulatory agent (such as inhibitors of the PD1/PDL1 immune checkpoint axis, for example antibodies (or peptides) that bind to and/or inhibit the activity of PD-1 or the activity of PD-L1 and or CTLA-4); (b) engineered chimeric antigen receptor T cells (CART) targeting tumor associated antigens; (c) radiotherapy; (d) chemotherapy; and (e) agents that stimulate or enhance the immune response, such as vaccines.

The present invention is directed to compounds of Formula (I) and pharmaceutically acceptable salts, and solvates thereof, for use as a medicament.

The present invention is directed to compounds of Formula (I) and pharmaceutically acceptable salts, and solvates thereof, for use in the inhibition of MCL-1 activity.

As used herein, unless otherwise noted, the term "anti-cancer agents" shall encompass "anti-tumor cell growth agents" and "anti-neoplastic agents".

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing,
in particular for treating, MCL-1 mediated diseases or conditions, preferably cancer, more preferably a cancer as herein described (for example, multiple myeloma).

The present invention is directed to compounds of Formula (I) and pharmaceutically acceptable salts, and solvates thereof, for use in treating and / or preventing, in particular for use in treating, MCL-1 mediated diseases or conditions, preferably cancer, more preferably a cancer as herein described (for example, multiple myeloma).

The present invention relates to compounds of Formula (I) and pharmaceutically acceptable salts, and solvates thereof, for the manufacture of a medicament.

The present invention relates to compounds of Formula (I) and pharmaceutically acceptable salts, and solvates thereof, for the manufacture of a medicament for the inhibition of MCL-1.

The present invention relates to compounds of Formula (I) and pharmaceutically acceptable salts, and solvates

thereof, for the manufacture of a medicament for treating and / or preventing, in particular for treating, a cancer, preferably a cancer as herein described. More particularly, the cancer is a cancer which responds to inhibition of MCL-1 (for example, multiple myeloma).

The present invention is directed to compounds of Formula (I) and pharmaceutically acceptable salts, and solvates thereof, for the manufacture of a medicament for treating and / or preventing, in particular for treating, any one of the disease conditions mentioned hereinbefore.

The present invention is directed to compounds of Formula (I) and pharmaceutically acceptable salts, and solvates thereof, for the manufacture of a medicament for treating and / or preventing any one of the disease conditions mentioned hereinbefore.

The compounds of Formula (I) and pharmaceutically acceptable salts, and solvates thereof, can be administered to subjects, preferably humans, for treating and / or preventing of any one of the diseases mentioned hereinbefore.

In view of the utility of the compounds of Formula (I) and pharmaceutically acceptable salts, and solvates thereof, there is provided compound of formula (I) for use in a method of treating subjects, preferably mammals such as humans, suffering from any of the diseases mentioned hereinbefore; or a method of slowing the progression of any of the diseases mentioned hereinbefore in subject, humans; or a method of preventing subjects, preferably mammals such as humans, from suffering from any one of the diseases mentioned hereinbefore.

Said methods comprise the administration, i.e. the systemic or topical administration, preferably oral or intravenous administration, more preferably oral administration, of an effective amount of a compound of Formula (I) or a pharmaceutically acceptable salt, or a solvate thereof, to subjects such as humans.

One skilled in the art will recognize that a therapeutically effective amount of the compounds of the present invention is the amount sufficient to have therapeutic activity and that this amount varies *inter alias,* depending on the type of disease, the concentration of the compound in the therapeutic formulation, and the condition of the patient. In an embodiment, a therapeutically effective daily amount may be from about 0.005 mg/kg to 100 mg/kg.

The amount of a compound according to the present invention, also referred to herein as the active ingredient, which is required to achieve a therapeutic effect may vary on case-by-case basis, for example with the specific compound, the route of administration, the age and condition of the recipient, and the particular disorder or disease being treated. The methods of the present invention may also include administering the active ingredient on a regimen of between one and four intakes per day. In these methods of the present invention, the compounds according to the invention are preferably formulated prior to administration.

The present invention also provides compositions for use in treating and / or preventing the disorders (preferably a cancer as described herein) referred to herein. Said compositions comprise a therapeutically effective amount of a compound of Formula (I), or a pharmaceutically acceptable salt, or a solvate thereof, and a pharmaceutically acceptable carrier or diluent.

While it is possible for the active ingredient (e.g. a compound of the present invention) to be administered alone, it is preferable to administer it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of the present invention may be prepared by any methods well known in the art of pharmacy, for example, using methods such as those described in, for example, Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own separate pharmaceutical dosage formulation.

Therefore, also disclosed herein is a product comprising, as a first active ingredient a compound according to the invention and as further, as an additional active ingredient one or more anti-cancer agent(s), as a combined preparation for simultaneous, separate or sequential use in the treatment of patients suffering from cancer.

The one or more other anti-cancer agents and the compound according to the present invention may be administered simultaneously (e.g. in separate or unitary compositions) or sequentially, in either order. In an embodiment, the two or more compounds are administered within a period and / or in an amount and / or a manner that is sufficient to ensure that an advantageous or synergistic effect is achieved. It will be appreciated that the preferred method and order of administration and the respective dosage amounts and regimes for each component of the combination will depend on the particular other anti-cancer agent and the compound of the present invention being administered, their route of administration, the particular condition, in particular tumor, being treated and the particular host being treated.

The following examples further illustrate the present invention.

EXAMPLES

Several methods for preparing the Compounds of this invention are illustrated in the following examples. Unless otherwise noted, all starting materials were obtained from commercial suppliers and used without further purification, or alternatively can be synthesized by a skilled person by using published methods.

Table 1: Abbreviations

| Abbreviation | Meaning |
|---|---|
| **ACN** | acetonitrile |
| **AcOH** | acetic acid |
| **DCM** | dichloromethane |
| **DCE** | dichloroethane |
| **DMF** | *N,N*-dimethylformamide |
| **DIPE** | diisopropyl ether |
| **DMSO** | dimethyl sulfoxide |
| **DMAP** | 4-dimethylaminopyridine |
| **Me** | methyl |
| **EDC.HCl or EDCI** | N-(3-dimethylaminopropyl)-N'-ethylcarbodiimide hydrochloride |
| **EtOAc** | ethyl acetate |
| **eq.** | equivalent(s) |
| **Et** | ethyl |
| **$Et_3N$** | triethylamine |
| **EtOH** | ethanol |
| **MeOH** | methanol |
| **MeI** | methyl iodide |
| **h** | hour(s) |
| **min** | minute(s) |
| **HPLC** | high performance liquid chromatography |
| **SFC** | supercritical fluid chromatography |
| **Prep** | preparative |
| **RP** | reversed phase |
| **LiHMDS** | lithium bis(trimethylsilyl)amide |
| **$NaBH(OAc)_3$** | sodium triacetoxyborohydride |
| **$NH_4OAc$** | ammonium acetate |
| **THF** | tetrahydrofuran |
| **Celite®** | diatomaceous earth |
| **Dicalite®** | diatomaceous earth |
| **RP** | reversed phase |
| **$iPrNH_2$** | isopropylamine |
| **Boc** | tert-butyloxycarbonyl |
| **DEAD** | diethyl azodicarboxylate |
| **$PBu_3$** | tributylphosphine |
| **MTBE** | methyl -tert-butyl ether |

(continued)

| Abbreviation | Meaning |
|---|---|
| **NaOAc** | sodium acetate |
| **NaOMe** | sodium methoxide |
| **$PPh_3$ or $Ph_3P$** | triphenylphosphine |
| **PMB** | p-methoxybenzyl |
| **PTSA** | p-toluenesulfonic acid |
| **TBAF** | tetrabutylammonium fluoride |
| **TBSCl** | tert-butyl(chloro)dimethylsilane |
| **TFA** | trifluoroacetic acid |
| **tBuOK** | potassium tert-butoxide |
| **$PdCl_2$(dppf)** | [1,1'-bis(diphenylphosphino)ferrocene]dichloropalladium(II) |
| **Xantphos** | 4,5-bis(diphenylphosphino)-9,9-dimethylxanthene |

As understood by a person skilled in the art, Compounds synthesized using the protocols as indicated may contain residual solvent or minor impurities.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

A crossed double bond like for example in intermediates 37-39 means 'undetermined mixture of E/Z'.

**Preparation of intermediates**

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

**Intermediate 1**

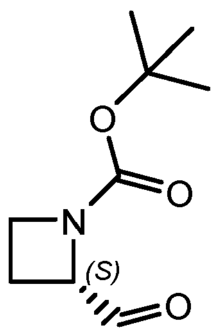

Dess-Martin periodinane (CAS [87413-09-0]) (30 g, 1.3 eq.) was added to a stirred solution of (*S*)-1-Boc-2-azetidinemethanol (CAS [161511-85-9]) (10 g, 53.4 mmol) in DCM (250 mL) at 0 °C. The reaction mixture was stirred at room temperature for 2 h. The reaction mixture was quenched by the addition of a solution of sodium thiosulfate in a saturated aqueous NaHCOs solution. The resulting mixture was stirred vigorously for 15 min. The resulting suspension was filtered over a pad of Celite®. The filter pad was washed with DCM. The combined filtrate was separated, and the aqueous layer was extracted with DCM (2x). The combined organic layer was washed twice with saturated aqueous $NaHCO_3$, dried with $MgSO_4$, and filtered. The solvents of the filtrate were evaporated to give 10.17 g of Intermediate 1 (quantitative) as an oil, used without further purification.

## Intermediate 2

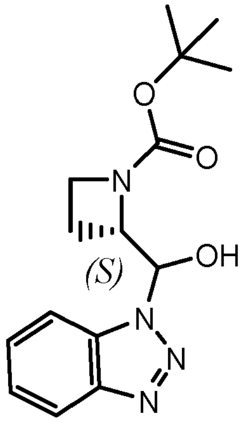

Tert-butyl methyl ether (40 mL) was added to Intermediate 1 (9 g, 48.59 mmol). The resulting suspension was stirred for 10 minutes at room temperature and then filtered. The solid was rinsed with tert-butyl methyl ether. The filtrate was transferred to a round bottom flask and then cooled down to 0 °C. 1H-benzotriazole (5.79 g, 1 eq.) was added to the solution and the reaction mixture was stirred at room temperature for 18 h. The solvents were evaporated to give Intermediate 2 (14.79 g, quantitative), used without further purification.

## Intermediate 3

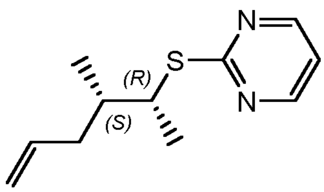

A solution of DEAD (25.79 mL, 1.7 eq.) in THF (100 mL) was added dropwise to $PBu_3$ (36.09 mL, 1.5 eq.) in degassed THF (300 mL) under nitrogen atmosphere at 0 °C. A solution of (2S,3S)-3-methylhex-5-en-2-ol (CAS [125225-80-1], 11 g, 1 eq.) in THF (200 mL) was added dropwise to the mixture at 0 °C. The mixture was stirred at 0 °C for 30 min (the solution turned light orange). Pyrimidine-2-thiol (CAS [131242-36-9], 30.79 g, 2.85 eq.) was added gradually to the mixture. The reaction mixture was stirred at 0 °C for 1 h and then at room temperature overnight. The reaction mixture was filtered. To the filtrate was added 500 mL EtOAc. The solution was washed twice with 1 N $K_2CO_3$ (300 mL) and then twice with brine (300 mL). The aqueous layer was back-extracted with 400 mL EtOAc. The combined organic layer was dried over $Na_2SO_4$ and concentrated in vacuo. The residue was purified by flash column chromatography over silica gel (eluent: petroleum ether/EtOAc from 100/0 to 0/100). The desired fractions were collected and the solvent was concentrated to dryness under vacuum to give Intermediate 3 (35.4 g, yield: 68 %).

## Intermediate 4

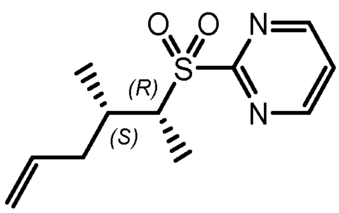

To a mixture of $Na_2WO_4$ (CAS [10213-10-2], 1.075 g, 0.1 eq.), phenylphosphonic acid (CAS [157171-33-1], 0.515 g, 0.1 eq.) and tetrabutylammonium sulfate (3.75 mL, 0.1 eq.) was added $H_2O_2$ (9.24 g, 2.5 eq.) all at once at room temperature. The mixture was aged at room temperature for 5 minutes before a solution of Intermediate 3 (10 g, 1 eq.) in toluene (150 mL) was added in one portion. The biphasic reaction mixture was heated to 50 °C with vigorous stirring. After

60 minutes the reaction was cooled to room temperature. The reaction mixture was poured into water (150 mL). The layers were separated and the aqueous layer was extracted with EtOAc (300 mL × 3). The combined organic layer was washed with a saturated aqueous solution of $Na_2S_2O_5$ (200 mL × 2), dried over $MgSO_4$, filtered, and concentrated. The residue was purified by flash column chromatography over silica gel (eluent: petroleum ether/EtOAc from 100/0 to 0/100). The desired fractions were collected and the solvent was concentrated under vacuum to give Intermediate 4 (5.6 g, yield: 68 %) as a yellow oil.

## Intermediate 5

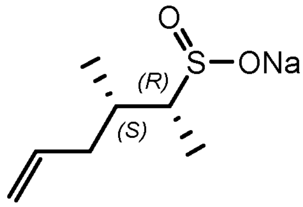

A solution of Intermediate 4 (5.6 g, 1 eq.) in MeOH (30 mL) was cooled to 0 °C and treated with NaOMe (4.794 g, 1 eq.). The mixture was allowed to warm to room temperature for 20 min. The solvent was removed under vacuum. Water (10 mL) was added to the mixture and it was extracted with EtOAc (10 mL x 3). The combined organic layer was concentrated under vacuum to give Intermediate 5 (6.3 g, quantitative) as a yellow solid. The product was used for the next step without further purification.

## Intermediate 6

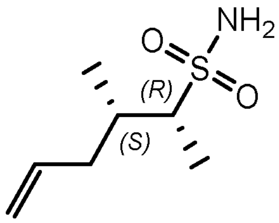

Intermediate 5 (33.2 g, 1 eq.) was dissolved in MeOH (200 mL) and treated with NaOAc (18.48 g, 1.25 eq.), followed by a solution of hydroxylamine O-sulfonic acid (25.48 g, 1.25 eq.) in water (15 mL). The reaction was stirred overnight at room temperature. The mixture was neutralized with solid NaHCOs and extracted with EtOAc (400 mL x 3). The combined organic layers was dried over $MgSO_4$, filtered and concentrated. The residue was purified by flash column chromatography over silica gel (eluent: petroleum ether/EtOAc from 100/0 to 0/100). The desired fractions were collected, and the solvent was concentrated under vacuum to give Intermediate 6 (18.1 g, yield:56 %) as clear oil.

## Intermediate 7

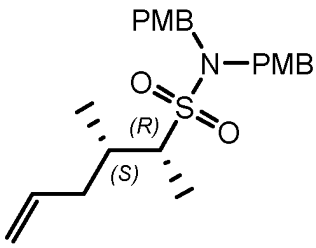

To a solution of Intermediate 6 (5 g, 28.2 mmol) in DMF (50 mL) was added $K_2CO_3$ (15.593 g, 4 eq.), followed by slow addition of 4-methoxybenzyl chloride (11.474 mL, 3 eq). Once the addition was complete, the reaction was heated to 70 °C and was stirred at this temperature overnight. The reaction was filtered through a pad of dicalite® to remove the inorganics and concentrated under reduced pressure to give a pale-yellow oil. The oil was dissolved in EtOAc (150 mL) and washed with brine (2 × 100 mL). The organic layer was dried over $MgSO_4$, filtered, and concentrated under reduced pressure to afford a pale-yellow oil. The crude product was purified by flash column chromatography on silica gel (heptane:EtOAc - 1:0 to 8:2). After evaporation of the fractions containing product, the residue was purified by preparative HPLC (Stationary phase: RP XBridge Prep C18 OBD-10 μm,50×150 mm, Mobile phase: 0.25 %$NH_4HCO_3$ solution in

water, $CH_3CN$) to give Intermediate 7 (5.98 g, yield: 48 %) as a white solid.

## Intermediate 8

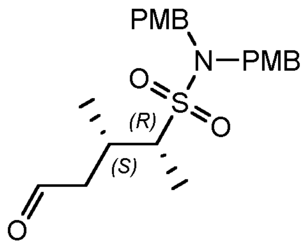

Intermediate 7 (1 g, 2.275 mmol) was dissolved in a mixture of DCM (12.5 mL) and MeOH (12.5 mL) and the resulting mixture was cooled to -78 °C. Ozone (109.199 mg, 1 eq.) was subsequently bubbled through the reaction mixture until a blue persistent color was observed (5 min). Nitrogen was then bubbled through the solution (still at -78 °C) to remove the blue color and this was followed by addition of $PPh_3$ (2.984 g, 5 eq). Once the addition was complete, the reaction was left stirring at -78 °C for 1 h. The reaction mixture was then slowly allowed to warm to room temperature and was stirred for 1 h. The heterogenous mixture was filtered through a pad of dicalite®. The pad was thoroughly washed with DCM. The filtrate was concentrated under reduced pressure to give a green oil that was purified by flash column chromatography on silica gel (heptane:EtOAc - 1:0 to 3:1) to give Intermediate 8 (920 mg, yield: 87 %) as a colorless oil.

## Intermediate 9

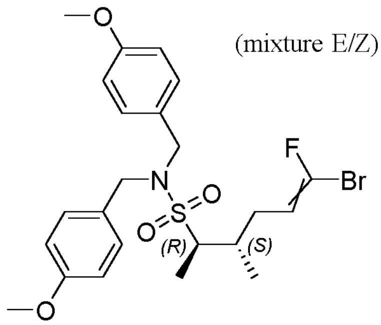

(mixture E/Z)

Diethyl zinc (CAS [557-20-0], 27 mL, 1 M in heptane, 1.4 eq.) was added over 4 h via a syringe pump at room temperature to a stirred solution of Intermediate 8 (8 g, 19.07 mmol), fluorotribromomethane (CAS [353-54-8], 2.8 mL, 1.5 eq.) and triphenylphosphine (CAS [603-35-0], 7.5 g, 1.5 eq.) in dry THF (150 mL). Right after addition, the yellow solution was quenched with MeOH (20 mL) and evaporated under reduced pressure. The residue was purified by column chromatography on silica gel with heptane/EtOAc (1:0 to 4:1) to afford Intermediate 9 (8.4 g, yield: 86 %) as a clear oil (48/52 mixture of Z/E isomers).

## Intermediate 10

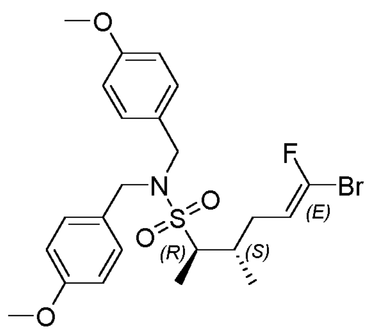

LiHMDS (60 mL, 1 M in THF, 1.1 eq.) was added over 14 h via a syringe pump to a cold (0 °C) stirred solution of

Intermediate 9 (28.0 g, 54.42 mmol) in dry THF (350 mL). After the addition, the mixture was further stirred for 30 min at 0 °C before quenching with water (100 mL). EtOAc (150 mL) was added. The organic layer was extracted, washed with brine (100 mL), dried ($MgSO_4$), filtered, and evaporated under reduced pressure. The residue was purified by column chromatography on silica gel using heptane/EtOAc (1:0 to 4:1) to afford Intermediate 10 (13.72 g, yield: 49 %) as a clear oil with >99:1 E/Z ratio.

**Intermediate 11**

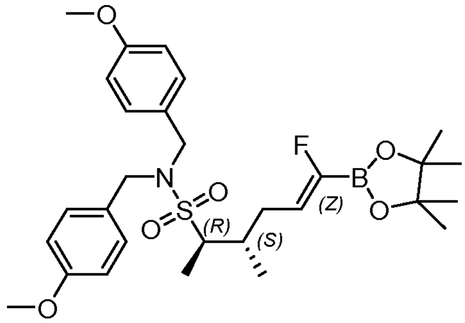

Intermediate 10 (1.55 g, 3.013 mmol) was dissolved in dry 1,4-dioxane (15 mL). This solution was added dropwise over 30min to a degassed solution of bis(pinacolato)diboron (CAS [73183-34-3], 3.1 g, 4 eq.), potassium acetate (900 mg, 3 eq.), and 1,1'-bis(diphenylphosphino)ferrocene-Palladium(II) dichloride dichloromethane complex (CAS [95464-05-4], 250 mg, 0.1 eq.) in dry 1,4-dioxane (45 mL) at 95 °C in a closed vessel. After addition the reaction mixture was stirred at 95 °C for 5 h and then overnight at 25 °C. Dicalite® was added to the reaction mixture and it was filtered. The filtrate was concentrated and the residue was taken up in heptane, stirred for 5 min, then filtered. The filter was washed with heptane. The combined filtrate was concentrated and purified by flash chromatography on silica gel using heptane/EtOAc (from 100:0 to 70:30) as eluent, affording Intermediate 11 (1.4 g, yield: 83 %).

**Intermediate 12**

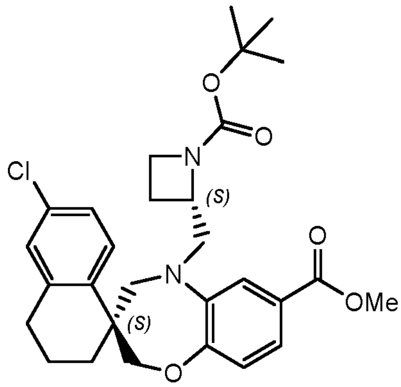

AcOH (12 mL, 25 eq.) was added to a solution of methyl (S)-6'-chloro-3',4,4',5-tetrahydro-2H,2'H-spiro[benzo[b][1,4]oxazepine-3,1'-naphthalene]-7-carboxylate (CAS [1883726-85-9], 3 g, 8.384 mmol) and Intermediate 2 (7.82 g, 3 eq.) in dry DCM (50 mL) at room temperature. After being stirred for 1 h at room temperature, the reaction mixture was cooled to 0 °C. Then, sodium triacetoxyborohydride (890 mg, 0.5 eq.) was added in 9 portions, every 30 min. The reaction mixture was quenched with a solution of saturated NaHCOs, and diluted with EtOAc. Layers were separated, and the organic layer was washed with brine, dried with $MgSO_4$, filtered and solvents were removed under reduced pressure. The residue was purified by flash chromatography on silica gel (eluent: EtOAc/Heptane: 0/100 to 30/70) to afford Intermediate 12 (3.6 g, yield: 73 %) as a white powder.

## Intermediate 13

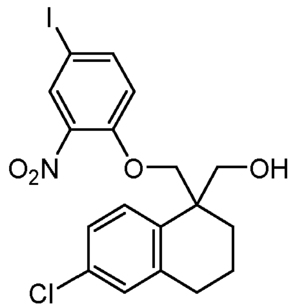

This reaction was performed in two batches. For each batch, (6-chloro-1,2,3,4-tetrahydronaphthalene-1,1-diyl) dimethanol (CAS [1883726-74-6], 300 g, 1.32 mol) and 1-fluoro-4-iodo-2-nitrobenzene (353 g, 1.32 mol) were dissolved in acetonitrile (1.4 L). $K_2CO_3$ (549 g, 3.97 mol) was added to the reaction mixture and it was stirred at 50 °C for 16 h. The reaction mixture was filtered and the filtrate was evaporated. The two batches were then combined and the residue was purified by column chromatography ($SiO_2$, petroleum ether:DCM 3:1 to petroleum ether:EtOAc 1:1). Intermediate 13 was obtained as a yellow oil (600 g, yield: 48 %).

## Intermediate 14

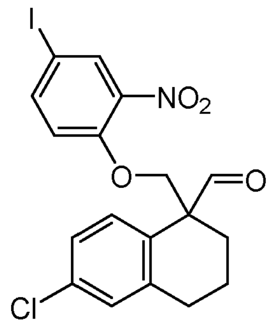

This reaction was performed in three batches. For each batch, DMSO (99.0 g, 1.27 mol) was added to a solution of $(COCl)_2$ (161 g, 1.27 mol) in DCM (2.4 L) at -78 °C. The reaction mixture was stirred at -78 °C for 15 min. Intermediate 13 (200 g, 422 mmol) in DCM (0.90 L) was then added at -78 °C and stirring was continued for 30 min. at -78 °C. $Et_3N$ (214 g, 2.11 mol) was added at -78°C and the reaction mixture was allowed to warm to room temperature. Stirring was continued at room temperature for 1.5 h. Aqueous $NaHCO_3$ (1 L) was added and the mixture was extracted with DCM (0.5 L x 2). The three batches were then combined and evaporated to yield Intermediate 14 (560 g) as a yellow solid, used without further purification.

## Intermediate 15 (and its enantiomer 15')

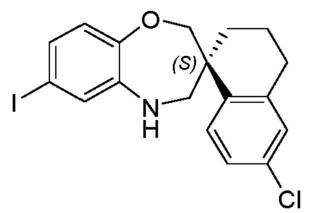

Intermediate 15

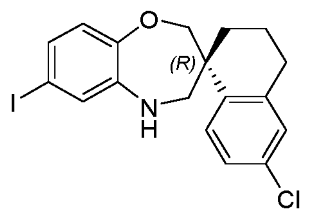

Intermediate 15'

This reaction was performed in three batches. Iron (153 g, 2.75 mol) was added to a solution of Intermediate 14 (185 g, 392 mmol) in AcOH (2.5 L) at 70 °C and the reaction mixture was stirred at 70 °C for 3 h. The solvent was evaporated and DCE (1.9 L) was added to the residue. $NaBH(OAc)_3$ (333 g, 1.57 mol) was then added portionwise at 0°C. Stirring was continued at room temperature for 1 h. The three batches were combined. Citric acid (10 % solution in water, 5 L) was added and the mixture was extracted with DCM (2 L x 2). The combined organic layer was evaporated. The residue was purified by SFC (column: DAICEL CHIRALPAK AD (250 mm*50 mm, 10 um); mobile phase: [0.1 % $NH_3.H_2O$ in EtOH]; B %:

50 % - 50 %, 8.5 min) to afford Intermediate 15 (95.2 g, yield: 40 %) and its enantiomer (105.1 g, yield: 44 %), both as yellow solids.

## Intermediate 16

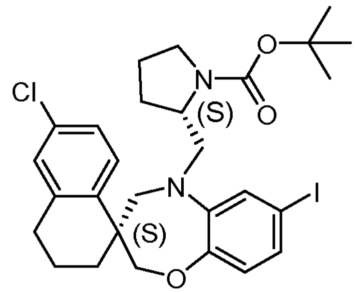

Intermediate 15 (13.197 g, 31 mmol) and N-Boc-L-prolinal (18.53 g, 3 eq.) were dissolved in $CH_2Cl_2$ (150 mL) and then AcOH (35.5 mL, 20 eq.) was added. The mixture was stirred for 30 min at room temperature and then cooled down to 0 °C. Then sodium triacetoxyborohydride (19.711 g, 3 eq.) was added portionwise. After addition the reaction mixture was stirred at room temperature for 18 hours. The reaction mixture was poured out portionwise into a cooled (0 °C) solution of NaOH (31 g) in 620 mL water. After addition the mixture was diluted with $CH_2Cl_2$ and water. The organic layer was separated, washed with water, dried with $MgSO_4$, filtered and the solvents of the filtrate were evaporated. The residue was purified by flash chromatography on silica gel (eluent: $CH_2Cl_2$) The fractions containing product were combined and the solvents were evaporated. This residue was purified again by HPLC (gradient ethylacetate/hexane) to give Intermediate 16 (12.2 g, yield: 64 %).

## Intermediate 17

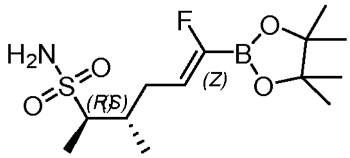

TFA (60 mL, 784 mmol, 28.4 eq.) was added dropwise to a stirred solution of Intermediate 11 (15.5 g, 27.6 mmol) in dry DCM (140 mL) and molecular sieves (15 g). The resulting mixture was stirred at room temperature overnight. The reaction mixture was filtered over celite and the filter pad was washed with DCM. The filtrate was concentrated under reduced pressure and coevaporated 5 times with toluene (10 mL) to afford Intermediate 17 (9.1 g, yield: 82 %) as a yellow solid, used without further purification.

## Intermediate 18

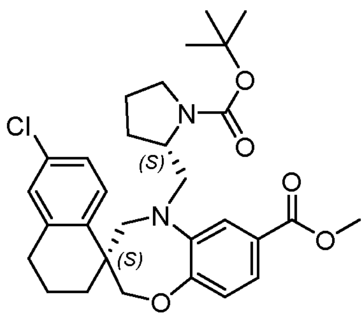

An autoclave was charged with Intermediate 16 (6.7 g, 0.011 mol), MeOH (940 mL), $Et_3N$ (4.87 mL, 0.0351 mol, 3.2 eq.), and THF (174 mL). Then [1,1'-bis(diphenylphosphino)ferrocene]dichloropalladium(II) (CAS [72287-26-4], 1.183 g, 0.00162 mol, 0.15 eq.) was added and the setup was closed. This was flushed once with carbon monoxide and then pressurized with ca 30 bars carbon monoxide. The reaction mixture was heated at 100 °C for 20 h. The reaction mixture was concentrated and the residue was purified twice by chromatography on silicagel (eluent: DCM/MeOH 100/0 to 98/2) to

give Intermediate 18 (5.25 g, yield: 88 %).

## Intermediate 19

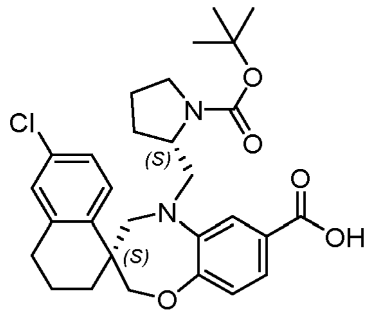

LiOH (1.125 g, 46.976 mmol, 4.8 eq.) was added to a solution of Intermediate 18 (5.25 g, 9.703 mmol) in THF (158 mL), water (158 mL), and MeOH (30 mL). The reaction mixture was stirred at 50 °C for 16 h. The reaction mixture was cooled to 10 °C, acidified to pH = 3-4 with 1 N aqueous HCl, and extracted with DCM. The organic layer was dried over $MgSO_4$, filtered, and evaporated to give Intermediate 19 (5.34 g, quantitative) as an off-white foam.

## Intermediate 20

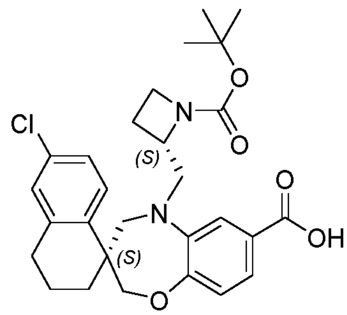

Intermediate 20 was prepared by an analogous reaction protocol as Intermediate 19, starting from Intermediate 12 instead of Intermediate 18.

## Intermediate 21

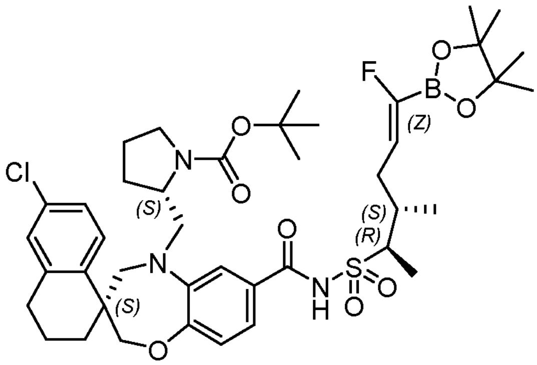

EDCI (600 mg, 3.13 mmol, 2.46 eq.) was added to a mixture of Intermediate 19 (670 mg, 1.271 mmol), Intermediate 17 (480 mg, 1.494 mmol, 1.17 eq.), DMAP (350 mg, 2.865 mmol, 2.25 eq.), and $Et_3N$ (1.25 mL, 8.993 mmol, 7.1 eq.) in DCM (15 mL) at room temperature. The reaction mixture was stirred at room temperature for 24 h. AcOH (1 mL, 17.468 mmol, 13.74 eq.) was added. The solvent was removed under reduced pressure and the residue was purified by flash chromatography on silica gel (heptane/EtOAc 100/0 to 0/100) to give Intermediate 21 (820 mg, 67 % purity), used without further purification.

## Intermediate 22

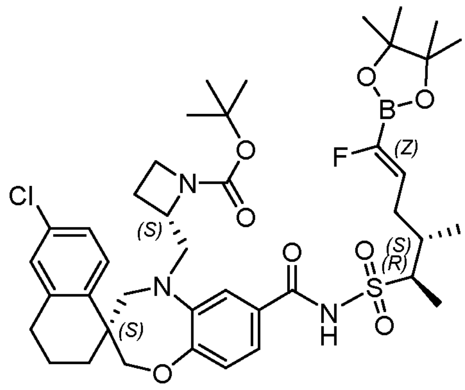

Intermediate 22 was prepared by an analogous reaction protocol as Intermediate 21, starting from Intermediate 20 instead of Intermediate 19.

## Intermediate 23

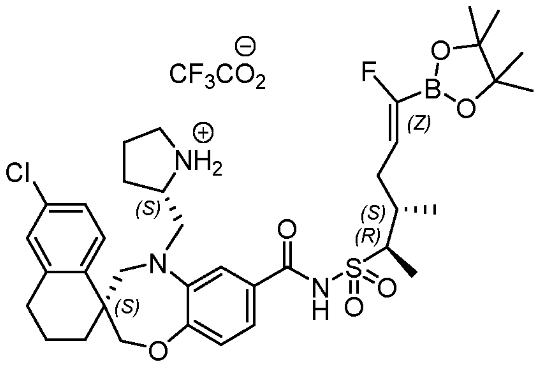

TFA (15 mL, 196 mmol, 81 eq.) was added to a solution of Intermediate 21 (2000 mg, 2.409 mmol) in DCM (30 mL) at room temperature. The reaction mixture was stirred at room temperature for 20 h. The reaction mixture was concentrated under reduced pressure and coevaporated with toluene. The residue was triturated in DIPE. The solid was filtered off, washed, and dried under vacuum at 35 °C to give Intermediate 23 (TFA salt, 1900 mg, yield: 93 %), used without further purification.

## Intermediate 24

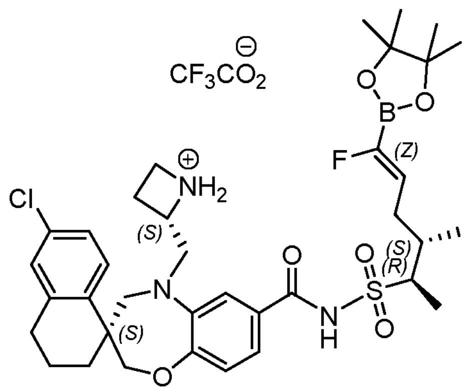

Intermediate 24 was prepared by an analogous reaction protocol as Intermediate 23, starting from Intermediate 22 instead of Intermediate 21.

## Intermediate 25

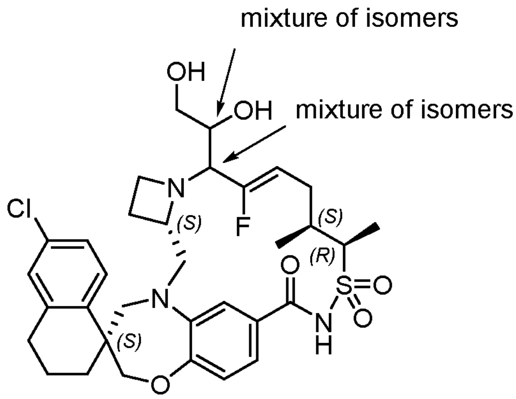


A solution of Intermediate 24 (500 mg, 0.698 mmol) and 3,6-dihydroxy-1,4-dioxane-2,5-dimethanol (CAS [26793-98-6], 252 mg, 1.396 mmol, 2 eq.) in MeOH (14 mL) was stirred at 60 °C for 12 h. The solvent was removed under reduced pressure and the residue was purified by column chromatography (silica 24 g, MeOH/DCM 0/100 to 5/95) to give Intermediate 25 (329 mg, yield: 71 %) as a yellow foam.

## Intermediate 27

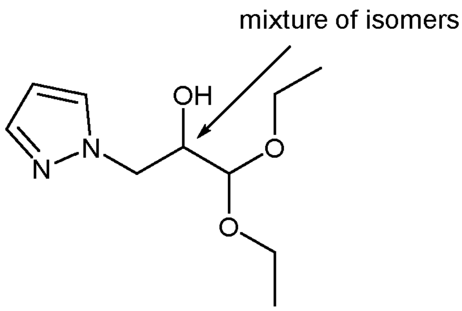


A sealed tube was charged with glycidaldehyde diethylacetal (CAS [13269-77-7], 200 mg, 1.368 mmol), pyrazole (140 mg, 2.052 mmol, 1.5 mmol), $Cs_2CO_3$ (669 mg, 2.052 mmol, 1.5 eq.) in DMF (1 mL). The reaction mixture was stirred at 90 °C for 12 h. After cooling, the mixture was partitioned between water and EtOAc. The layers were separated and the aqueous layer was extracted with EtOAc. The combined organic layer was washed with brine, dried over $MgSO_4$, filtered and evaporated. The residue was purified by column chromatography (silica gel 12 g, EtOAc/heptane 0/100 to 50/50) to afford Intermediate 26 (253 mg, yield: 86 %) as a colourless oil.

## Intermediate 28

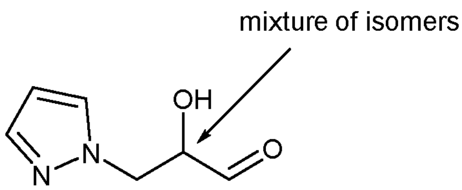


Amberlyst® 15 (hydrogen form, CAS [39389-20-3], 250 mg) was added to a solution of Intermediate 26 (250 mg, 1.167 mmol) in water (3 mL) and acetone (3 mL). The reaction mixture was stirred at room temperature for 6 h. The solid was filtered off through a short pad of Celite® and the filtrate was evaporated to afford Intermediate 27 (160 mg, yield: 98 %) as a white oil, used without further purification.

## Intermediate 29

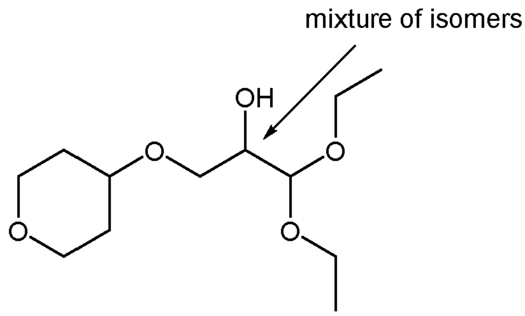


A sealed tube was charged with glycidaldehyde diethylacetal (CAS [13269-77-7], 300 mg, 1.847 mmol), tetrahydro-2H-pyran-4-ol (CAS [2081-44-9], 754 mg, 7.388 mmol, 4 eq.) and KOH (207 mg, 3.694 mmol, 2 eq.) and the reaction mixture was stirred at 70 °C for 2 h. After cooling to room temperature, the reaction mixture was diluted with DCM and water and the layers were separated. The aqueous layer was extracted again with DCM. The combined organic layer was dried on $MgSO_4$, filtered, and evaporated to give a colourless oil. This oil was purified by column chromatography (silica gel 24 g, EtOAc/heptane 0/100 to 100/0) to afford Intermediate 28 (325 mg, yield: 71 %) as a colourless oil.

## Intermediate 30

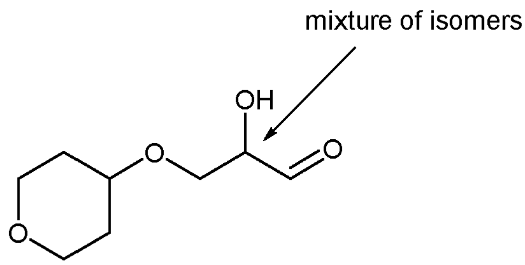


HCl (1 M in water, 2.6 mL, 2.618 mmol, 2 eq.) was added to Intermediate 28 (325 mg, 1.309 mmol) at room temperature and the mixture was stirred at 60 °C for 1 h. The solvent was removed under reduced pressure to give Intermediate 29 (208 mg, yield: 91 %) as a colourless gum, used without further purification.

The following intermediates were synthesized by analogy with Intermediate 30:

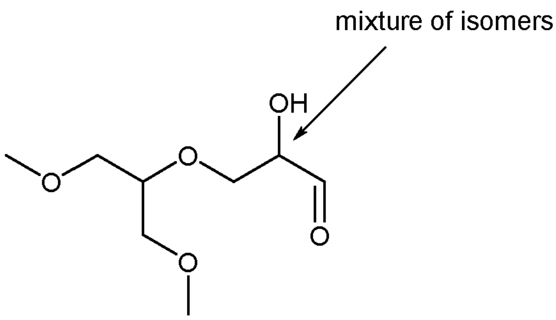


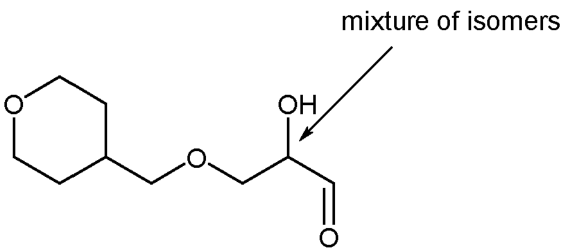

## Intermediate 32

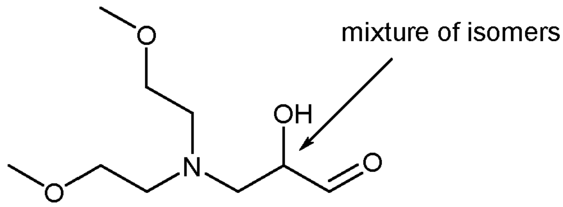


A sealed tube was charged with glycidaldehyde diethylacetal (CAS [13269-77-7], 200 mg, 1.368 mmol), bis(2-methoxyethyl)amine (CAS [111-95-5], 219 mg, 1.642 mmol, 1.2 eq.) in MeOH (2 mL) and the reaction mixture was stirred at 70 °C for 2 h. After cooling to room temperature, the solvent was evaporated. HCl (1 M in water, 1.4 mL, 1.368 mmol, 1 eq.) was added to the residue and the mixture was stirred at 60 °C for 1 h. The solvent was removed under reduced pressure to give Intermediate 31 (250 mg, yield: 89 %), used without further purification.

The following intermediates were synthesized by analogy with Intermediate 32:

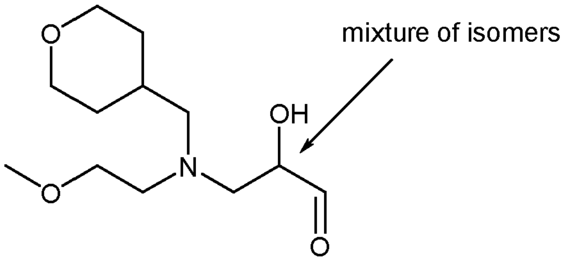


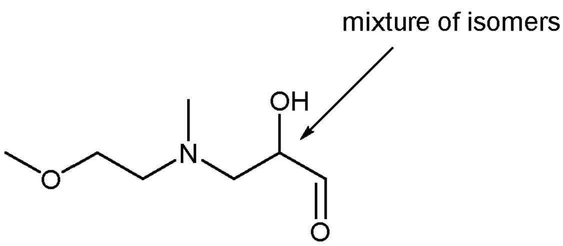


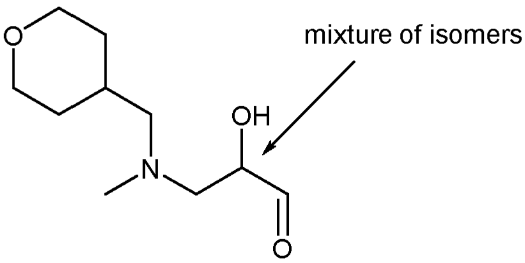


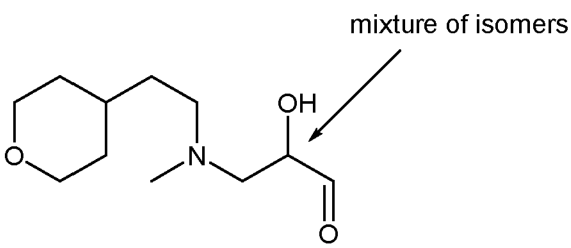

mixture of isomers

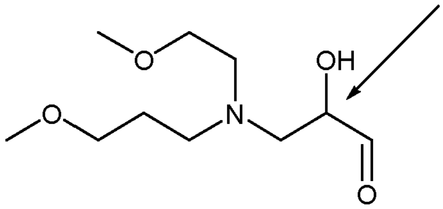

mixture of isomers

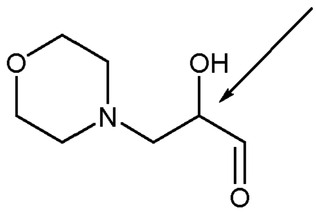

mixture of isomers

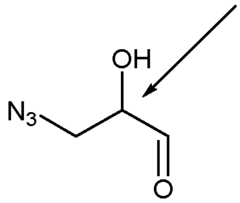

**Intermediate 31**

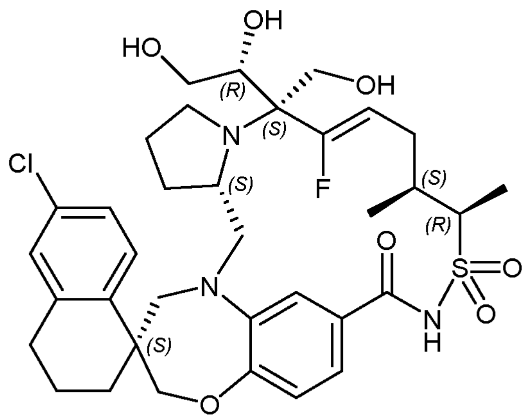

A solution of Intermediate 23 (50 mg, 0.059 mmol) and fresh Z-(+)-erythrulose (CAS [533-50-6], 20 mg, 0.167 mmol, 2.8 eq.) in MeOH (3 mL) and DCM (2 mL) was stirred for 5 days at 60 °C. After cooling to room temperature, the reaction mixture was diluted with DCM and water. The mixture was carefully acidified with 1 M aqueous HCl to reach pH ca 5-6. The organic layer was separated and the aqueous layer was back-extracted twice with DCM. The combined organic layer was dried ($MgSO_4$), filtered, and evaporated. The residue was purified by column chromatography on silica gel (DCM/MeOH 100/0 to 95/5) to yield Intermediate 30 (16 mg, yield: 38 %) as an off-white solid.

## Intermediate 32

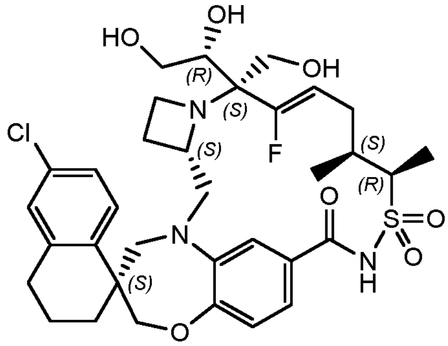

Intermediate 24 (25 mg, 0.035 mmol) and fresh Z-(+)-erythrulose (10 mg, 0.083 mmol, 2.4 eq.) were stirred in MeOH (1 mL) and DCM (0.5 mL) at 60 °C for 15 h. The reaction mixture was cooled to room temperature and diluted with DCM and water. The mixture was carefully acidified to pH 5-6 with 1 M aqueous HCl. The layers were separated and the aqueous layer was extracted again twice with DCM. The combined organic layer was dried on $MgSO_4$, filtered, and evaporated. The residue was purified by column chromatography on silica gel (DCM/MeOH 100/0 to 95/5) to afford Intermediate 32 (20 mg, yield: 85 %) as a white solid.

## Intermediate 33

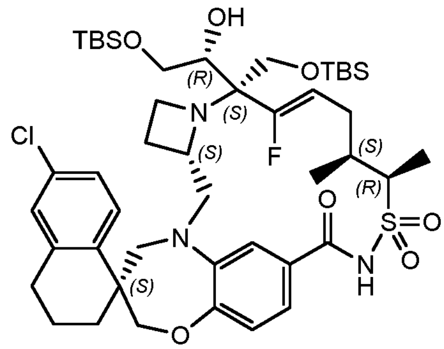

TBSCl (CAS [18162-48-6], 71.9 mg, 0.477 mmol) was added portionwise to a stirred solution of Intermediate 32 (150 mg, 0.217 mmol) and imidazole (CAS [288-32-4], 59.0 mg, 0.867 mmol) in 10 mL of anhydrous DCM. The resulting mixture was stirred at room temperature overnight. Solvent were evaporated under reduced pressure. The crude was directly subjected to column chromatography on silica gel with Heptane/EtOAc (1:0 to 75:25) to afford Intermediate 33 (180 mg, yield 90%) as a white solid.

## Intermediate 34

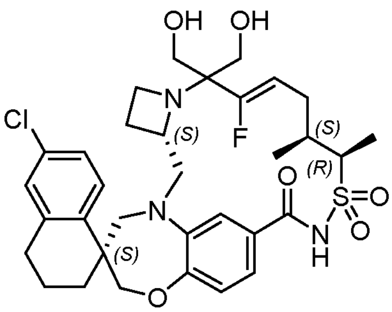

Intermediate 24 (140 mg, 0.123 mmol) was suspended in 10 mL of a 9:1 mixture of MeOH/DCM, followed by addition of triethylamine (88.2 μL, 0.728 g/mL, 0.635 mmol) and 1,3-dihydroxyacetone (CAS [96-26-4], 52.92 mg, 0.587 mmol). The resulting mixture was stirred 1 h at room temperature. Volatiles were evaporated under reduced pressure. The crude was dissolved in DCM (30 mL) followed by addition of water (10 mL). The pH was then carefully adjusted to ca 5-6. The organic layer was separated and the aqueous layer was back-extracted with DCM (10 mL). The combined organic

layers were dried ($MgSO_4$), filtered off and evaporated under reduced pressure. The crude was purified by column chromatography on silica gel with DCM/MeOH (1:0 to 95:5) to get Intermediate 34 (42 mg, yield 51%) as a white solid.

## Intermediate 35

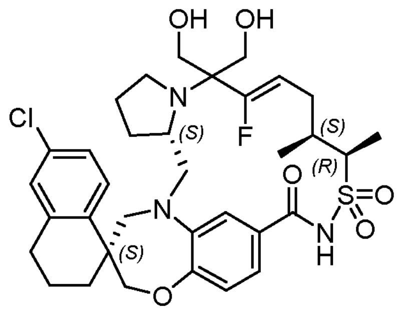

Intermediate 35 was prepared as an analogous maneer as Intermediate 34, using Intermediate 23 as starting material.

## Intermediate 36

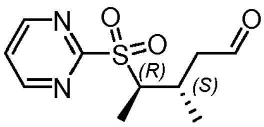

2-(((2R,3S)-3-Methylhex-5-en-2-yl)sulfonyl)pyrimidine (CAS [1638587-10-6], 2.2 g, 9.154 mmol) was dissolved in DCM (55 mL) and the resulting solution was cooled to - 78 °C. Ozone (440 mg, 9.154 mmol) was subsequently bubbled through until a grey-blue persistent colour was observed (after 25 min). Nitrogen was then bubbled through the solution (still at -78 °C). This was followed by addition of triphenylphosphine (3.4 g, 12.96 mmol). The reaction mixture was stirred over the weekend at room temperautre. The reaction mixture was concentrated under reduced pressure. The crude product was purified by flash column chromatography on silica gel (heptane:EtOAc - 1:0 to 0:1) to get Intermediate 36 (3 g, 66 % yield, estimated purity 50% due to presence of triphenylphosphine oxide).

## Intermediate 37

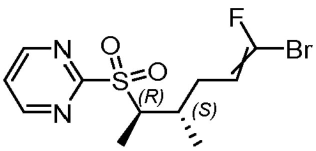

Diethylzinc (1 M in heptane) (CAS [557-20-0], 6.9 mL, 1 M, 6.9 mmol) was added dropwise via syringe pump over 2 h to a stirred solution of Intermediate 36 (1.4 g, 3.467 mmol), fluorotribromomethane (CAS [353-54-8], 0.7 mL, 2.765 g/mL, 7.149 mmol) and triphenylphosphine (2 g, 7.625 mmol) in anhydrous THF (30 mL). The solution was quenched 30 min. after addition with 2 mL of MeOH and evaporated under reduced pressure. The crude was subjected to column chromatography with heptane/EtOAc (1:0 to 4:1) to afford Intermediate 37 (500 mg, yield 43%) as an E/Z mixture.

## Intermediate 38

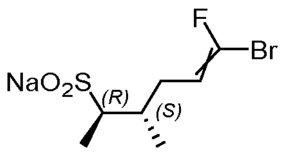

Intermediate 37 (500 mg, 1.483 mmol) was dissolved in methanol (3.5 mL), and to this solution NaOMe (30% in MeOH) (0.275 mL, 1.483 mmol) was added dropwise at 0°C, while stirring. The mixture was stirred for 15 min at 0°C and then for 30 min at room temperature. The solvent was removed by evaporation. The residue was dissolved in water (10 mL), the side product was extracted with EtOAc. The water layer was used as such in the next step without further

purification.

## Intermediate 39

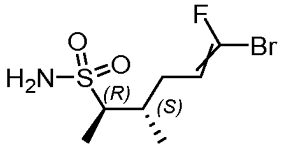

Intermediate 38 in water was stirred at 5 °C. A solution of sodium acetate (170 mg, 2.072 mmol) and hydroxylamine-O-sulfonic acid (235 mg, 2.078 mmol) in water (10 mL) was added to the reaction mixture whilst keeping the temperature below 10 °C. This suspension was stirred at 20 °C for 16 h. The pH of the reaction mixture was adjusted to pH=6 with NaHCOs solid using a the pH-meter (OptiMax apparatus, Mettler-Toledo AG) whilst keeping the temperature below 10 °C. The product was extracted with 3 x 15 mL MTBE. The combined organic layers were washed with brine and then dried with $Na_2SO_4$, filtered and concentrated to get Intermediate 39 (340 mg, 83%).

From Intermediate 39, Intermediate 9 can be obtained by PMB protection following the procedure reported for Intermediate 43.

## Intermediate 40

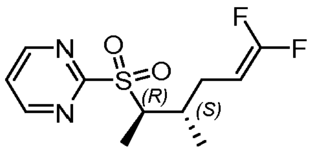

Sodium chlorodifluoroacetate (2.0 g, 13.118 mmol) was added to a stirred solution of Intermediate 36 (49% pure) (3.0 g, 6.067 mmol) and triphenylphosphine (3.8 g, 14.488 mmol) in 60 mL of anhydrous DMF. The resulting mixture was then heated to 100 °C for 3 h. More sodium chlorodifluoroacetate (1.0 g, 6.559 mmol) was added at 100 °C, and the reaction mixutre was stirred for 2 more h at 100 °C.After cooling to 0 °C, water was carefully added. The mixture was extracted with $Et_2O$ (2 x 100 mL), the combined organic layers were washed with water (50 mL) and brine (50 mL), then dried ($MgSO_4$), filtered off and evaporated under reduced pressure. The crude was purified by column chromatography on silica gel with Heptane/EtOAc (100:0 to 70:30) to afford Intermediate 40 (700 mg, yield 41 %) .

## Intermediate 41

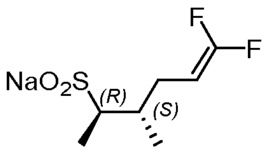

Intermediate 40 (700 mg, 2.533 mmol) was dissolved in methanol (6 mL) and to this solution NaOMe (30% in MeOH) (0.47 mL, 2.538 mmol) was added dropwise at 0 °C, while stirring. The reaction mixture was stirred for 15 min at 0 °C and then for 30 min at 20°C.The solvent was removed by evaporation. The residue was dissolved in water (15 mL), the side product was washed away with EtOAc (2 x 10 mL) and the water layer was used as such in the next step without further purification.

## Intermediate 42

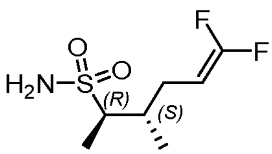

Intermediate 41 in water was stirred at 5 °C. A solution of sodium acetate (300 mg, 3.657 mmol) and hydroxylamine-O-sulfonic acid (410 mg, 3.625 mmol) in water (15 mL) was added to the reaction mixture, while keeping the temperature below 10 °C. This reaction mixture was stirred at 20 °C for 16 h. The pH of the mixture was adjusted to

pH=6 with NaHCOs solid while keeping the temperature below 10 °C. MTBE was added. The organic layer was separated and the aqueous layer was back-extracted with MTBE (x3). The combined organic layers were washed with brine and then dried with $Na_2SO_4$, filtered and concentrated to yield Intermediate 42 (465 mg, 86%).

## Intermediate 43

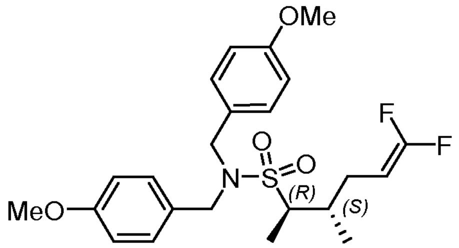

To a stirred solution of Intermediate 42 (460 mg, 2.157 mmol) in DMF (4 mL) was added potassium carbonate (1.2 g, 8.683 mmol) followed by dropwise addition of 4-methoxybenzyl chloride (0.9 mL, 1.155 g/mL, 6.637 mmol). The resulting suspension was stirred at 70 °C during 18 h. After cooling to room temperature, solids were filtered off through a pad of celite and washed with EtOAc. The filtrate was evaporated under reduced pressure to remove most of the DMF. Then, the yellow residue was taken up in 100 mL of EtOAc and washed with brine (2 x 10 mL). The organic layer was dried ($MgSO_4$), filtered off and evaporated under reduced pressure.The residue was purified by flash column chromatography on silica gel (40 g Redisep flash column eluting with 0-40% EtOAc in heptane) to afford Intermediate 43 (870 mg, 80%) as a clear oil.

From Intermediate 43, Intermediate 11 can be obtained according to the following procedure:

Copper(I) chloride (1.096 mg, 0.0111 mmol) was placed in a 2 mL crimped vial equipped with a stir bar together with Xanthphos (4,5-bis(diphenylphosphino)-9,9-dimethylxanthene (CAS [161265-03-8]), 6.404 mg, 0.0111 mmol). The vial was sealed and purged ($N_2$, 3 exchanges), then dried and degassed. 1,2-dimethoxyethane (1.107 mL, 0.1 M, 0.111 mmol) was added and the reaction mixture was heated to 40 °C and aged for 1 h at this temperature. Intermediate 43 (50.2 mg, 0.111 mmol) was placed in a 40 mL vial equipped with a stir bar then bis(pinacolato)diboron (67.457 mg, 0.266 mmol) was added. Vial was sealed and purged ($N_2$, 3 exchanges), then the cooled (rt) CuCl/Xantphos/DME mixture was added under inert atmosphere to the substrates vial.

The crimped vial was rinsed (2 x 50 μL DME) and the rinsates were added to the main reaction vial. Dried and degassed MeOH (8.967 μL, 0.791 g/mL, 0.221 mmol) was added in a single addition to the reaction mixture then tBuOK (221 μL, 1 M, 0.221 mmol) was added dropwise over 10 minutes at rt. The reaction was aged at rt for 2 h, then was heated to 40 °C and aged at this temperature. After 16 h, the reaction was cooled to rt then a saturated aqueous NaHCOs solution (10 mL) followed by EtOAc (10 mL) were added to the mixture. The crude mixture was poored into a separatory funnel and phases were separated. Aqueous phase was extracted 3 times with EtOAc (10 mL), organics were combined, washed (brine), dried ($MgSO_4$) then solvents removed under reduced pressure. Crude material was purified over silica gel eluting heptane/EtOAc and to afford Intermediate 11 (31.2 mg, 48%) as a colorless oil.

## Intermediate 44

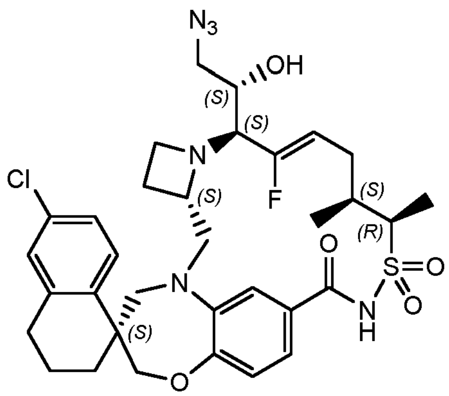

To the solution of Intermediate 24 (400 mg, 0.352 mmol) in 10 mL MeOH and 5 mL DCM was added Et3N [121-44-8] (244.567 μL, 0.728 g/mL, 1.759 mmol) and 3-azido-2-hydroxypropanal(162.001 mg, 1.408 mmol) at rt. The reaction was heated to 40°C for 16 hours. The reaction mixture was diluted with $CH_3CN$ and purified by RP-HPLC

separation [Condition: (solid phase: C18 column, mobile phase: $CH_3CN+NH_4OAc$ solution)]. The desired fractions were collected and concentrated to remove organic solvent. Then aqueous mixture was extracted with EtOAc (20mLx3), organic layers were combined, washed with brine and concentrated under reduced pressure to give brownish oil. The residue was purified by column chromatography, silica gel 40g, eluted with 0-5% MeOH in DCM to give Intermediate 44 (214 mg, yield 80%) as yellowish solid.

**Preparation of Compounds**

Compound 1

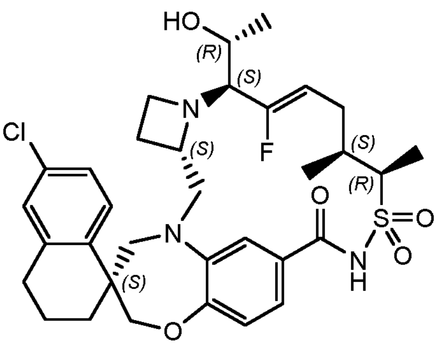

Intermediate 24 (50 mg, 0.050 mmol) and (2R)-2-hydroxypropanal (0.101 mL, 1 M in water, 0.101 mmol, 2 eq.) were dissolved in MeOH (0.5 mL) and the reaction mixture was stirred at 60 °C for 12 h. The solvent was removed under reduced pressure and the residue was purified by column chromatography (silica 24 g, MeOH/DCM 0/100 to 5/95) to give Compound 1 (15 mg, yield: 33 %) as a colourless solid.

$^1$H NMR (400 MHz, CHLOROFORM-*d*) δ ppm 0.01 - 0.08 (m, 1 H) 1.08 (d, J=6.38 Hz, 3 H) 1.18 (d, J=6.16 Hz, 3 H) 1.47 (d, J=7.26 Hz, 4 H) 1.77 - 1.88 (m, 1 H) 1.91 - 2.11 (m, 6 H) 2.29 - 2.39 (m, 1 H) 2.71 - 2.84 (m, 3 H) 3.10 (dd, J=15.41, 9.90 Hz, 1 H) 3.19 - 3.26 (m, 1 H) 3.26 - 3.37 (m, 2 H) 3.64 - 3.89 (m, 5 H) 4.05 - 4.17 (m, 2 H) 4.21 (q, J=7.19 Hz, 1 H) 4.79 - 4.94 (m, 1 H) 6.90 - 7.00 (m, 3 H) 7.10 (d, J=2.20 Hz, 1 H) 7.17 - 7.25 (m, 1 H) 7.69 (d, J=8.58 Hz, 1 H); LCMS confirms the MW (RT: 1.10, $[M+H]^+$ 646, Method 1).

Compound 2

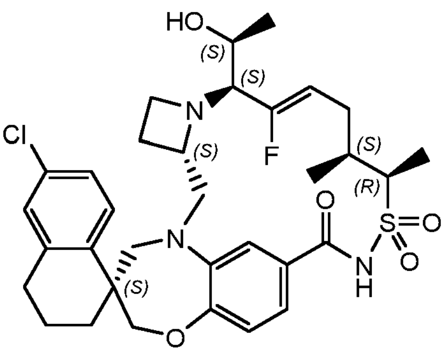

Compound 2 was prepared by an analogous reaction protocol as Compound 1, starting from (2S)-2-hydroxypropanal instead of (2R)-2-hydroxypropanal.

$^1$H NMR (400 MHz, CHLOROFORM-*d*) δ ppm 1.07 (d, J=6.38 Hz, 3 H) 1.30 (d, J=6.27 Hz, 3 H) 1.45 (d, J=7.21 Hz, 3 H) 1.78 - 1.88 (m, 2 H) 1.90 - 2.11 (m, 7 H) 2.31 - 2.41 (m, 2 H) 2.70 - 2.85 (m, 2 H) 2.99 (dd, J=31.51, 7.16 Hz, 1 H) 3.06 (br s, 1 H) 3.11 (br dd, J=15.36, 9.93 Hz, 1 H) 3.23 (br d, J=14.21 Hz, 1 H) 3.29 (br t, J=6.69 Hz, 1 H) 3.36 - 3.49 (m, 1 H) 3.61 - 3.78 (m, 3 H) 3.78 - 3.86 (m, 2 H) 4.02 - 4.15 (m, 2 H) 4.18 (q, J=6.90 Hz, 1 H) 4.73 - 5.01 (m, 1 H) 6.94 (s, 2 H) 7.09 (d, J=2.30 Hz, 1 H) 7.14 - 7.22 (m, 2 H) 7.69 (d, J=8.47 Hz, 1 H); LCMS confirms the MW (RT: 1.11, $[M+H]^+$ 646, Method 1)

## Compound 3

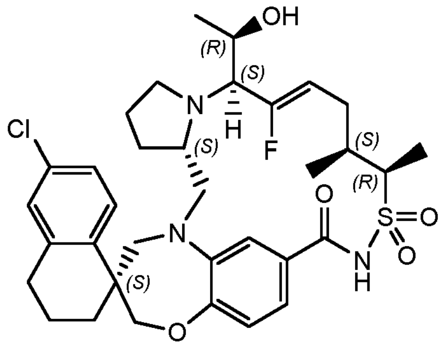

Intermediate 23 (40 mg, 0.055 mmol), (2R)-2-hydroxypropanal (164 μL, 1 M solution in water, 0.164 mmol, 3 eq.) and $Et_3N$ (8 μL, 0.055 mmol, 1 eq.) were stirred in MeOH (2 mL) at 60 °C for 24 h. The solvent was then evaporated under reduced pressure and the residue was purified by preparative HPLC (Stationary phase: RP XBridge Prep C18 OBD - 5 μm, 50 x 250 mm, Mobile phase: 0.25 % $NH_4HCO_3$ solution in water, MeOH) to afford Compound 3 (7 mg, yield: 19 %) as a white solid.

$^1$H NMR (400 MHz, CHLOROFORM-*d*) δ ppm 1.07 (d, J=6.4 Hz, 3 H) 1.28 (br d, J=5.9 Hz, 3 H) 1.44 (d, J=7.3 Hz, 4 H) 1.59 - 1.75 (m, 4 H) 1.76 - 1.87 (m, 1 H) 1.91 - 2.00 (m, 2 H) 2.00 - 2.14 (m, 3 H) 2.15 - 2.26 (m, 1 H) 2.72 - 2.89 (m, 4 H) 2.89 - 2.99 (m, 1 H) 3.11 (dd, J=28.5, 9.7 Hz, 1 H) 3.20 - 3.29 (m, 1 H) 3.33 (d, J=14.3 Hz, 1 H) 3.87 - 4.05 (m, 4 H) 4.05 - 4.15 (m, 1 H) 4.22 (d, J=12.1 Hz, 1 H) 4.62 - 4.86 (m, 1 H) 6.93 (s, 2 H) 7.02 - 7.13 (m, 2 H) 7.20 (dd, J=8.6, 2.2 Hz, 1 H) 7.70 (d, J=8.4 Hz, 1 H); $^{19}$F NMR (377 MHz, CHLOROFORM-*d*) δ ppm -111.62 - -110.20 (m, 1 F); LCMS confirms the MW (RT: 2.13, $[M+H]^+$ 660, Method 5).

## Compound 4

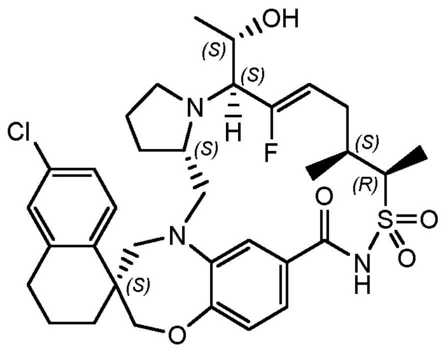

Intermediate 23 (150 mg, 0.192 mmol), (2S)-2-hydroxypropanal (600 μL, 1 M solution in water, 0.600 mmol, 3.1 eq.) and $Et_3N$ (27 μL, 0.192 mmol, 1 eq.) were stirred in MeOH (5 mL) at 30 °C for 1.5 h. The solvent was then evaporated under reduced pressure and the residue was purified by column chromatography on silica gel (DCM/MeOH 100/0 to 98/2) followed by preparative SFC (Stationary phase: Chiralpak Diacel AD 20 x 250 mm, Mobile phase: $CO_2$, EtOH) to afford Compound 4 (39 mg, yield: 31 %) as a white solid.

$^1$H NMR (400 MHz, CHLOROFORM-*d*) δ ppm 1.05 (d, J=6.4 Hz, 3 H) 1.37 (d, J=6.2 Hz, 3 H) 1.43 (d, J=7.3 Hz, 4 H) 1.54 - 1.70 (m, 3 H) 1.76 - 1.91 (m, 2 H) 1.91 - 2.10 (m, 5 H) 2.19 - 2.29 (m, 1 H) 2.69 - 2.88 (m, 4 H) 2.89 - 2.98 (m, 1 H) 3.13 - 3.25 (m, 1 H) 3.26 - 3.36 (m, 2 H) 3.87 - 4.05 (m, 3 H) 3.97 - 4.04 (m, 1 H) 4.12 - 4.24 (m, 2 H) 4.65 - 4.86 (m, 1 H) 6.94 (d, J=0.7 Hz, 2 H) 7.04 (s, 1 H) 7.09 (d, J=2.2 Hz, 1 H) 7.20 (dd, J=8.5, 2.3 Hz, 1 H) 7.70 (d, J=8.6 Hz, 1 H); LCMS confirms the MW (RT: 2.29, [M+H]+ 660, Method 3).

## Compound 5 and Compound 6

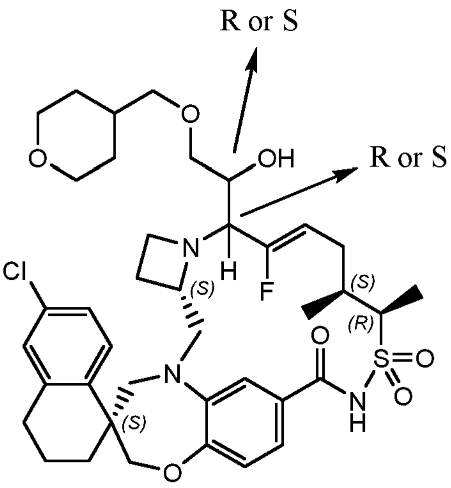


Compound 5

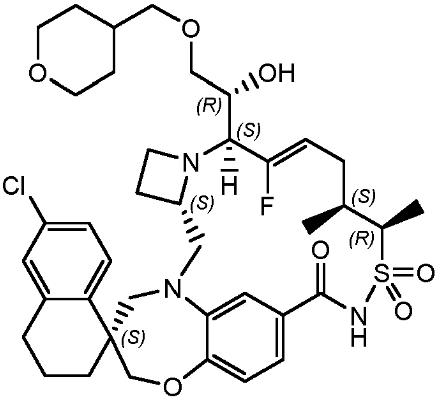

Compound 6

NaH (60 % dispersion in mineral oil, 31 mg, 0.761 mmol, 3 eq.) was added to a solution of Intermediate 25 (168 mg, 0.254 mmol) in DMF (3 mL) at room temperature and the reaction mixture was stirred at room temperature for 30 min. 4-(bromomethyl)tetrahydropyran (CAS [125552-89-8], 136 mg, 0.761 mmol, 3 eq.) was added and the mixture was stirred at 80 °C for 4 h. The reaction mixture was cooled and the reaction was quenched with water then acidified with 1 N HCl. The mixture was extracted with EtOAc. The organic layer was washed with brine, dried on $MgSO_4$, filtered, and evaporated. The residue was purified by column chromatography (silicagel, eluent: DCM/MeOH 100/0 to 90/10) followed by preparative SFC (Stationary phase: Chiralcel Diacel IH 20 x 250 mm, Mobile phase: $CO_2$, EtOH + 0.4 % $iPrNH_2$) to afford Compound 5 (5 mg, yield: 3 %) and Compound 6 (4 mg, yield: 2 %).

Compound 5: $^1$H NMR (400 MHz, CHLOROFORM-*d*) δ ppm 1.05 - 1.09 (m, 3 H) 1.31 - 1.34 (m, 2 H) 1.38 - 1.42 (m, 3 H) 1.50 - 1.65 (m, 3 H) 1.72 - 1.84 (m, 2 H) 1.88 - 2.11 (m, 7 H) 2.31 - 2.39 (m, 1 H) 2.72 - 2.81 (m, 2 H) 3.05 - 3.43 (m, 10 H) 3.45 - 3.51 (m, 1 H) 3.64 - 3.78 (m, 3 H) 3.83 - 3.98 (m, 4 H) 4.02 - 4.14 (m, 3 H) 4.74 - 4.94 (m, 1 H) 6.89 - 6.95 (m, 1 H) 6.96 - 7.04 (m, 2 H) 7.06 - 7.12 (m, 1 H) 7.14 - 7.21 (m, 1 H) 7.65 - 7.72 (m, 1 H); LCMS confirms the MW (RT: 2.17, [M+H]+ 760, Method 3); SFC (Rt 6.00min 100.00% isomer 1), (Rt 6.62 min 0.00% isomer 2) (RT: 6.00, $[M+H]^+$ 760, Method 11).

Compound 6: $^1$H NMR (400 MHz, CHLOROFORM-*d*) δ ppm 1.05 - 1.10 (m, 3 H) 1.32 - 1.34 (m, 3 H) 1.44 - 1.51 (m, 3 H) 1.60 - 1.74 (m, 4 H) 1.86 - 1.95 (m, 2 H) 1.99 - 2.11 (m, 5 H) 2.19 - 2.25 (m, 1 H) 2.33 - 2.41 (m, 1 H) 2.76 - 2.81 (m, 1 H) 2.99 - 3.14 (m, 2 H) 3.17 - 3.26 (m, 3 H) 3.31 - 3.44 (m, 5 H) 3.58 - 3.81 (m, 6 H) 3.91 - 4.01 (m, 2 H) 4.04 - 4.23 (m, 3 H) 4.73 - 4.94 (m, 1 H) 6.91 - 6.97 (m, 2 H) 6.97 - 7.01 (m, 1 H) 7.06 - 7.12 (m, 1 H) 7.15 - 7.22 (m, 1 H) 7.67 - 7.71 (m, 1 H); LCMS confirms the MW (RT: 2.20, $[M+H]^+$ 760, Method 3); SFC (Rt 6.00 min 1.69% isomer 1), (Rt 6.62 min 98.31% isomer 2) manual integration (RT: 6.62, $[M+H]^+$ 760, Method 11).

## Compound 7

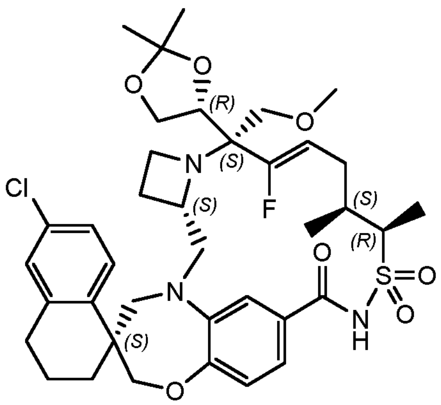

Fresh *L*-(+)-erythrulose (CAS [533-50-6], 5 mg, 0.041 mmol, 2.3 eq.) was added to a stirred solution of Intermediate 24 (13 mg, 0.018 mmol) in MeOH (1 mL) and DCM (500 μL). The resulting mixture was stirred at 50 °C

for 24 h. Solvents were evaporated under reduced pressure and the residue was co-evaporated with DCM. The residue was suspended in acetone (1 mL), and PTSA (1 mg, 0.006 mmol, 0.3 eq.) was added. The resulting solution was stirred at room temperature for 24 h. The reaction mixture was diluted with EtOAc and saturated aqueous $NaHCO_3$. The organic layer was separated and washed twice with saturated aqueous NaHCOs followed by brine, dried on $MgSO_4$, filtered, and evaporated under reduced pressure.

The crude alcohol obtained was dissolved in dry THF (1 mL), and NaH (60 % dispersion in mineral oil, 3 mg, 0.075 mmol, 4 eq.) was added. After 20 min of stirring at room temperature, MeI (10 μL, 0.161 mmol, 8.9 eq.) was added in one portion and the resulting mixture was stirred at 40 °C for 1 h. The reaction mixture was diluted with EtOAc and water. The organic layer was separated, washed with brine, dried on $MgSO_4$, filtered, and evaporated under reduced pressure. The residue was purified by two successive column chromatography on silica gel (heptane/EtOAc (first 100:0 to 0:100, then 100:0 to 50/50) to afford Compound 7 (4 mg, yield: 29 %) as a white solid.

$^1H$ NMR (400 MHz, CHLOROFORM-*d*) δ ppm 1.08 (d, J=5.9 Hz, 3 H) 1.34 (s, 3 H) 1.36 - 1.42 (m, 1 H) 1.43 - 1.47 (m, 3 H) 1.49 (d, J=7.0 Hz, 3 H) 1.80 - 2.04 (m, 5 H) 2.06 - 2.19 (m, 2 H) 2.42 - 2.56 (m, 1 H) 2.70 - 2.86 (m, 2 H) 3.16 (dd, J=14.9, 10.2 Hz, 1 H) 3.21 - 3.28 (m, 1 H) 3.28 - 3.40 (m, 1 H) 3.48 (dd, J=10.0, 2.8 Hz, 1 H) 3.51 (s, 3 H) 3.66 (d, J=9.9 Hz, 1 H) 3.73 (q, J=8.4 Hz, 1 H) 3.80 (br d, J=14.3 Hz, 1 H) 3.88 (q, J=8.1 Hz, 1 H) 3.93 - 3.98 (m, 1 H) 4.04 - 4.14 (m, 3 H) 4.20 - 4.29 (m, 2 H) 4.40 (t, J=6.5 Hz, 1 H) 5.07 - 5.24 (m, 1 H) 6.84 - 6.90 (m, 1 H) 6.91 - 6.94 (m, 1 H) 7.09 (d, J=2.2 Hz, 1 H) 7.19 (dd, J=8.5, 2.3 Hz, 1 H) 7.33 (d, J=1.5 Hz, 1 H) 7.70 (d, J=8.6 Hz, 1 H) 7.84 - 8.18 (m, 1 H); LCMS confirms the MW (RT: 2.10, $[M+H]^+$ 746, Method 5).

## Compound 8

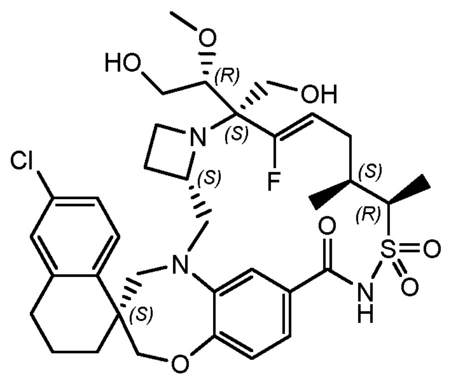

NaH (60% dispersion in mineral oil, 5.2 mg, 0.13 mmol) was added to a stirred solution of Intermediate 33 (40 mg, 0.0434 mmol) in 5 mL of anhydrous THF. After 10 min, MeI (30 μL, 2.28 g/mL, 0.482 mmol) was added and the mixture was heated at 50 °C during 18 h. Upon completion, the mixture was cooled to room temperature and quenched with few drops of MeOH, followed by addition of TBAF (1 M in THF, 200 μL, 0.2 mmol). The resulting mixture was heated at 50 °C and stirred 2 d.

Upon cooling to room temperature, the mixture was quenched with water and extracted with EtOAc. The organic layer was washed with brine, dried ($MgSO_4$), filtered off and evaporated under reduced pressure. A purification was performed via Prep HPLC (Stationary phase: RP XBridge Prep C18 OBD-10μm, 30x150mm, Mobile phase: 0.5% $NH_4OAc$ solution in water + 10% $CH_3CN$, $CH_3CN$) to yield Compound 8 (6.1 mg, yield 20%) as an off-white solid.

$^1H$ NMR (400 MHz, CHLOROFORM-*d*) δ ppm 1.09 (br d, J=6.2 Hz, 3 H) 1.49 (br d, J=7.1 Hz, 4 H) 1.79 - 1.90 (m, 2 H) 1.92 - 2.02 (m, 4 H) 2.03 - 2.11 (m, 2 H) 2.14 - 2.22 (m, 1 H) 2.31 - 2.39 (m, 1 H) 2.51 (br dd, J=14.0, 9.5 Hz, 1 H) 2.74 - 2.84 (m, 2 H) 3.16 (br dd, J=14.8, 10.4 Hz, 1 H) 3.26 - 3.37 (m, 2 H) 3.43 (s, 3 H) 3.53 - 3.58 (m, 1 H) 3.71 - 3.76 (m, 1 H) 3.81 (br d, J=12.3 Hz, 2 H) 3.89 (t, J=5.1 Hz, 2 H) 3.92 - 3.97 (m, 1 H) 3.98 - 4.02 (m, 1 H) 4.02 - 4.05 (m, 1 H) 4.10 - 4.17 (m, 1 H) 4.25 - 4.37 (m, 1 H) 5.02 - 5.19 (m, 1 H) 6.83 - 6.98 (m, 2 H) 7.10 (d, J=2.2 Hz, 1 H) 7.12 - 7.23 (m, 2 H) 7.68 (d, J=8.5 Hz, 1 H); LCMS confirms the MW (RT: 1.94, $[M+H]^+$ 706, Method 3).

## Compound 9 and Compound 10

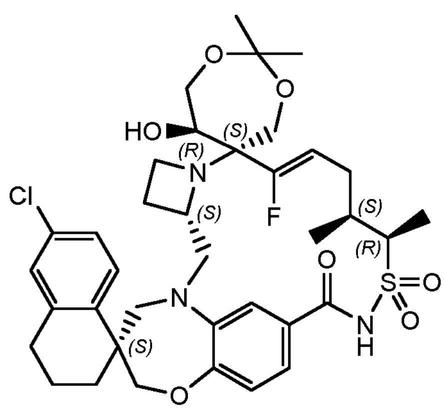

Compound 9

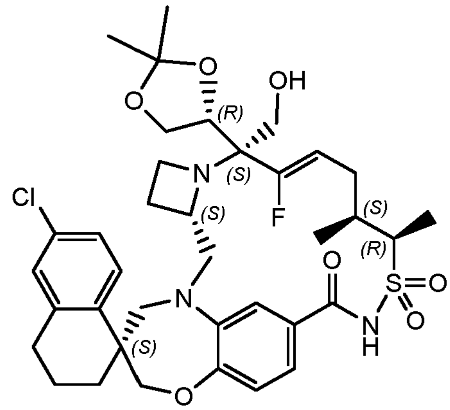

Compound 10

PTSA (0.2 mg, 0.001 mmol, 0.05 eq.) was added to a stirred solution of Intermediate 32 (13 mg, 0.019 mmol) in acetone (2 mL) and the resulting mixture was stirred at room temperature for 1 h. The reaction mixture was diluted with EtOAc and saturated aqueous $NaHCO_3$. The organic layer was separated, washed with brine, dried ($MgSO_4$), filtered, and evaporated under reduced pressure. The residue was purified by column chromatography on silica gel (heptane/EtOAc (100/0 to 50/50) to give a yellow solid. This solid was further purified by preparative HPLC (Stationary phase: RP XBridge Prep C18 OBD - 5 μm,50 x 250 mm, Mobile phase: 0.25 % $NH_4HCO_3$ solution in water, $CH_3CN$) to afford Compound 9 (3.6 mg, yield: 26 %) and Compound 10 (7.4 mg, yield: 54 %) as white solids.

Compound 9: $^1$H NMR (400 MHz, CHLOROFORM-*d*) δ ppm 1.06 (br d, J=5.7 Hz, 3 H) 1.33 - 1.37 (m, 1 H) 1.40 (s, 6 H) 1.45 (br d, J=7.0 Hz, 3 H) 1.85 - 2.02 (m, 6 H) 2.07 - 2.13 (m, 1 H) 2.43 (br dd, J=13.9, 7.9 Hz, 1 H) 2.74 - 2.83 (m, 2 H) 3.08 (br dd, J=15.3, 10.5 Hz, 1 H) 3.18 (br t, J=6.8 Hz, 1 H) 3.33 (br d, J=14.3 Hz, 1 H) 3.39 - 3.47 (m, 1 H) 3.50 (dd, J=12.1, 3.7 Hz, 1 H) 3.59 - 3.69 (m, 2 H) 3.75 - 3.88 (m, 3 H) 4.01 - 4.14 (m, 3 H) 4.30 - 4.40 (m, 1 H) 4.65 - 4.86 (m, 1 H) 5.33 - 5.52 (m, 1 H) 6.83 - 7.01 (m, 2 H) 7.10 (d, J=2.2 Hz, 1 H) 7.12 - 7.15 (m, 1 H) 7.19 (dd, J=8.6, 1.8 Hz, 1 H) 7.70 (d, J=8.6 Hz, 1 H); LCMS confirms the MW (RT: 2.03, $[M+H]^+$ 732, Method 4).

Compound 10: $^1$H NMR (400 MHz, CHLOROFORM-*d*) δ ppm 1.04 (br d, J=6.4 Hz, 3 H) 1.40 (br d, J=39.0 Hz, 10 H) 2.02 (s, 6 H) 2.06 - 2.12 (m, 1 H) 2.17 - 2.29 (m, 1 H) 2.47 (br dd, J=14.2, 9.8 Hz, 1 H) 2.70 - 2.88 (m, 2 H) 3.18 - 3.35 (m, 3 H) 3.78 (br s, 4 H) 3.90 - 4.14 (m, 6 H) 4.18 - 4.28 (m, 1 H) 4.32 - 4.44 (m, 1 H) 4.88 - 5.13 (m, 1 H) 5.37 - 5.65 (m, 1 H) 6.78 - 6.93 (m, 1 H) 6.94 - 7.03 (m, 1 H) 7.09 (d, J=2.2 Hz, 1 H) 7.18 (dd, J=8.5, 2.1 Hz, 1 H) 7.22 (s, 1 H) 7.70 (d, J=8.4 Hz, 1 H); LCMS confirms the MW (RT: 1.17, $[M+H]^+$ 732, Method 2).

## Compound 11

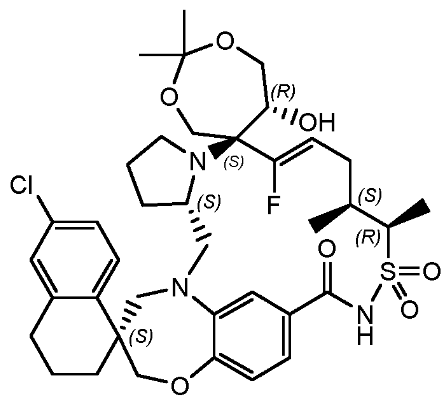

PTSA (0.2 mg, 0.001 mmol, 0.05 eq.) was added to a stirred solution of Intermediate 30 (16 mg, 0.023 mmol) in acetone (2 mL). The resulting mixture was stirred at room temperature for 3 h. The reaction mixture was diluted with EtOAc and saturated aqueous $NaHCO_3$. The organic layer was separated, washed with brine, dried ($MgSO_4$), filtered, and evaporated. The residue was purified by column chromatography on silica gel (heptane/EtOAc 100/0 to 50/50) followed by preparative HPLC (Stationary phase: RP XBridge Prep C18 OBD - 5 μm, 50 x 250 mm, Mobile phase: 0.25 % $NH_4HCO_3$ solution in water, $CH_3CN$) to afford Compound 11 (6 mg, yield: 37 %) as a white solid.

$^1$H NMR (400 MHz, CHLOROFORM-*d*) δ ppm 0.99 - 1.11 (m, 3 H) 1.35 - 1.47 (m, 10 H) 1.63 - 1.73 (m, 2 H) 1.77 - 1.86 (m, 3 H) 1.92 - 2.11 (m, 5 H) 2.36 - 2.52 (m, 1 H) 2.72 - 2.86 (m, 2 H) 2.87 - 3.07 (m, 2 H) 3.22 - 3.31 (m, 1 H) 3.32 - 3.46 (m, 1 H) 3.60 - 3.88 (m, 4 H) 3.90 - 3.98 (m, 1 H) 4.00 - 4.28 (m, 5 H) 4.53 (br s, 2 H) 5.35 - 5.52 (m, 1 H) 6.75 - 7.00 (m, 2 H) 7.10 (s, 1 H) 7.16 - 7.26 (m, 2 H) 7.62 - 7.76 (m, 1 H); LCMS confirms the MW (RT: 2.10, $[M+H]^+$ 746, Method 3).

## Compound 12

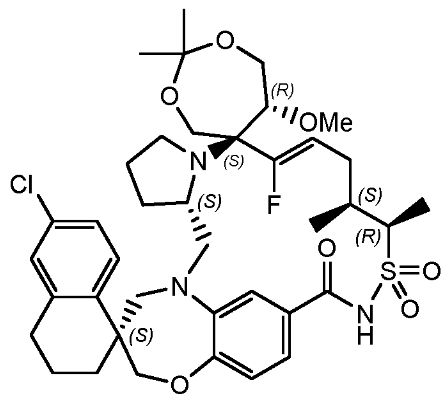

NaH (60% dispersion in mineral oil) (CAS [7646-69-7], 4.019 mg, 0.1 mmol) was added to a stirred solution of Compound 11 (25 mg, 0.0335 mmol) in 5 mL of anhydrous THF.
The resulting mixture was stirred 10 min at room temperature, followed by addition of MeI (20 μL, 2.28 g/mL, 0.321 mmol). The mixture was then stirred 18 h at 40 °C.
The mixture was diluted with DCM and quenched with sat. NaHCOs sol. The organic layer was separated and the aqueous layer was back-extracted with DCM (x3). The combined dried (MgSO4) organic layers were evaporated under reduced pressure. A purification was performed via Prep HPLC (Stationary phase: RP XBridge Prep C18 OBD-10μm, 30x150mm, Mobile phase: 0.25% $NH_4HCO_3$ solution in water, CH3CN) yielding Compound 12 (12 mg, yield 47%) as an off-white solid.
$^1$H NMR (400 MHz, CHLOROFORM-*d*) δ ppm 1.06 (d, J=6.4 Hz, 3 H) 1.46 (br d, J=18.3 Hz, 9 H) 1.52 - 1.66 (m, 3 H) 1.71 - 1.91 (m, 3 H) 1.92 - 2.02 (m, 3 H) 2.03 - 2.08 (m, 1 H) 2.28 (br dd, J=15.8, 7.7 Hz, 1 H) 2.71 (br dd, J=15.0, 10.3 Hz, 1 H) 2.75 - 2.82 (m, 2 H) 2.82 - 2.89 (m, 1 H) 2.95 - 3.09 (m, 2 H) 3.24 - 3.35 (m, 2 H) 3.52 (s, 3 H) 3.72 - 3.84 (m, 2 H) 3.91 - 4.01 (m, 2 H) 4.01 - 4.13 (m, 2 H) 4.13 - 4.25 (m, 2 H) 4.43 - 4.59 (m, 1 H) 4.66 - 4.92 (m, 1 H) 6.91 (s, 3 H) 7.07 - 7.13 (m, 1 H) 7.18 - 7.24 (m, 1 H) 7.70 (d, J=8.4 Hz, 1 H); LCMS confirms the MW (RT: 2.16, $[M+H]^+$ 760, Method 4).

## Compound 13

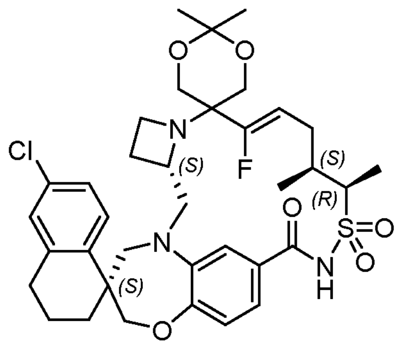

PTSA (1.1 mg, 0.00634 mmol) was added to a stirred solution of Intermediate 34 (42 mg, 0.0634 mmol) in 9 mL of acetone and 1 mL of 2,2-dimethoxypropane. The resulting mixture was stirred 4 d at room temperature. Volatiles were evaporated under reduced pressure. The crude was taken up in EtOAc, followed by addition of sat. NaHCOs sol. The organic layer was separated, washed with brine, dried ($MgSO_4$), filtered off and evaporated under reduced pressure. The crude was purified by column chromatography on silica gel with Hept/EtOAc (1:0 to 3:2) to afford Compound 13 (31 mg, yield 70%) as a white solid.
1H NMR (400 MHz, CHLOROFORM-*d*) δ ppm 1.08 (d, J=6.2 Hz, 3 H) 1.36 - 1.47 (m, 7 H) 1.48 (d, J=7.0 Hz, 3 H) 1.61 - 1.75 (m, 1 H) 1.78 - 1.89 (m, 1 H) 1.91 - 2.02 (m, 4 H) 2.03 - 2.10 (m, 1 H) 2.11 - 2.21 (m, 1 H) 2.49 (br dd, J=13.9, 9.5 Hz, 1 H) 2.78 (br d, J=4.6 Hz, 2 H) 3.21 - 3.38 (m, 3 H) 3.53 - 3.62 (m, 1 H) 3.67 (d, J=11.4 Hz, 1 H) 3.72 - 3.77 (m, 1 H) 3.78 -

3.94 (m, 3 H) 4.00 - 4.19 (m, 3 H) 4.26 - 4.38 (m, 1 H) 5.10 - 5.28 (m, 1 H) 6.85 - 7.04 (m, 3 H) 7.09 (d, J=2.2 Hz, 1 H) 7.19 (dd, J=8.5, 2.3 Hz, 1 H) 7.69 (d, J=8.6 Hz, 1 H) 7.94 - 8.51 (m, 1 H); LCMS confirms the MW (RT: 2.06, $[M+H]^+$ 702, Method 6).

## Compound 14

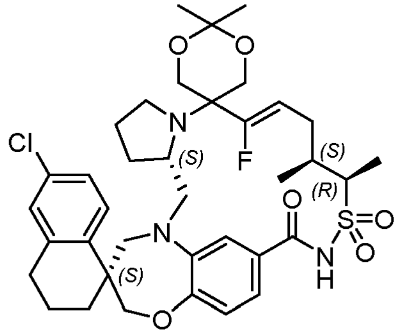

Compound 14 was prepared in an analogous manner as Compound 13, using Intermediate 35 as starting material.

$^1H$ NMR (400 MHz, CHLOROFORM-*d*) δ ppm 0.78 - 0.93 (m, 1 H) 1.03 - 1.08 (m, 3 H) 1.45 - 1.50 (m, 6 H) 1.53 - 1.68 (m, 5 H) 1.69 - 1.81 (m, 3 H) 1.85 - 1.97 (m, 3 H) 1.98 - 2.06 (m, 1 H) 2.39 - 2.51 (m, 1 H) 2.67 - 2.82 (m, 2 H) 2.83 - 2.92 (m, 1 H) 2.99 - 3.14 (m, 2 H) 3.19 - 3.27 (m, 1 H) 3.47 - 3.55 (m, 1 H) 3.70 - 3.79 (m, 1 H) 3.88 - 4.00 (m, 3 H) 4.02 - 4.15 (m, 2 H) 4.17 - 4.26 (m, 2 H) 4.30 - 4.40 (m, 1 H) 5.12 - 5.35 (m, 1 H) 6.80 - 6.87 (m, 1 H) 6.88 - 6.96 (m, 1 H) 7.06 - 7.14 (m, 2 H) 7.17 - 7.23 (m, 1 H) 7.63 - 7.71 (m, 1 H) 7.83 - 8.17 (m, 1 H); LCMS confirms the MW (RT: 1.29, $[M+H]^+$ 716, Method 1).

## Compounds 15 and 16

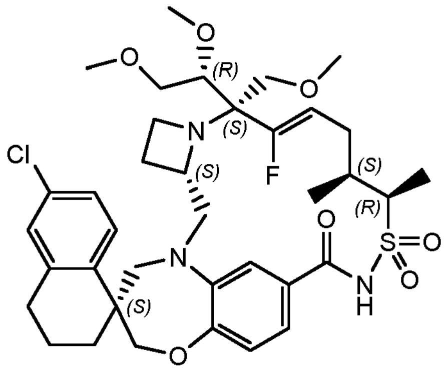

Compound 15

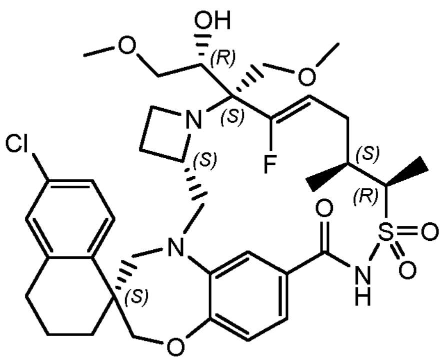

Compound 16

NaH (60% dispersion in mineral oil, 40 mg, 1 mmol) was added to a stirred solution of Intermediate 32 (80 mg, 0.116 mmol) in 5 mL of THF. The resulting mixture was stirred at room temperature for 10 min before addition of MeI (50 μL, 2.28 g/mL, 0.803 mmol). The mixture was heated at 50 °C for 3 h. Upon cooling to room temperature, the mixture was quenched with MeOH and evaporated under reduced pressure. The crude was subjected to column chromatography on silica gel with DCM/MeOH (1:0 to 98:2) to afford Compound 15 (47.4 mg, yield 56%) as a white solid. The second fraction containing many products (based on LCMS mono-, di- and some tri-protected) was purified via Prep HPLC (Stationary phase: RP XBridge Prep C18 OBD-10μm, 30x150mm, Mobile phase: 0.5% $NH_4OAc$ solution in water + 10% $CH_3CN$, $CH_3CN$) to yield **Compound 16** (9.1 mg, yield 11%) as a white solid.

Compound 15: $^1H$ NMR (400 MHz, CHLOROFORM-*d*) δ ppm 1.07 (d, J=6.3 Hz, 3 H) 1.31 - 1.41 (m, 1 H) 1.48 (d, J=7.2 Hz, 3 H) 2.05 (s, 6 H) 2.07 - 2.19 (m, 2 H) 2.50 (dd, J=14.5, 9.8 Hz, 1 H) 2.70 - 2.87 (m, 2 H) 3.15 (dd, J=15.0, 10.2 Hz, 1 H) 3.25 - 3.31 (m, 1 H) 3.33 (d, J=14.3 Hz, 1 H) 3.40 (s, 3 H) 3.46 (s, 3 H) 3.48 (s, 3 H) 3.51 - 3.56 (m, 1 H) 3.57 - 3.65 (m, 2 H) 3.66 - 3.75 (m, 1 H) 3.76 - 3.87 (m, 3 H) 3.95 - 4.17 (m, 3 H) 4.21 - 4.31 (m, 1 H) 5.12 (ddd, J=40.2, 9.8, 2.4 Hz, 1 H) 6.87 - 6.96 (m, 2 H) 7.09 (d, J=2.2 Hz, 1 H) 7.19 (dd, J=8.5, 2.2 Hz, 1 H) 7.25 - 7.27 (m, 1 H) 7.70 (d, J=8.6 Hz, 1 H) 7.96 - 8.38 (m, 1 H); LCMS confirms the MW (RT: 2.29, $[M+H]^+$ 734, Method 3).

Compound 16: $^1H$ NMR (400 MHz, CHLOROFORM-*d*) δ ppm 1.09 (d, J=6.0 Hz, 3 H) 1.33 - 1.42 (m, 1 H) 1.48 (d, J=7.2 Hz, 3 H) 1.80 - 1.89 (m, 1 H) 1.89 - 2.01 (m, 4 H) 2.02 - 2.12 (m, 2 H) 2.46 (br dd, J=14.1, 8.9 Hz, 1 H) 2.70 - 2.86 (m, 2 H) 3.09 (dd, J=14.9, 10.4 Hz, 1 H) 3.30 (d, J=14.2 Hz, 1 H) 3.33 - 3.38 (m, 1 H) 3.39 (s, 3 H) 3.49 - 3.62 (m, 5 H) 3.64 - 3.85 (m,

5 H) 3.87 - 3.96 (m, 1 H) 3.97 - 4.14 (m, 3 H) 4.20 - 4.31 (m, 1 H) 5.21 - 5.35 (m, 1 H) 6.86 - 6.96 (m, 2 H) 7.10 (d, J=2.3 Hz, 1 H) 7.19 (dd, J=8.5, 2.3 Hz, 1 H) 7.31 (d, J=1.5 Hz, 1 H) 7.69 (d, J=8.6 Hz, 1 H); LCMS confirms the MW (RT: 1.99, $[M+H]^+$ 720, Method 3).

**Compound 17** and **Compound 18**

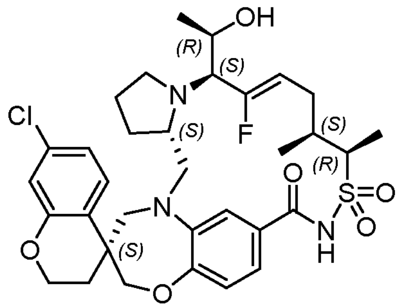

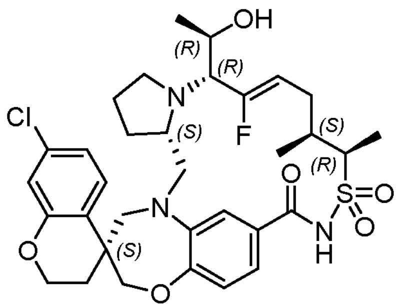

**Compounds 17** and **18** was prepared by an analogous reaction protocol as Compound 3, using (2*R*)-2-hydroxypropanal as aldehyde source.

Compound 17: $^1$H NMR (400 MHz, CHLOROFORM-*d*) δ ppm 1.03 - 1.10 (m, 3 H) 1.34 (d, J=6.2 Hz, 4 H) 1.43 - 1.50 (m, 3 H) 1.53 - 1.70 (m, 3 H) 1.72 - 1.86 (m, 3 H) 1.91 - 2.05 (m, 3 H) 2.13 - 2.21 (m, 2 H) 2.78 - 2.94 (m, 2 H) 3.07 - 3.16 (m, 1 H) 3.19 - 3.29 (m, 1 H) 3.32 - 3.39 (m, 1 H) 3.73 - 3.78 (m, 1 H) 3.98 - 4.03 (m, 1 H) 4.04 - 4.19 (m, 4 H) 4.20 - 4.27 (m, 1 H) 4.28 - 4.36 (m, 1 H) 4.92 - 5.12 (m, 1 H) 6.82 - 6.87 (m, 1 H) 6.89 - 6.98 (m, 3 H) 7.28 - 7.33 (m, 1 H) 7.54 - 7.61 (m, 1 H); LCMS confirms the MW (RT: 1.95, $[M+H]^+$ 662, Method 3).

Compound 18: $^1$H NMR (400 MHz, CHLOROFORM-*d*) δ ppm 1.01 -1.10 (m, 3 H) 1.24 - 1.30 (m, 4 H) 1.40 - 1.49 (m, 3 H) 1.53 - 1.73 (m, 3 H) 1.74 - 1.85 (m, 1 H) 1.91 - 2.10 (m, 4 H) 2.14 - 2.23 (m, 1 H) 2.67 - 2.82 (m, 1 H) 2.82 - 2.97 (m, 2 H) 2.99 - 3.14 (m, 1 H) 3.17 - 3.31 (m, 1 H) 3.33 - 3.47 (m, 1 H) 3.84 - 3.93 (m, 1 H) 3.95 - 4.04 (m, 2 H) 4.05 - 4.11 (m, 1 H) 4.11 - 4.20 (m, 2 H) 4.20 - 4.25 (m, 1 H) 4.31 - 4.37 (m, 1 H) 4.57 - 4.79 (m, 1 H) 6.83 - 6.87 (m, 1 H) 6.91 - 6.99 (m, 3 H) 7.02 - 7.08 (m, 1 H) 7.50 - 7.72 (m, 1 H); LCMS confirms the MW (RT: 2.06, $[M+H]^+$ 662, Method 3).

**Compound 19**

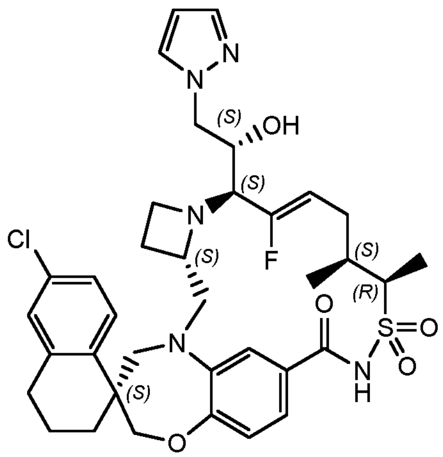

**Intermediate 28** (39 mg, 0.279 mmol, 4 eq.) and $Et_3N$ (19 μL, 0.14 mmol, 2 eq.) were added to a solution of Intermediate 24 (50 mg, 0.070 mmol) in MeOH (1 mL) at room temperature. The reaction mixture was stirred at 40 °C overnight. The solvent was evaporated under reduced pressure and the residue was purified by column chromatography (silica gel 12 g, EtOAc/heptane 0/100 to 50/50) followed by preparative SFC (Stationary phase: Chiralcel Diacel OJ 20 x 250 mm, Mobile phase: $CO_2$, MeOH + 0.4 % $iPrNH_2$) to give Compound 19 (8 mg, yield: 16 %).

$^1$H NMR (400 MHz, CHLOROFORM-*d*) δ ppm 1.04 (br d, J=4.81 Hz, 3 H) 1.41 (br d, J=7.00 Hz, 3 H) 1.82 - 2.10 (m, 6 H) 2.37 (br dd, J=13.80, 8.15 Hz, 1 H) 2.63 - 2.91 (m, 4 H) 3.05 (br dd, J=15.47, 10.24 Hz, 1 H) 3.16 - 3.29 (m, 2 H) 3.39 (br s, 1 H) 3.73 (br d, J=13.69 Hz, 3 H) 3.94 - 4.14 (m, 5 H) 4.29 (br dd, J=13.74, 6.43 Hz, 1 H) 4.58 (br d, J=13.48 Hz, 1 H) 4.84 (br dd, J=38.77, 6.27 Hz, 1 H) 6.30 (t, J=2.04 Hz, 1 H) 6.91 (br d, J=8.05 Hz, 2 H) 6.99 (br d, J=7.21 Hz, 1 H) 7.10 (d, J=2.19 Hz, 1 H) 7.18 (dd, J=8.41, 1.83 Hz, 1 H) 7.50 (d, J=2.09 Hz, 1 H) 7.57 (s, 1 H) 7.69 (d, J=8.57 Hz, 1 H); LCMS confirms the MW (RT: 2.07, $[M+H]^+$ 712, Method 3); SFC RT: 3.62, Area %: 100.00, [M+H]+ 712, Method 12).

The following compounds were prepared by an analogous reaction protocol as Compound 19,

| Compound | Structure | Analytical data |
|---|---|---|
| **Compound 20** | 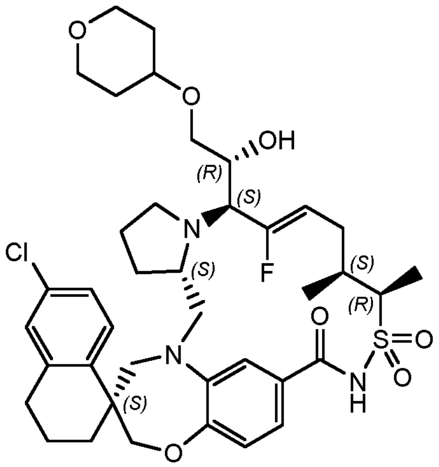 | $^1$H NMR (400 MHz, CHLOROFORM-*d*) δ ppm 1.06 (d, J=6.16 Hz, 3 H) 1.44 (d, J=7.26 Hz, 3 H) 1.53 - 2.33 (m, 15 H) 2.52 (br s, 1 H) 2.65 - 2.86 (m, 4 H) 2.91 (br d, J=3.08 Hz, 1 H) 3.23 (br d, J=6.60 Hz, 1 H) 3.31 (br d, J=14.30 Hz, 1 H) 3.42 - 3.51 (m, 2 H) 3.60 (tt, J=8.67, 4.21 Hz, 1 H) 3.72 (br dd, J=12.43, 3.63 Hz, 2 H) 3.89 - 4.09 (m, 6 H) 4.14 - 4.28 (m, 2 H) 4.55 - 4.77 (m, 1 H) 6.86 - 6.91 (m, 1 H) 6.93 - 6.97 (m, 1 H) 7.06 (d, J=1.54 Hz, 1 H) 7.10 (d, J=1.98 Hz, 1 H) 7.15 - 7.25 (m, 1 H) 7.70 (d, J=8.58 Hz, 1 H); LCMS confirms the MW (RT: 1.15, $[M+H]^+$ 746, Method 1). |
| **Compound 21** | 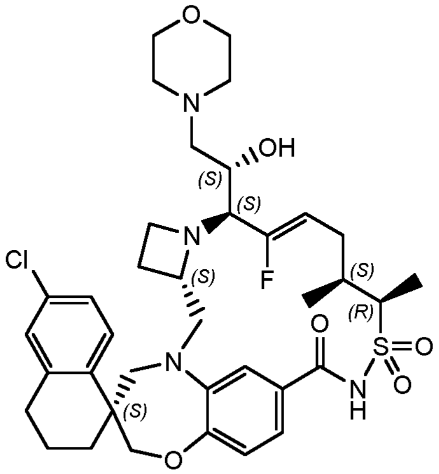 | $^1$H NMR (400 MHz, CHLOROFORM-*d*) δ ppm 1.02 - 1.12 (m, 3 H) 1.44 (d, J=7.21 Hz, 3 H) 1.80 - 1.87 (m, 1 H) 1.89 - 2.11 (m, 5 H) 2.33 - 2.57 (m, 4 H) 2.60-2.84 (m, 6 H) 2.95 (br d, J=8.15 Hz, 1 H) 3.01 - 3.11 (m, 2 H) 3.16 - 3.26 (m, 2 H) 3.36 - 3.48 (m, 1 H) 3.64 - 3.86 (m, 8 H) 4.00 - 4.14 (m, 2 H) 4.19 (br d, J=7.11 Hz, 1 H) 4.87 (br dd, J=37.99, 6.11 Hz, 1 H) 6.89 - 7.01 (m, 3 H) 7.09 (d, J=2.19 Hz, 1 H) 7.18 (dd, J=8.47, 2.30 Hz, 1 H) 7.69 (d, J=8.47 Hz, 1 H); LCMS confirms the MW (RT: 1.09, $[M+H]^+$ 731, Method 1). |
| **Compound 22** | 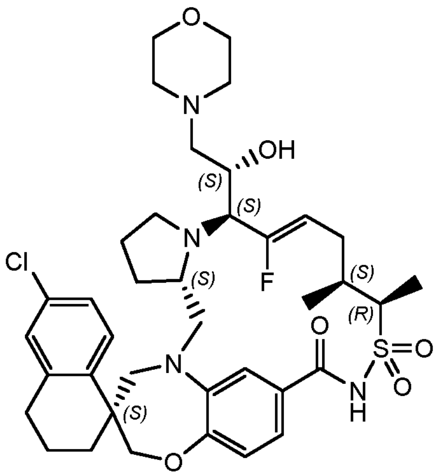 | $^1$H NMR (400 MHz, CHLOROFORM-*d*) δ ppm 1.04 (br d, J=6.16 Hz, 3 H) 1.35 - 1.46 (m, 3 H) 1.51 - 1.68 (m, 3 H) 1.79 - 1.98 (m, 4 H) 2.02 - 2.12 (m, 3 H) 2.17 - 2.30 (m, 1 H) 2.46 - 2.63 (m, 3 H) 2.66 - 2.84 (m, 7 H) 2.97 (br d, J=7.70 Hz, 1 H) 3.20 - 3.38 (m, 3 H) 3.69 - 3.83 (m, 4 H) 3.85 - 4.04 (m, 4 H) 4.18 (br d, J=12.32 Hz, 2 H) 4.68 - 4.90 (m, 1 H) 5.30 (s, 1 H) 6.89 - 6.98 (m, 2 H) 7.03 (br s, 1 H) 7.06 - 7.11 (m, 1 H) 7.20 (dd, J=8.47, 2.09 Hz, 1 H) 7.70 (d, J=8.36 Hz, 1 H); LCMS confirms the MW (RT: 1.16, $[M+H]^+$ 745, Method 1). |

(continued)

| Compound | Structure | Analytical data |
|---|---|---|
| **Compound 23** | 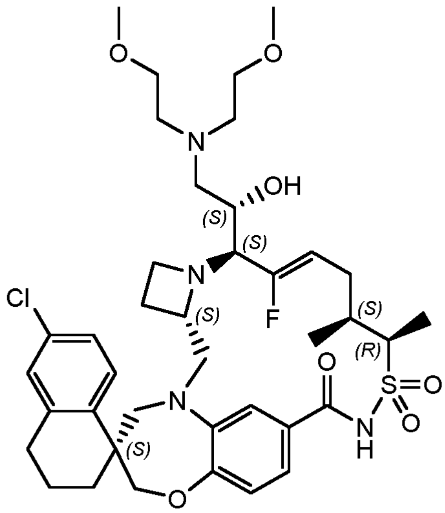 | $^1$H NMR (400 MHz, CHLOROFORM-*d*) δ ppm 1.05 (br d, J=5.85 Hz, 3 H) 1.43 (br d, J=7.11 Hz, 3 H) 1.77 - 2.13 (m, 6 H) 2.32 - 2.43 (m, 2 H) 2.51 - 2.64 (m, 2 H) 2.69 - 3.12 (m, 10 H) 3.13 - 3.29 (m, 2 H) 3.37 (s, 6 H) 3.42 (br s, 1 H) 3.46 - 3.56 (m, 4 H) 3.59 - 3.86 (m, 4 H) 4.00 - 4.12 (m, 2 H) 4.15 (br d, J=8.36 Hz, 1 H) 4.79 - 5.01 (m, 1 H) 6.89 - 6.94 (m, 1 H) 6.94 - 7.04 (m, 2 H) 7.09 (d, J=2.19 Hz, 1 H) 7.18 (dd, J=8.47, 2.19 Hz, 1 H) 7.69 (d, J=8.47 Hz, 1 H); LCMS confirms the MW (RT: 2.15, $[M+H]^+$ 777, Method 3). |
| **Compound 24** | 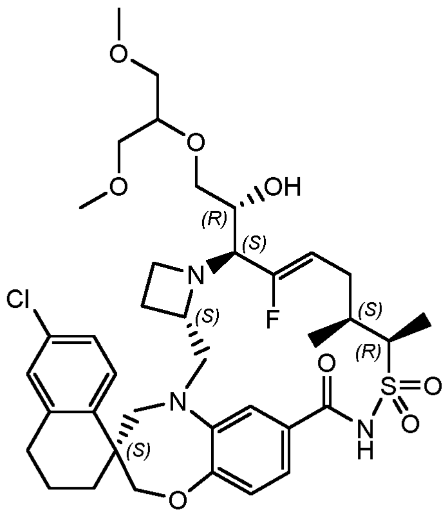 | $^1$H NMR (400 MHz, CHLOROFORM-*d*) δ ppm 1.06 (br d, J=6.16 Hz, 3 H) 1.44 (br d, J=7.26 Hz, 3 H) 1.75 - 2.13 (m, 8 H) 2.32 - 2.46 (m, 1 H) 2.69 - 2.86 (m, 2 H) 2.96 - 3.26 (m, 4 H) 3.27 - 3.37 (m, 3 H) 3.39 (s, 2 H) 3.42 (s, 2 H) 3.47 (dd, J=5.17, 4.29 Hz, 4 H) 3.61 - 3.87 (m, 7 H) 3.93 (br d, J=8.36 Hz, 1 H) 4.00 - 4.15 (m, 3 H) 4.19 (br s, 1 H) 4.73 - 4.93 (m, 1 H) 6.87 - 7.02 (m, 3 H) 7.09 (br d, J=2.20 Hz, 1 H) 7.18 (br dd, J=8.58, 2.20 Hz, 1 H) 7.69 (d, J=8.58 Hz, 1 H); LCMS confirms the MW (RT: 1.17, $[M+H]^+$ 764, Method 1). |
| **Compound 25** | 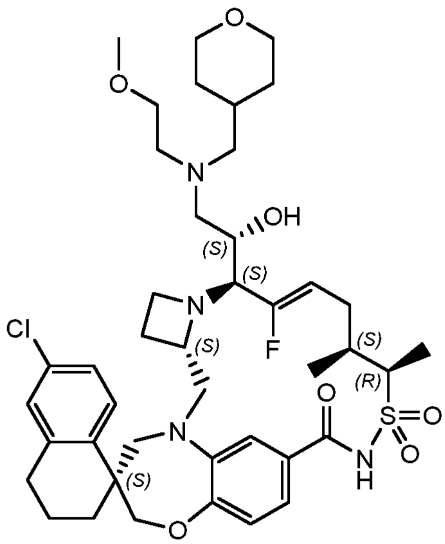 | $^1$H NMR (400 MHz, CHLOROFORM-*d*) δ ppm 1.06 (br d, J=6.16 Hz, 3 H) 1.19 - 1.33 (m, 4 H) 1.43 (br d, J=7.04 Hz, 3 H) 1.56 - 1.64 (m, 1 H) 1.66 - 2.15 (m, 8 H) 2.33 - 2.50 (m, 4 H) 2.56 - 2.67 (m, 1 H) 2.71 - 3.00 (m, 6 H) 3.02 - 3.13 (m, 1 H) 3.16 - 3.28 (m, 2 H) 3.36 (s, 3 H) 3.37 - 3.52 (m, 5 H) 3.62 (td, J=9.41, 2.97 Hz, 1 H) 3.69 - 3.86 (m, 3 H) 3.90 - 4.00 (m, 2 H) 4.00 - 4.13 (m, 2 H) 4.19 (br d, J=7.04 Hz, 1 H) 4.77 - 5.01 (m, 1 H) 6.90 - 7.03 (m, 3 H) 7.09 (d, J=1.98 Hz, 1 H) 7.18 (dd, J=8.36, 2.20 Hz, 1 H) 7.69 (d, J=8.36 Hz, 1 H); LCMS confirms the MW (RT: 2.10, $[M+H]^+$ 817, Method 4); SFC (Rt 3.19 min 0.00% isomer 1) (Rt 3.50 min 100.00% isomer 2) (RT: 3.50, [M+H]+ 817, Method 13). |

(continued)

| Compound | Structure | Analytical data |
|---|---|---|
| **Compound 26** | 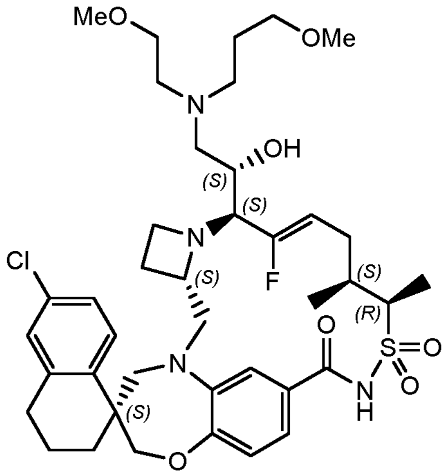 | $^1$H NMR (400 MHz, CHLOROFORM-*d*) δ ppm 1.05 (br d, J=5.23 Hz, 3 H) 1.36 - 1.48 (m, 4 H) 1.75 - 1.91 (m, 4 H) 1.93 - 2.08 (m, 5 H) 2.31 - 2.42 (m, 1 H) 2.50 - 2.59 (m, 1 H) 2.68 -2.89 (m, 6 H) 2.91 - 3.07 (m, 3 H) 3.14 - 3.25 (m, 2 H) 3.28 - 3.32 (m, 1 H) 3.33 (s, 3 H) 3.36 (s, 3 H) 3.40 - 3.57 (m, 5 H) 3.60 - 3.81 (m, 4 H) 3.98-4.19 (m, 3 H) 4.77 - 5.05 (m, 1 H) 6.90 (br d, J=7.94 Hz, 1 H) 6.93 - 7.05 (m, 2 H) 7.08 (d, J=2.19 Hz, 1 H) 7.13 - 7.20 (m, 1 H) 7.69 (d, J=8.57 Hz, 1 H); LCMS confirms the MW (RT: 2.12, $[M+H]^+$ 791, Method 3). |
| **Compound 27** | 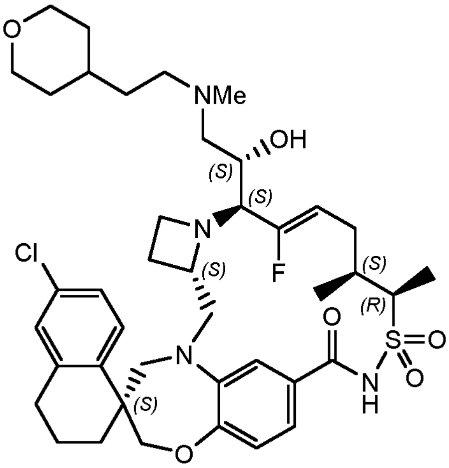 | $^1$H NMR (400 MHz, CHLOROFORM-*d*) δ ppm 1.01 - 1.14 (m, 3 H) 1.22 - 1.52 (m, 8 H) 1.60 - 1.74 (m, 4 H) 1.86 - 2.15 (m, 7 H) 2.27 - 2.36 (m, 1 H) 2.70 - 2.88 (m, 5 H) 2.98 - 3.04 (m, 1 H) 3.11 - 3.39 (m, 8 H) 3.63 - 3.83 (m, 3 H) 3.86 - 4.13 (m, 7 H) 4.87 - 5.12 (m, 1 H) 6.84 - 7.01 (m, 2 H) 7.04 - 7.21 (m, 3 H) 7.69 (d, J=8.58 Hz, 1 H); LCMS confirms the MW (RT: 1.10, $[M+H]^+$ 787, Method 1). |
| **Compound 28** | 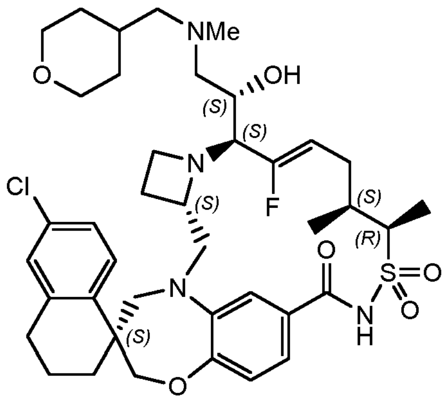 | $^1$H NMR (400 MHz, CHLOROFORM-*d*) δ ppm 1.02 - 1.14 (m, 3 H) 1.43 (br d, J=7.21 Hz, 3 H) 1.54 - 1.67 (m, 1 H) 1.72 (br d, J=14.42 Hz, 1 H) 1.77 - 1.86 (m, 1 H) 1.88 - 2.10 (m, 5 H) 2.27 - 2.52 (m, 7 H) 2.69 - 2.82 (m, 4 H) 2.87-2.97 (m, 1 H) 2.97 - 3.12 (m, 2 H) 3.17 - 3.27 (m, 2 H) 3.29 - 3.48 (m, 4 H) 3.68 - 3.83 (m, 4 H) 3.90 - 3.99 (m, 3 H) 4.02-4.14 (m, 2 H) 4.14 - 4.20 (m, 1 H) 4.89 (br dd, J=38.67, 6.58 Hz, 1 H) 6.88 - 7.05 (m, 3 H) 7.08 (s, 1 H) 7.17 (dd, J=8.41, 1.41 Hz, 1 H) 7.68 (d, J=8.47 Hz, 1 H); LCMS confirms the MW (RT: 1.06, $[M+H]^+$ 344, Method 1). |

(continued)

| Compound | Structure | Analytical data |
|---|---|---|
| **Compound 29** | 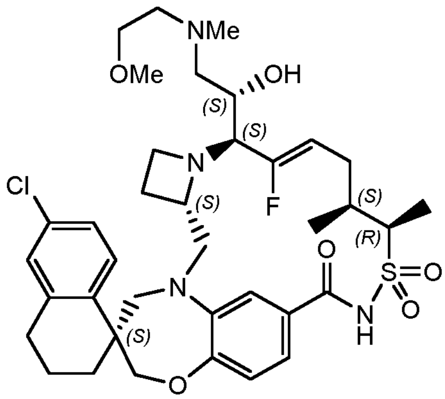 | $^1$H NMR (400 MHz, CHLOROFORM-*d*) δ ppm 1.04 (d, J=6.69 Hz, 3 H) 1.36 (br d, J=6.38 Hz, 3 H) 1.71 - 2.08 (m, 7 H) 2.11 - 2.19 (m, 1 H) 2.25 - 2.39 (m, 1 H) 2.62 - 2.82 (m, 6 H) 2.92 - 3.20 (m, 8 H) 3.29 - 3.39 (m, 4 H) 3.54 - 3.78 (m, 5 H) 3.85 (br d, J=8.26 Hz, 1 H) 3.90 - 4.07 (m, 3 H) 4.91 - 5.09 (m, 1 H) 6.87 (d, J=8.05 Hz, 1 H) 6.95 - 7.20 (m, 4 H) 7.70 (d, J=8.57 Hz, 1 H); LCMS confirms the MW (RT: 2.01, $[M+H]^+$ 733, Method 3). |
| **Compound 30** | 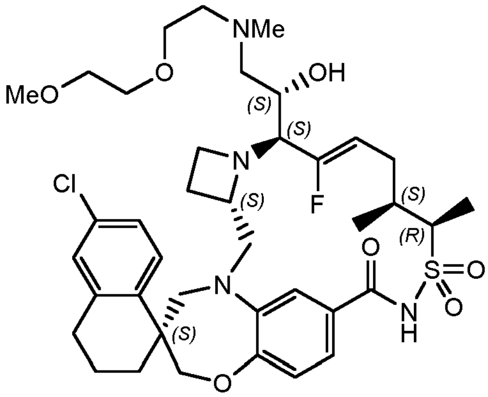 | $^1$H NMR (400 MHz, CHLOROFORM-*d*) δ ppm 1.03 (br d, J=6.69 Hz, 3 H) 1.35 (br d, J=6.69 Hz, 3 H) 1.71 - 2.09 (m, 6 H) 2.12-2.18 (m, 1 H) 2.19 - 2.38 (m, 2 H) 2.76 (br s, 5 H) 2.90 - 3.36 (m, 9 H) 3.36 - 3.42 (m, 3 H) 3.44 - 4.14 (m, 13 H) 4.90 - 5.13 (m, 1 H) 6.86 (d, J=8.15 Hz, 1 H) 6.98 (br s, 1 H) 7.07 (d, J=2.19 Hz, 1 H) 7.17 (br dd, J=8.47, 2.19 Hz, 2 H) 7.70 (d, J=8.57 Hz, 1 H); LCMS confirms the MW (RT: 1.09, $[M+H]^+$ 777, Method 1). |

## Compound 31

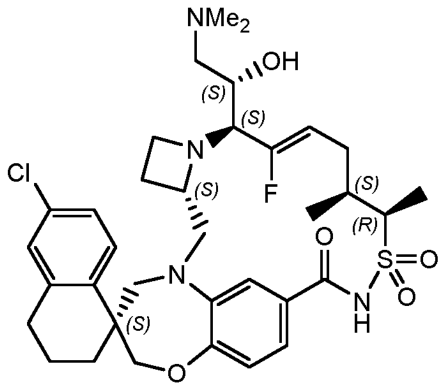

To the stirred solution of Intermediate 44 (20 mg, 0.0262 mmol) in 1 mL of DCM was added trimethylphosphine (52 μL, 1 M, 0.0524 mmol) at RT. The reaction was stirred for 1 hour until all starting material was consumed. Then paraformaldehyde (5 mg) was added to the reaction and stirred for 2 hours. Then, acetic acid (150 μL, 1.049 g/mL, 2.619 mmol) was added into the reaction. After 5 min, sodium triacetoxyborohydride (28 mg, 0.131 mmol) was added into the reaction, was added to the reaction.

Water was added to the reaction and it was extracted with DCM. Organic layers were combined, washed with brine, dried over $MgSO_4$ and concentrated under reduced pressure. A purification was performed via Prep HPLC (Stationary phase: RP XBridge Prep C18 OBD - 5 μm, 50 x 250 mm, Mobile phase: 0.25% $NH_4HCO_3$ solution in water, $CH_3CN$) yielding Compound 31 (3 mg, 17%) as a white solid.

$^1$H NMR (400 MHz, CHLOROFORM-*d*) δ ppm 1.00 (br d, J=6.38 Hz, 3 H) 1.16 - 1.29 (m, 3 H) 1.80 - 2.08 (m, 9 H) 2.21 - 2.32 (m, 3 H) 2.71 - 2.86 (m, 7 H) 3.05 - 3.33 (m, 5 H) 3.60 - 3.86 (m, 4 H) 3.95-4.01 (m, 3 H) 4.99 - 5.29 (m, 1 H) 6.82 (br d, J=7.92 Hz, 1 H) 6.99 (br d, J=3.96 Hz, 1 H) 7.06 (d, J=1.98 Hz, 1 H) 7.12 - 7.19 (m, 1 H) 7.26 (s, 1 H) 7.71 (d, J=8.58 Hz, 1

H); LCMS confirms the MW (RT: 1.93, $[M+H]^+$ 689, Method 3).

## Compound 32

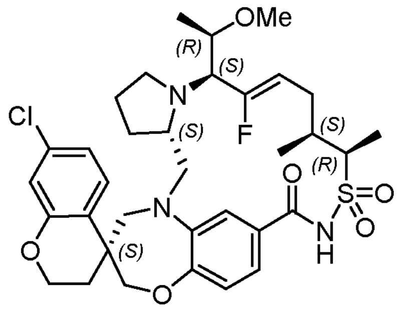

**Compound 17** (10 mg, 0.0151 mmol) was dissolved in THF (0.5 ml), then NaH (60% dispersion in mineral oil) (4 mg, 0.1 mmol) was added, this was stirred for 10 min at RT. Then MeI (10μL, 0.161 mmol) was added and the reaction vessel was closed and stirred at 60 °C for 20 h. The RM was diluted with DCM and washed with water and a little HCl 1N to neutralize. The OL was separated, dried(MgSO4), filtered and concentrated, then coevaporated with DIPE to complete dryness, giving **Compound 32** (10 mg, 97%) as white solid.

$^1$H NMR (400 MHz, CHLOROFORM-d) δ ppm 1.02 - 1.08 (m, 3 H) 1.22 (br s, 3 H) 1.43 - 1.48 (m, 3 H) 1.57 - 1.65 (m, 1 H) 1.67 - 1.74 (m, 2 H) 1.74 - 1.81 (m, 1 H) 1.87 - 2.08 (m, 4 H) 2.18 - 2.26 (m, 1 H) 2.77 - 2.86 (m, 1 H) 2.93 - 2.98 (m, 1 H) 2.99 - 3.05 (m, 1 H) 3.24 - 3.34 (m, 2 H) 3.37 - 3.40 (m, 1 H) 3.41 - 3.44 (m, 3 H) 3.57 - 3.65 (m, 1 H) 3.90 - 3.97 (m, 1 H) 3.99 - 4.04 (m, 1 H) 4.05 - 4.10 (m, 1 H) 4.11 - 4.21 (m, 3 H) 4.28 - 4.35 (m, 1 H) 4.62 - 4.78 (m, 1 H) 6.83 - 6.86 (m, 1 H) 6.88 - 6.97 (m, 3 H) 7.03 - 7.08 (m, 1 H) 7.59 (d, J=8.4 Hz, 1 H); LCMS confirms the MW (RT: 2.12, $[M+H]^+$ 676, Method 3).

### Analytical Analysis

LCMS

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method Codes (Flow expressed in mL/min; column temperature (T) in °C; Run time in minutes)

| Method Code | Instrument | column | mobile phase | gradient | Flow<br>ColT | Run time |
|---|---|---|---|---|---|---|
| 1 | Waters: Acquity® UPLC® - DAD and SQD | Waters : BEH C18 (1.7 μm, 2.1 * 50 mm) | A: 10 mM $CH_3COONH_4$ in 95 % $H_2O$ + 5 % $CH_3CN$<br>B: $CH_3CN$ | From 95 % A to 5 % A in 1.3 min, held for 0.7 min. | 0.8<br>-------<br>55 | 2 |

(continued)

| Method Code | Instrument | column | mobile phase | gradient | Flow Col T | Run time |
|---|---|---|---|---|---|---|
| 2 | Waters: Acquity® UPLC® - DAD and SQD | Waters :BEH (1.8μm, 2.1 * 50 mm) | A: 0.1 % $NH_4HCO_3$ in 95 % $H_2O$ + 5 % $CH_3CN$ B: $CH_3CN$ | From 100 % A to 5 % A in 1.3 min, hold 0.7 min | 0.8 ------- 55 | 2.0 |
| 3 | Waters: Acquity® UPLC® - DAD and SQD | Waters : BEH (1.8 μm, 2.1 * 100 mm) | A: 10 mM $CH_3COONH_4$ in 95 % $H_2O$ + 5 % $CH_3CN$ B: $CH_3CN$ | From 100 % A to 5% A in 2.10 min, to 0 % A in 0.90 min, to 5 % A in 0.5 min | 0.6 ------- 55 | 3.5 |
| 4 | Waters: Acquity® UPLC® - DAD and SQD | Waters :BEH (1.8 μm, 2.1 * 100 mm) | A: 0.1 % $NH_4HCO_3$ in 95 % $H_2O$ + 5 % $CH_3CN$ B: $CH_3CN$ | From 100 % A to 5 % A in 2.10 min, to 0 % A in 0.9 min, to 5 % A in 0.5 min | 0.6 ------- 55 | 3.5 |
| 5 | Waters: Acquity® UPLC® - DAD and SQD | Waters :BEH (1.8 μm, 2.1 * 100 mm) | A: 0.1 % $NH_4HCO_3$ in 95 % $H_2O$ + 5 % $CH_3CN$ B: $CH_3CN$ | From 100 % A to 5 % A in 2.10 min, to 0 % A in 0.9 min, to 5 % A in 0.4 min | 0.6 ------- 55 | 3.5 |
| 6 | Waters: Acquity® UPLC® - DAD and SQD3 | Waters :BEH (1.8 μm, 2.1 * 100 mm) | A: 10mM $NH_4HCO_3$ in 95% $H_2O$ + 5% $CH_3CN$ B: $CH_3CN$ | From 100 % A to 5 % A in 2.10 min, to 0 % A in 0.9 min, to 5 % A in 0.4 min | 0.6 ------- 55 | 3.5 |

SFC-MS methods

The SFC measurement was performed using an Analytical Supercritical fluid chromatography (SFC) system composed by a binary pump for delivering carbon dioxide ($CO_2$) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Analytical SFC-MS Methods (Flow expressed in mL/min; column temperature (T) in °C; Run time in minutes, Backpressure (BPR) in bars. "$iPrNH_2$" means isopropylamine, "EtOH" means ethanol, "min" mean minutes.

| SFC Method | Column | mobile phase | gradient | FlowColT | Run time-BPR |
|---|---|---|---|---|---|
| 11 | Daicel Chiralpak® IH3 column (3.0 μm, 150 x 4.6 mm) | A:$CO_2$ B: EtOH + 0.2 % $iPrNH_2$ | 10%-50% B in 6 min, hold 3.5 min | 2.5 ------- 40 | 9.5 ------- 130 |
| 12 | Daicel Chiralcel® OJ3 column (3.0 μm, 150 × 4.6 mm) | A:$CO_2$ B: MeOH + 0.2 % $iPrNH_2$ | 10%-50% B in 6 min, hold 3.5 min | 2.5 ------- 40 | 9.5 ------- 130 |

(continued)

| SFC Method | Column | mobile phase | gradient | FlowColT | Run time-BPR |
|---|---|---|---|---|---|
| 13 | Daicel Chiralcel® OJ3 column (3.0 μm, 150 × 4.6 mm) | A:$CO_2$<br>B: EtOH + 0.2 % $iPrNH_2$ | 10%-50% B in 6 min, hold 3.5 min | 2.5<br>-------<br>40 | 9.5<br>-------<br>130 |

NMR

$^1$H NMR spectra were recorded on Bruker Avance III and Avance NEO spectrometers. $CDCl_3$ was used as solvent, unless otherwise mentioned. The chemical shifts are expressed in ppm relative to tetramethylsilane.

**Pharmacological Analysis**

Biological Example 1

Terbium labeled Myeloid Cell Leukemia 1(Mcl-1) homogeneous time-resolved fluorescence (HTRF) binding assay utilizing the BIM BH3 peptide ($H_2$N-(C/Cy5Mal) WIAQELRRIGDEFN-OH) as the binding partner for Mcl-1.

Apoptosis, or programmed cell death, ensures normal tissue homeostasis, and its dysregulation can lead to several human pathologies, including cancer. Whilst the extrinsic apoptosis pathway is initiated through the activation of cell-surface receptors, the intrinsic apoptosis pathway occurs at the mitochondrial outer membrane and is governed by the binding interactions between pro- and anti-apoptotic Bcl-2 family proteins, including Mcl-1. In many cancers, the anti-apoptotic Bcl-2 protein(s), such as the Mcl-1, are upregulated, and in this way the cancer cells can evade apoptosis. Thus, inhibition of the Bcl-2 protein(s), such as Mcl-1, may lead to apoptosis in cancer cells, providing a method for the treatment of said cancers.

This assay evaluated inhibition of the BH3 domain : Mcl-1 interaction by measuring the displacement of Cy5-labeled BIM BH3 peptide ($H_2$N-(C/Cy5Mal) WIAQELRRIGDEFN-OH) in the HTRF assay format.

Assay Procedure

The following assay and stock buffers were prepared for use in the assay: (a) Stock buffer: 10 mM Tris-HCl, pH = 7.5 + 150 mM NaCl, filtered, sterilized, and stored at 4°C; and (b) 1X assay buffer, where the following ingredients were added fresh to stock buffer: 2 mM dithiothreitol (DTT), 0.0025% Tween-20, 0.1 mg/mL bovine serum albumin (BSA). The 1X Tb-Mcl-1 + Cy5 Bim peptide solution was prepared by diluting the protein stock solution using the 1X assay buffer (b) to 25 pM Tb-Mcl-1 and 8 nM Cy5 Bim peptide.

Using the Acoustic ECHO, 100 nL of 100x test compound(s) were dispensed into individual wells of a white 384-well Perkin Elmer Proxiplate, for a final compound concentration of 1x and final DMSO concentration of 1%. Inhibitor control and neutral control (NC, 100 nL of 100% DMSO) were stamped into columns 23 and 24 of assay plate. Into each well of the plate was then dispensed 10 μL of the 1X Tb-Mcl-1 + Cy5 Bim peptide solution. The plate was centrifuged with a cover plate at 1000 rpm for 1 minute, then incubated for 60 minutes at room temperature with plates covered.

The TR-FRET signal was read on an BMG PHERAStar FSX MicroPlate Reader at room temperature using the HTRF optic module (HTRF: excitation: 337nm, light source: laser, emission A: 665 nm, emission B: 620 nm, integration start: 60 μs, integration time: 400 μs).

Data Analysis

The BMG PHERAStar FSX MicroPlate Reader was used to measure fluorescence intensity at two emission wavelengths - 665 nm and 620 nm - and report relative fluorescence units (RFU) for both emissions, as well as a ratio of the emissions (665 nm/620 nm)*10,000. The RFU values were normalized to percent inhibition as follows:

$$\% \ inhibition = (((NC - IC) - (compound - IC)) / (NC - IC)) * 100$$

where IC (inhibitor control, low signal) = mean signal of 1X Tb-MCl-1 + Cy5 Bim peptide+ inhibitor control or 100% inhibition of Mcl-1; NC (neutral control, high signal) = mean signal 1X Tb-MCl-1 + Cy5 Bim peptide with DMSO only or 0% inhibition

An 11-point dose response curve was generated to determine $IC_{50}$ values (using GenData) based on the

following equation:

$$Y=Bottom + (Top-Bottom)/(1+10^{\wedge}((logIC_{50}-X)*HillSlope))$$

where Y = % inhibition in the presence of X inhibitor concentration; Top = 100% inhibition derived from the IC (mean signal of Mcl-1 + inhibitor control); Bottom = 0% inhibition derived from the NC (mean signal of Mcl-1 + DMSO); Hillslope = Hill coefficient; and $IC_{50}$ = concentration of compound with 50% inhibition in relation to top/neutral control (NC).

$$K_i = IC_{50} / (1 + [L]/Kd)$$

In this assay [L] = 8 nM and Kd = 10 nM

Representative compounds of the present invention were tested according to the procedure as described above, with results as listed in the Table below (n.d. means not determined).

| Compound | Tb-MCL1 $K_i$ (nM) |
|---|---|
| **1** | 0.326 |
| **2** | 0.106 |
| **3** | 1.560 |
| **4** | 0.236 |
| **5** | 0.230 |
| **6** | 0.053 |
| **7** | 0.221 |
| **8** | 0.357 |
| **9** | 0.040 |
| **10** | 0.551 |
| **11** | 0.172 |
| **12** | 0.359 |
| **13** | 1.770 |
| **14** | 0.241 |
| **15** | 0.177 |
| **16** | 0.072 |
| **17** | 2.41 |
| **18** | 0.606 |
| **19** | 0.049 |
| **20** | 0.052 |
| **21** | 0.051 |
| **22** | 0.080 |
| **23** | 0.023 |
| **24** | 0.036 |
| **25** | 0.025 |
| **26** | 0.044 |
| **27** | 0.053 |
| **28** | 0.037 |

(continued)

| Compound | Tb-MCL1 $K_i$ (nM) |
|---|---|
| **29** | 0.044 |
| **30** | 0.036 |
| **31** | 0.041 |
| **32** | 1.93 |

Biological Example 2

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

The Caspase-Glo® 3/7 Assay is a luminescent assay that measures caspase-3 and -7 activities in purified enzyme preparations or cultures of adherent or suspension cells. The assay provides a proluminescent caspase-3/7 substrate, which contains the tetrapeptide sequence DEVD. This substrate is cleaved to release aminoluciferin, a substrate of luciferase used in the production of light. Addition of the single Caspase-Glo® 3/7 Reagent in an "add-mix-measure" format results in cell lysis, followed by caspase cleavage of the substrate and generation of a "glow-type" luminescent signal.

This assay uses the MOLP-8 human multiple myeloma cell line, which is sensitive to MCL-1 inhibition.

Materials:

- Perkin Elmer Envision
- Multidrop 384 and small volume dispensing cassettes
- Centrifuge
- Countess automated cell counter
- Countess counting chamber slides
- Assay plate: ProxiPlate-384 Plus, White 384-shallow well Microplate
- Sealing tape: Topseal A plus
- T175 culture flask

| Product | Units | Storage |
|---|---|---|
| RPMI1640 (no L-Glutamine, no phenol red) | 500 mL | 4 °C |
| Foetal Bovine Serum (FBS) (Heat inactivated) | 500 mL | 4 °C |
| L-Glutamine (200 mM) | 100 mL | -20 °C |
| Gentamicin (50 mg/mL) | 100 mL | 4 °C |
| Caspase 3/7 Detection kit | 100 mL<br>10 × 10 mL | -20 °C |

Cell culture media:

| **MOLP8** | |
|---|---|
| | |
| RPMI-1640 medium | 500 mL |
| 20 % FBS (heat inactivated) | 120 mL |
| 2 mM L-Glutamine | 6.2 mL |
| 50 μg/mL Gentamicin | 620 μL |
| | |
| **Assay media** | |
| | |
| RPMI-1640 medium | 500 mL |
| 10 % FBS (Heat inactivated) | 57 mL |
| 2 mM L-Glutamine | 5.7 mL |
| 50 μg/mL Gentamicin | 570 μL |

Cell culture:

Cell cultures were maintained between 0.2 and 2.0 $\times 10^6$ cells/mL. The cells were harvested by collection in 50 mL conical tubes. The cells were then pelleted at 500 g for 5 mins before removing supernatant and resuspension in fresh pre-warmed culture medium. The cells were counted and diluted as needed.

Caspase-Glo reagent:

The assay reagent was prepared by transferring the buffer solution to the substrate vial and mixing. The solution may be stored for up to 1 week at 4 °C with negligible loss of signal.

Assay procedure:

Compounds were delivered in assay-ready plates (Proxiplate) and stored at -20°C.

Assays always include 1 reference compound plate containing reference compounds. The plates were spotted with 40 nL of compounds (0.5 % DMSO final in cells; serial dilution; 30 μM highest conc. 1/3 dilution, 10 doses, duplicates). The compounds were used at room temperature and 4 μL of pre-warmed media was added to all wells except column 2 and 23. The negative control was prepared by adding 1 % DMSO in media. The positive control was prepared by adding the appropriate positive control compound in final concentration of 60 μM in media. The plate was prepared by adding 4 μL negative control to column 23, 4 μL positive control to column 2, and 4 μL cell suspension to all wells in the plate. The plate with cells was then incubated at 37 °C for 2 hours. The assay signal reagent is the Caspase-Glo solution described above, and 8 μL was added to all wells. The plates were then sealed and measured after 30 minutes.

The activity of a test compound was calculated as percent change in apoptosis induction as follows:

LC = median of the Low Control values

= Central Reference in Screener

= DMSO

= 0 %

HC = Median of the High Control values

= Scale Reference in Screener

= 30 μM of positive control

= 100 % apoptosis induction

$$\%\text{Effect } (AC_{50}) = 100 - ((\text{sample-LC}) / (\text{HC-LC})) * 100$$

$$\%\text{Control} = (\text{sample} / \text{HC}) * 100$$

$$\%\text{Control min} = ((\text{sample-LC}) / (\text{HC-LC})) * 100$$

Table: Measured $AC_{50}$ for Representative Compounds of Formula (I). Averaged values are reported over all runs on all batches of a particular compound.

| Compound | MOLP8 $AC_{50}$ (nM) |
|---|---|
| 1 | 116.0 |
| 2 | 121.6 |
| 3 | 216.6 |
| 4 | 237.8 |
| 5 | 237.6 |
| 6 | 68.5 |
| 7 | 133.9 |
| 8 | 3957.3 |
| 9 | 64.8 |
| 10 | 553.2 |
| 11 | 96.5 |
| 12 | 885.1 |
| 13 | 1105.9 |
| 14 | 758.9 |
| 15 | 503.4 |
| 16 | 221.6 |
| 17 | 3349.7 |
| 18 | 3415.1 |
| 19 | 126.3 |
| 20 | 271.9 |
| 21 | 87.9 |
| 22 | 239.3 |
| 23 | 69.6 |
| 24 | 46.3 |
| 25 | 36.6 |
| 26 | 123.3 |

(continued)

| Compound | MOLP8 $AC_{50}$ (nM) |
|---|---|
| **27** | 746.5 |
| **28** | 210.9 |
| **29** | 384.8 |
| **30** | 944.5 |
| **31** | 1022.9 |
| **32** | 3708.5 |

## Claims

**1.** A compound of Formula (I)

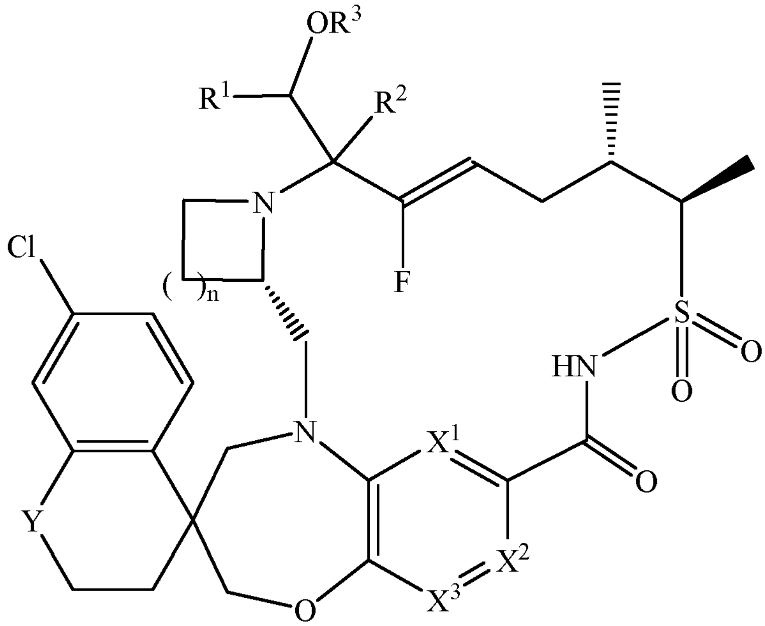

(I),

wherein

$R^1$ represents $C_{1-4}$alkyl, $-C_{1-4}$alkyl-$NR^{4a}R^{4b}$, or $-C_{1-4}$alkyl-$OR^5$;
$R^2$ represents hydrogen, methyl, $-CH_2-NR^{4c}R^{4d}$, or $-CH_2-OR^6$; and
$R^3$ represents hydrogen, $C_{1-4}$alkyl, or $-C_{2-4}$alkyl-O-$C_{1-4}$alkyl;
or
$R^1$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and
$R^2$ represents hydrogen, methyl, $-CH_2-NR^{4c}R^{4d}$, or $-CH_2-OR^6$;
or
$R^2$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;

and
$R^1$ represents hydrogen, $C_{1-4}$alkyl, -$C_{1-4}$alkyl-$NR^{4a}R^{4b}$, or -$C_{1-4}$alkyl-$OR^5$;
or
$R^1$ and $R^2$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three substituents each independently selected from $C_{1-4}$alkyl, -O-$C_{1-4}$alkyl, and -OH;
and
$R^3$ represents hydrogen, $C_{1-4}$alkyl, or -$C_{2-4}$alkyl-O-$C_{1-4}$alkyl;
$R^{4a}$ and $R^{4b}$ are each independently selected from the group consisting of $C_{1-4}$alkyl, -$C_{1-4}$alkyl-$Het^{1b}$, -$C_{2-4}$alkyl-O-$C_{1-4}$alkyl, and -$C_{2-4}$alkyl-O-$C_{1-4}$alkyl-O-$C_{1-4}$alkyl;
or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;
or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;
$R^{4c}$ and $R^{4d}$ are each independently selected from the group consisting of $C_{1-4}$alkyl and -$C_{2-4}$alkyl-O-$C_{1-4}$alkyl;
or $R^{4c}$ and $R^{4d}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;
or $R^{4c}$ and $R^{4d}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;
$R^5$ represents hydrogen, $C_{1-4}$alkyl, $Het^{1a}$, -$C_{2-4}$alkyl-$NR^{7a}R^{7b}$, -$C_{1-4}$alkyl-$Het^{1b}$, or $C_{1-4}$alkyl substituted with one or two -O-$C_{1-4}$alkyl;
$R^{7a}$ and $R^{7b}$ are each independently selected from the group consisting of hydrogen and $C_{1-4}$alkyl;
$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;
$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$;
$R^6$ represents hydrogen or $C_{1-4}$alkyl;
n is 1 or 2;
Y represents O or $CH_2$;
$X^1$ represents CH;
$X^2$ represents CH;
$X^3$ represents CH;
or a pharmaceutically acceptable salt, or a solvate thereof.

**2.** The compound according to claim 1, wherein

$R^1$ represents $C_{1-4}$alkyl, -$C_{1-4}$alkyl-$NR^{4a}R^{4b}$, or -$C_{1-4}$alkyl-$OR^5$;
$R^2$ represents hydrogen, or -$CH_2$-$OR^6$; and
$R^3$ represents hydrogen, or $C_{1-4}$alkyl;
or
$R^1$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and
$R^2$ represents -$CH_2$-$OR^6$;
or
$R^2$ and $R^3$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and

$R^1$ represents hydrogen;
or
$R^1$ and $R^2$ are taken together to form together with the atoms to which they are attached a 4- to 7-membered monocyclic fully saturated heterocyclyl containing one O-atom and optionally one additional heteroatom selected from O, S, and N, wherein said S-atom might be substituted to form S(=O) or $S(=O)_2$, and wherein said heterocyclyl is optionally substituted with one, two or three $C_{1-4}$alkyl substituents;
and
$R^3$ represents hydrogen or $C_{1-4}$alkyl;
$R^{4a}$ and $R^{4b}$ are each independently selected from the group consisting of $C_{1-4}$alkyl, -$C_{1-4}$alkyl-$Het^{1b}$, -$C_{2-4}$alkyl-O-$C_{1-4}$alkyl, and -$C_{2-4}$alkyl-O-$C_{1-4}$alkyl-O-$C_{1-4}$alkyl;
or $R^{4a}$ and $R^{4b}$ are taken together to form together with the N-atom to which they are attached a 4- to 7-membered monocyclic fully saturated heterocyclyl containing one N-atom and optionally one additional heteroatom selected from O, S, and N, wherein said S-atom might be substituted to form S(=O) or $S(=O)_2$;
or $R^{4a}$ and $R^{4b}$ are taken together to form together with the N-atom to which they are attached a 5- to 6-membered monocyclic aromatic ring containing one N-atom and optionally one or two additional heteroatoms each independently selected from O, S, and N, wherein said S-atom might be substituted to form S(=O) or $S(=O)_2$;
$R^5$ represents hydrogen, $C_{1-4}$alkyl, $Het^{1a}$, -$C_{1-4}$alkyl-$Het^{1b}$, or
$C_{1-4}$alkyl substituted with one or two -O-$C_{1-4}$alkyl;
$Het^{1a}$ represents a C-linked 4- to 7-membered monocyclic fully saturated heterocyclyl containing one or two heteroatoms selected from O, S, and N, wherein said S-atom might be substituted to form S(=O) or $S(=O)_2$;
$Het^{1b}$ represents a 4- to 7-membered monocyclic fully saturated heterocyclyl containing one or two heteroatoms selected from O, S, and N, wherein said S-atom might be substituted to form S(=O) or $S(=O)_2$.

**3.** The compound according to claim 1 or 2, wherein Y represents $CH_2$.

**4.** The compound according to claim 1 or 2, wherein Y represents O.

**5.** The compound according to any one of claims 1 to 4, wherein n represents 2.

**6.** A pharmaceutical composition comprising a compound as claimed in any one of claims 1 to 5 and a pharmaceutically acceptable carrier or diluent.

**7.** A process for preparing a pharmaceutical composition as defined in claim 6 comprising mixing a pharmaceutically acceptable carrier with a therapeutically effective amount of a compound according to any one of claims 1 to 5.

**8.** A compound as claimed in any one of claims 1 to 5 or a pharmaceutical composition as claimed in claim 6 for use as a medicament.

**9.** A compound as claimed in any one of claims 1 to 5 or a pharmaceutical composition as claimed in claim 6 for use in the prevention or treatment of cancer.

**10.** The compound or a pharmaceutical composition for use according to claim 9, wherein cancer is selected from prostate, lung, pancreatic, breast, ovarian, cervical, melanoma, B-cell chronic lymphocytic leukemia (CLL), acute myeloid leukemia (AML), and acute lymphoblastic leukemia (ALL).

**Patentansprüche**

**1.** Verbindung von Formel (I)

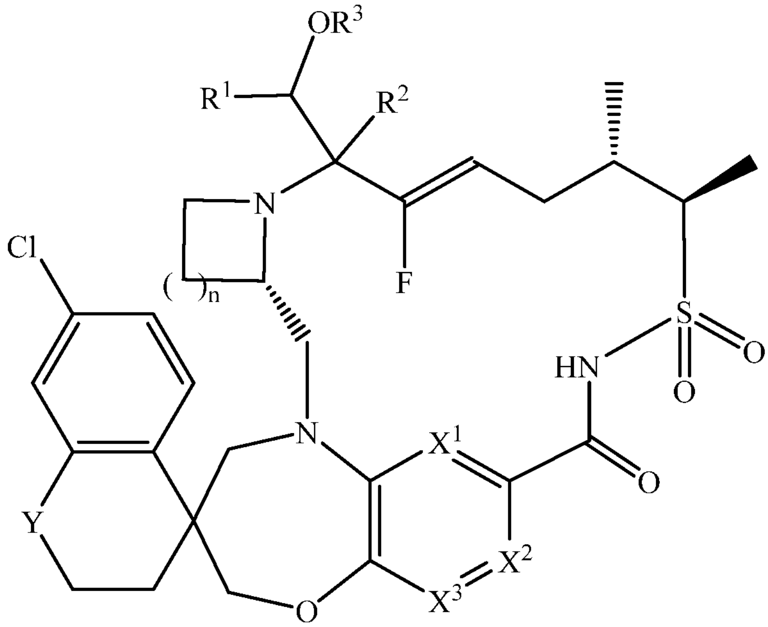

(I),

wobei

$R^1$ für $C_{1-4}$-Alkyl, $-C_{1-4}$-Alkyl-$NR^{4a}R^{4b}$ oder $-C_{1-4}$-Alkyl-$OR^5$ steht;
$R^2$ für Wasserstoff, Methyl, $-CH_2-NR^{4c}R^{4d}$ oder $-CH_2-OR^6$ steht; und
$R^3$ für Wasserstoff, $C_{1-4}$-Alkyl oder $-C_{2-4}$-Alkyl-O-$C_{1-4}$-Alkyl steht;
oder
$R^1$ und $R^3$ zusammengenommen werden, um mit den Atomen, an die sie gebunden sind, ein 4- bis 7-gliedriges monocyclisches, vollständig gesättigtes Heterocyclyl auszubilden, das ein O-Atom und optional ein zusätzliches Heteroatom enthält, das aus O, S und N ausgewählt ist, wobei das S-Atom substituiert sein kann, um S(=O) oder $S(=O)_2$ auszubilden, und wobei das Heterocyclyl optional mit einem, zwei oder drei Substituenten substituiert ist, die jeweils unabhängig aus $C_{1-4}$-Alkyl, -O-$C_{1-4}$-Alkyl und -OH ausgewählt sind;
und
$R^2$ für Wasserstoff, Methyl, $-CH_2-NR^{4c}R^{4d}$ oder $-CH_2-OR^6$ steht;
oder
$R^2$ und $R^3$ zusammengenommen werden, um mit den Atomen, an die sie gebunden sind, ein 4- bis 7-gliedriges monocyclisches, vollständig gesättigtes Heterocyclyl auszubilden, das ein O-Atom und optional ein zusätzliches Heteroatom enthält, das aus O, S und N ausgewählt ist, wobei das S-Atom substituiert sein kann, um S(=O) oder $S(=O)_2$ auszubilden, und wobei das Heterocyclyl optional mit einem, zwei oder drei Substituenten substituiert ist, die jeweils unabhängig aus $C_{1-4}$-Alkyl, -O-$C_{1-4}$-Alkyl und -OH ausgewählt sind;
und
$R^1$ für Wasserstoff, $C_{1-4}$-Alkyl, $-C_{1-4}$-Alkyl-$NR^{4a}R^{4b}$ oder $-C_{1-4}$-Alkyl-$OR^5$ steht;
oder
$R^1$ und $R^2$ zusammengenommen werden, um mit den Atomen, an die sie gebunden sind, einen 4- bis 7-gliedrigen monocyclisches, vollständig gesättigtes Heterocyclyl auszubilden, das ein O-Atom und optional ein zusätzliches Heteroatom enthält, das aus O, S und N ausgewählt ist, wobei das S-Atom substituiert sein kann, um S(=O) oder $S(=O)_2$ auszubilden, und wobei das Heterocyclyl optional mit einem, zwei oder drei Substituenten substituiert ist, die jeweils unabhängig aus $C_{1-4}$-Alkyl, -O-$C_{1-4}$-Alkyl und -OH ausgewählt sind;
und
$R^3$ für Wasserstoff, $C_{1-4}$-Alkyl oder $-C_{2-4}$-Alkyl-O-$C_{1-4}$-Alkyl steht;
$R^{4a}$ und $R^{4b}$ jeweils unabhängig aus der Gruppe ausgewählt sind, bestehend aus $C_{1-4}$-Alkyl, $-C_{1-4}$-Alkyl-$Het^{1b}$, $-C_{2-4}$-Alkyl-O-$C_{1-4}$-Alkyl und $-C_{2-4}$-Alkyl-O-$C_{1-4}$-Alkyl-O-$C_{1-4}$-Alkyl-,
oder $R^{4a}$ und $R^{4b}$ zusammengenommen werden, um zusammen mit dem N-Atom, an das sie gebunden sind, ein 4- bis 7-gliedriges monocyclisches vollständig gesättigtes Heterocyclyl auszubilden, das ein N-Atom und optional ein zusätzliches Heteroatom enthält, das aus O, S und N ausgewählt ist, wobei das S-Atom substituiert sein kann, um S(=O) oder $S(=O)_2$ auszubilden;
oder $R^{4a}$ und $R^{4b}$ zusammengenommen werden, um zusammen mit dem N-Atom, an das sie gebunden sind,

einen 5- bis 6-gliedrigen monocyclischen Ring auszubilden, der ein N-Atom und optional ein oder zwei zusätzliche Heteroatome enthält, die jeweils unabhängig aus O, S und N ausgewählt sind, wobei das S-Atom substituiert sein kann, um S(=O) oder $S(=O)_2$ auszubilden;

$R^{4c}$ und $R^{4d}$ unabhängig aus der Gruppe ausgewählt sind, bestehend aus $C_{1-4}$-Alkyl und $C_{2-4}$-Alkyl-O-$C_{1-4}$-Alkyl-,

oder $R^{4c}$ und $R^{4d}$ zusammengenommen werden, um zusammen mit dem N-Atom, an das sie gebunden sind, ein 4- bis 7-gliedriges monocyclisches vollständig gesättigtes Heterocyclyl auszubilden, das ein N-Atom und optional ein zusätzliches Heteroatom enthält, das aus O, S und N ausgewählt ist, wobei das S-Atom substituiert sein kann, um S(=O) oder $S(=O)_2$ auszubilden;

$R^{4c}$ und $R^{4d}$ zusammengenommen werden, um zusammen mit dem N-Atom, an das sie gebunden sind, einen 5- bis 6-gliedrigen monocyclischen aromatischen Ring auszubilden, der ein N-Atom und optional ein oder zwei zusätzliche Heteroatome enthält, die jeweils unabhängig aus O, S und N ausgewählt sind, wobei das S-Atom substituiert sein kann, um S(=O) oder $S(=O)_2$ auszubilden;

$R^5$ für Wasserstoff, $C_{1-4}$-Alkyl, $Het^{1a}$, -$C_{2-4}$-Alkyl-$NR^{7a}R^{7b}$, -$C_{1-4}$-Alkyl-$Het^{1b}$ oder $C_{1-4}$-Alkyl, das mit einem oder zwei -O-$C_{1-4}$-Alkylsubstituiert ist, steht;

$R^{7a}$ und $R^{7b}$ jeweils unabhängig aus der Gruppe ausgewählt sind, bestehend aus Wasserstoff und $C_{1-4}$-Alkyl;

$Het^{1a}$ für ein C-verknüpftes 4- bis 7-gliedriges monocyclisches vollständig gesättigtes Heterocyclyl steht, das ein oder zwei Heteroatome enthält, die aus O, S und N ausgewählt sind; wobei das S-Atom substituiert sein kann, um S(=O) oder $S(=O)_2$ auszubilden;

$Het^{1b}$ für ein 4- bis 7-gliedriges monocyclisches vollständig gesättigtes Heterocyclyl steht, das ein oder zwei Heteroatome enthält, die aus O, S und N ausgewählt sind; wobei das S-Atom substituiert sein kann, um S(=O) oder $S(=O)_2$ auszubilden;

$R^6$ für Wasserstoff oder $C_{1-4}$-Alkyl steht;

n 1 oder 2 ist;

Y für O oder $CH_2$ steht;

$X^1$ für CH steht;

$X^2$ für CH steht;

$X^3$ für CH steht;

oder ein pharmazeutisch unbedenkliches Salz oder ein Solvat davon.

**2.** Verbindung nach Anspruch 1, wobei

$R^1$ für $C_{1-4}$-Alkyl, -$C_{1-4}$-Alkyl-$NR^{4a}R^{4b}$ oder -$C_{1-4}$-Alkyl-$OR^5$ steht;

$R^2$ für Wasserstoff oder -$CH_2$-$OR^6$ steht; und

$R^3$ für Wasserstoff oder $C_{1-4}$-Alkyl steht;

oder

$R^1$ und $R^3$ zusammengenommen werden, um zusammen mit den Atomen, an dir sie gebunden sind, ein 4- bis 7-gliedriges monocyclisches vollständig gesättigtes Heterocyclyl auszubilden, das ein O-Atom und optional ein zusätzliches Heteroatom enthält, das aus O, S und N ausgewählt ist, wobei das S-Atom substituiert sein kann, um S(=O) oder $S(=O)_2$ auszubilden, und wobei das Heterocycyl optional mit einem, zwei oder drei $C_{1-4}$-Alkyl-Substituenten substituiert ist;

und

$R^2$ für -$CH_2$-$OR^6$ steht;

oder

$R^2$ und $R^3$ zusammengenommen werden, um zusammen mit den Atomen, an die sie gebunden sind, ein 4- bis 7-gliedriges monocyclisches vollständig gesättigtes Heterocyclyl auszubilden, das ein O-Atom und optional ein zusätzliches Heteroatom enthält, das aus O, S und N ausgewählt ist, wobei das S-Atom substituiert sein kann, um S(=O) oder $S(=O)_2$ auszubilden, und wobei das Heterocycyl optional mit einem, zwei oder drei $C_{1-4}$-Alkyl-Substituenten substituiert ist;

und

$R^1$ für Wasserstoff steht;

oder

$R^1$ und $R^2$ zusammengenommen werden, um zusammen mit den Atomen, an die sie gebunden sind, ein 4- bis 7-gliedriges monocyclisches vollständig gesättigtes Heterocyclyl auszubilden, das ein O-Atom und optional ein zusätzliches Heteroatom enthält, das aus O, S und N ausgewählt ist, wobei das S-Atom substituiert sein kann, um S(=O) oder $S(=O)_2$ auszubilden, und wobei das Heterocycyl optional mit einem, zwei oder drei $C_{1-4}$-Alkyl-Substituenten substituiert ist;

und

$R^3$ für Wasserstoff oder $C_{1-4}$-Alkyl steht;
$R^{4a}$ und $R^{4b}$ jeweils unabhängig aus der Gruppe ausgewählt sind, bestehend aus $C_{1-4}$-Alkyl, -$C_{1-4}$-Alkyl-$Het^{1b}$, -$C_{2-4}$-Alkyl-O$C_{1-4}$-Alkyl und -$C_{2-4}$-Alkyl-O-$C_{1-4}$-Alkyl-O-$C_{1-4}$-Alkyl-,
oder $R^{4a}$ und $R^{4b}$ zusammengenommen werden, um zusammen mit dem N-Atom, an das sie gebunden sind, ein 4- bis 7-gliedriges monocyclisches vollständig gesättigtes Heterocyclyl auszubilden, das ein N-Atom und optional ein zusätzliches Heteroatom enthält, das aus O, S und N ausgewählt ist, wobei das S-Atom substituiert sein kann, um S(=O) oder $S(=O)_2$ auszubilden;
oder $R^{4a}$ und $R^{4b}$ zusammengenommen werden, um zusammen mit dem N-Atom, an das sie gebunden sind, einen 5- bis 6-gliedrigen monocyclischen Ring auszubilden, der ein N-Atom und optional ein oder zwei zusätzliche Heteroatome enthält, die jeweils unabhängig aus O, S und N ausgewählt sind, wobei das S-Atom substituiert sein kann, um S(=O) oder $S(=O)_2$ auszubilden;
$R^5$ für Wasserstoff, $C_{1-4}$-Alkyl, $Het^{1a}$, -$C_{1-4}$-Alkyl-$Het^{1b}$ steht, oder
$C_{1-4}$-Alkyl, das mit einem oder zwei -O-$C_{1-4}$-Alkyl substituiert ist;
$Het^{1a}$ für ein C-verknüpftes 4- bis 7-gliedriges monocyclisches vollständig gesättigtes Heterocyclyl steht, das ein oder zwei Heteroatome enthält, die aus O, S und N ausgewählt sind; wobei das S-Atom substituiert sein kann, um S(=O) oder $S(=O)_2$ auszubilden;
$Het^{1b}$ für ein 4- bis 7-gliedriges monocyclisches vollständig gesättigtes Heterocyclyl steht, das ein oder zwei Heteroatome enthält, die aus O, S und N ausgewählt sind, wobei das S-Atom substituiert sein kann, um S(=O) oder $S(=O)_2$ auszubilden.

**3.** Verbindung nach Anspruch 1 oder 2, wobei Y für $CH_2$ steht.

**4.** Verbindung nach Anspruch 1 oder 2, wobei Y für O steht.

**5.** Verbindung nach einem der Ansprüche 1 bis 4, wobei n für 2 steht.

**6.** Pharmazeutische Zusammensetzung, umfassend eine Verbindung nach einem der Ansprüche 1 bis 5 und eine pharmazeutisch unbedenkliche Trägersubstanz oder ein pharmazeutisch unbedenkliches Verdünnungsmittel.

**7.** Verfahren zum Herstellen einer pharmazeutischen Zusammensetzung wie in Anspruch 6 definiert, umfassend ein Mischen eines pharmazeutisch unbedenklichen Trägers mit einer therapeutisch wirksamen Menge einer Verbindung nach einem der Ansprüche 1 bis 5.

**8.** Verbindung nach einem der Ansprüche 1 bis 5 oder eine pharmazeutische Zusammensetzung nach Anspruch 6 zur Verwendung als ein Medikament.

**9.** Verbindung nach einem der Ansprüche 1 bis 5 oder eine pharmazeutische Zusammensetzung nach Anspruch 6 zur Verwendung bei einer Vorbeugung oder einer Behandlung von Krebs.

**10.** Verbindung oder pharmazeutische Zusammensetzung zur Verwendung nach Anspruch 9, wobei der Krebs aus Prostata, Lunge, Bauchspeicheldrüse, Brust, Eierstock, Gebärmutterhals, Melanom, chronischer lymphatischer Leukämie (CLL) vom B-Zell-Typ, akuter myeloischer Leukämie (AML) und akuter lymphoblastischer Leukämie (ALL) ausgewählt ist.

## Revendications

**1.** Composé de Formule (I)

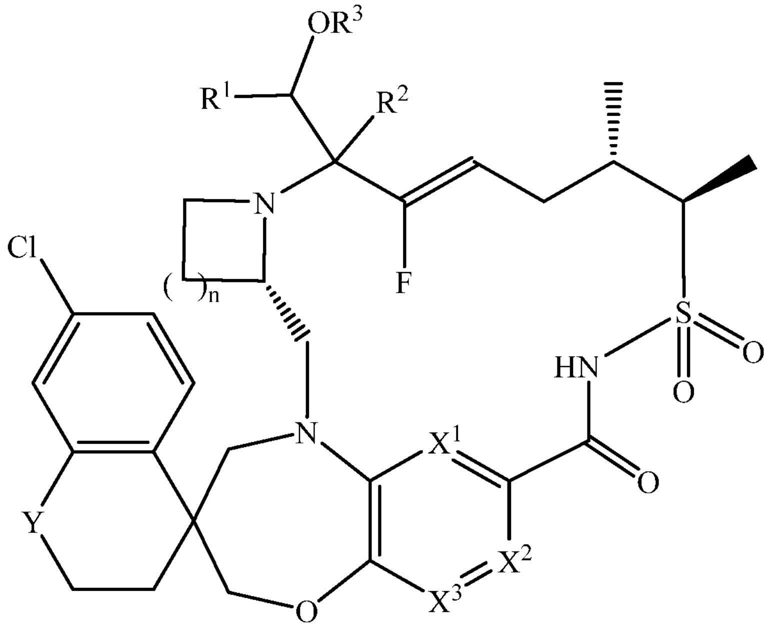

(I),

dans lequel

$R^1$ représente $C_{1-4}$alkyle, -$C_{1-4}$alkyl-$NR^{4a}R^{4}$b, ou -$C_{1-4}$alkyl-$OR^5$ ;
$R^2$ représente hydrogène, méthyle, -$CH_2$-$NR^{4c}R^{4d}$, ou -$CH_2$-$OR^6$ ; et
$R^3$ représente hydrogène, $C_{1-4}$alkyle, ou -$C_{2-4}$alkyl-O-$C_{1-4}$alkyle ;
ou
$R^1$ et $R^3$ sont pris ensemble pour former, conjointement avec les atomes auxquels ils sont attachés, un hétérocyclyle entièrement saturé monocyclique à 4 à 7 chaînons contenant un atome O et facultativement un hétéroatome supplémentaire choisi parmi O, S, et N, dans lequel l'atome S pourrait être substitué pour former S(=O) ou $S(=O)_2$, et dans lequel ledit hétérocyclyle est facultativement substitué par un, deux ou trois substituants chacun choisis indépendamment parmi $C_{1-4}$alkyle, -O-$C_{1-4}$alkyle, et -OH ;
et
$R^2$ représente hydrogène, méthyle, -$CH_2$-$NR^{4c}R^{4d}$, ou -$CH_2$-$OR^6$ ;
ou
$R^2$ et $R^3$ sont pris ensemble pour former, conjointement avec les atomes auxquels ils sont attachés, un hétérocyclyle entièrement saturé monocyclique à 4 à 7 chaînons contenant un atome O et facultativement un hétéroatome supplémentaire choisi parmi O, S, et N, dans lequel l'atome S pourrait être substitué pour former S(=O) ou $S(=O)_2$, et dans lequel ledit hétérocyclyle est facultativement substitué par un, deux ou trois substituants chacun choisis indépendamment parmi $C_{1-4}$alkyle, -O-$C_{1-4}$alkyle, et -OH ;
et
$R^1$ représente hydrogène, $C_{1-4}$alkyle, -$C_{1-4}$alkyl-$NR^{4a}R^{4b}$, ou -$C_{1-4}$alkyl-$OR^5$ ;
ou
$R^1$ et $R^2$ sont pris ensemble pour former, conjointement avec les atomes auxquels ils sont attachés, un hétérocyclyle entièrement saturé monocyclique à 4 à 7 chaînons contenant un atome O et facultativement un hétéroatome supplémentaire choisi parmi O, S, et N, dans lequel l'atome S pourrait être substitué pour former S(=O) ou $S(=O)_2$, et dans lequel ledit hétérocyclyle est facultativement substitué par un, deux ou trois substituants choisis chacun indépendamment parmi $C_{1-4}$alkyle, -O-$C_{1-4}$alkyle, et -OH ;
et
$R^3$ représente hydrogène, $C_{1-4}$alkyle, ou -$C_{2-4}$alkyl-O-$C_{1-4}$alkyle ;
$R^{4a}$ et $R^{4b}$ sont chacun choisis indépendamment dans le groupe constitué de $C_{1-4}$alkyle, -$C_{1-4}$alkyl-$Het^{1b}$, -$C_{2-4}$alkyl-O-$C_{1-4}$alkyle, et -$C_{2-4}$alkyl-O-$C_{1-4}$alkyl-$OC_{1-4}$alkyle ;
ou $R^{4a}$ et $R^{4b}$ sont pris ensemble pour former, conjointement avec l'atome N auquel ils sont attachés, un hétérocyclyle entièrement saturé monocyclique à 4 à 7 chaînons contenant un atome N et facultativement un hétéroatome supplémentaire choisi parmi O, S, et N, dans lequel ledit atome S pourrait être substitué pour former S(=O) ou $S(=O)_2$ ;
ou $R^{4a}$ et $R^{4b}$ sont pris ensemble pour former, conjointement avec l'atome N auquel ils sont attachés, un cycle

aromatique monocyclique à 5 à 6 chaînons contenant au moins un atome N et facultativement un ou deux hétéroatomes supplémentaires chacun choisis indépendamment parmi O, S, et N, dans lequel ledit atome S pourrait être substitué pour former S(=O) ou $S(=O)_2$ ;

$R^{4c}$ et $R^{4d}$ sont chacun choisis indépendamment dans le groupe constitué de $C_{1-4}$alkyle et -$C_{2-4}$alkyl-O-$C_{1-4}$alkyle ;

ou $R^{4c}$ et $R^{4d}$ sont pris ensemble pour former, conjointement avec l'atome N auquel ils sont attachés, un hétérocyclyle entièrement saturé monocyclique à 4 à 7 chaînons contenant un atome N et facultativement un hétéroatome supplémentaire choisi parmi O, S, et N, dans lequel ledit atome S pourrait être substitué pour former S(=O) ou $S(=O)_2$ ;

ou $R^{4c}$ et $R^{4d}$ sont pris ensemble pour former, conjointement avec l'atome N auquel ils sont attachés, un cycle aromatique monocyclique à 5 à 6 chaînons contenant au moins un atome N et facultativement un ou deux hétéroatomes supplémentaires chacun choisis indépendamment parmi O, S, et N, dans lequel ledit atome S pourrait être substitué pour former S(=O) ou $S(=O)_2$ ;

$R^5$ représente hydrogène, $C_{1-4}$alkyle, $Het^{1a}$, -$C_{2-4}$alkyl-$NR^{7a}R^{7b}$, -$C_{1-4}$alkyl-$Het^{1b}$, ou $C_{1-4}$alkyle substitué par un ou deux -O-$C_{1-4}$alkyle ;

$R^{7a}$ et $R^{7b}$ sont chacun choisis indépendamment dans le groupe constitué d'hydrogène et $C_{1-4}$alkyle ;

$Het^{1a}$ représente un hétérocyclyle totalement saturé monocyclique à 4 à 7 chaînons contenant un ou deux hétéroatomes chacun choisis indépendamment parmi O, S, et N, dans lequel ledit atome S pourrait être substitué pour former S(=O) ou $S(=O)_2$ ;

$Het^{1b}$ représente un hétérocyclyle totalement saturé monocyclique à 4 à 7 chaînons contenant un ou deux hétéroatomes choisis parmi O, S, et N, dans lequel ledit atome S pourrait être substitué pour former S(=O) ou $S(=O)_2$ ;

$R^6$ représente hydrogène ou $C_{1-4}$alkyle ;

n vaut 1 ou 2 ;

Y représente O ou $CH_2$ ;

$X^1$ représente CH ;

$X^2$ représente CH ;

$X^3$ représente CH ;

ou un sel pharmaceutiquement acceptable, ou un solvate de celui-ci.

**2.** Composé selon la revendication 1, dans lequel

$R^1$ représente $C_{1-4}$alkyle, -$C_{1-4}$alkyl-$NR^{4a}R^{4b}$, ou -$C_{1-4}$alkyl-$OR^5$ ;

$R^2$ représente hydrogène, ou -$CH_2OR^6$ ; et

$R^3$ représente hydrogène, ou $C_{1-4}$alkyle ;

ou

$R^1$ et $R^3$ sont pris ensemble pour former, conjointement avec les atomes auxquels ils sont attachés, un hétérocyclyle entièrement saturé monocyclique à 4 à 7 chaînons contenant un atome O et facultativement un hétéroatome supplémentaire choisi parmi O, S, et N, dans lequel ledit atome S pourrait être substitué pour former S(=O) ou $S(=O)_2$ ; et dans lequel ledit hétérocyclyle est facultativement substitué par un, deux ou trois substituants $C_{1-4}$alkyle ;

et

$R^2$ représente -$CH_2$-$OR^6$ ;

ou

$R^2$ et $R^3$ sont pris ensemble pour former, conjointement avec les atomes auxquels ils sont attachés, un hétérocyclyle entièrement saturé monocyclique à 4 à 7 chaînons contenant un atome O et facultativement un hétéroatome supplémentaire choisi parmi O, S, et N, dans lequel l'atome S pourrait être substitué pour former S(=O) ou $S(=O)_2$, et dans lequel ledit hétérocyclyle est facultativement substitué par un, deux ou trois substituants $C_{1-4}$alkyle ;

et

$R^1$ représente hydrogène ;

ou

$R^1$ et $R^2$ sont pris ensemble pour former, conjointement avec les atomes N auxquels ils sont attachés ; un hétérocyclyle entièrement saturé monocyclique à 4 à 7 chaînons contenant un atome O et facultativement un hétéroatome supplémentaire choisi parmi O, S, et N, dans lequel ledit atome S pourrait être substitué pour former S(=O) ou $S(=O)_2$ ; et dans lequel ledit atome S pourrait être facultativement substitué par un, deux ou trois substituants $C_{1-4}$alkyle ;

et

$R^3$ représente hydrogène ou $C_{1-4}$alkyle ;
$R^{4a}$ et $R^{4b}$ sont chacun choisis indépendamment dans le groupe constitué de $C_{1-4}$alkyle, -$C_{1-4}$alkyl-$Het^{1b}$, -$C_{2-4}$alkyl-O-$C_{1-4}$alkyle, et -$C_{2-4}$alkyl-O-$C_{1-4}$alkyl-O$C_{1-4}$alkyle ;
ou $R^{4a}$ et $R^{4b}$ sont pris ensemble pour former, conjointement avec l'atome N auquel ils sont attachés, un hétérocyclyle entièrement saturé monocyclique à 4 à 7 chaînons contenant un atome N et facultativement un hétéroatome supplémentaire choisi parmi O, S, et N, dans lequel ledit atome S pourrait être substitué pour former S(=O) ou $S(=O)_2$ ;
ou $R^{4a}$ et $R^{4b}$ sont pris ensemble pour former, conjointement avec l'atome N auquel ils sont attachés, un cycle aromatique monocyclique à 5 à 6 chaînons contenant au moins un atome N et facultativement un ou deux hétéroatomes supplémentaires chacun choisis indépendamment parmi O, S, et N, dans lequel ledit atome S pourrait être substitué pour former S(=O) ou $S(=O)_2$ ;
$R^5$ représente hydrogène, $C_{1-4}$alkyle, $Het^{1a}$, -$C_{1-4}$alkyl-$Het^{1b}$, ou
$C_{1-4}$alkyle substitué par un ou deux -O-$C_{1-4}$alkyle ;
$Het^{1a}$ représente un hétérocyclyle totalement saturé monocyclique à 4 à 7 chaînons contenant un ou deux hétéroatomes chacun choisis indépendamment parmi O, S, et N, dans lequel ledit atome S pourrait être substitué pour former S(=O) ou $S(=O)_2$ ;
$Het^{1b}$ représente un hétérocyclyle totalement saturé monocyclique à 4 à 7 chaînons contenant un ou deux hétéroatomes choisis parmi O, S, et N, dans lequel ledit atome S pourrait être substitué pour former S(=O) ou $S(=O)_2$.

**3.** Composé selon la revendication 1 ou 2, dans lequel Y représente $CH_2$.

**4.** Composé selon la revendication 1 ou 2, dans lequel Y représente O.

**5.** Composé selon l'une quelconque des revendications 1 à 4, dans lequel n représente 2.

**6.** Composition pharmaceutique comprenant un composé selon l'une quelconque des revendications 1 à 5 et un support ou diluant pharmaceutiquement acceptable.

**7.** Procédé permettant de préparer une composition pharmaceutique selon la revendication 6 comprenant le mélange d'un support pharmaceutiquement acceptable avec une quantité thérapeutiquement efficace d'un composé selon l'une quelconque des revendications 1 à 5.

**8.** Composé selon l'une quelconque des revendications 1 à 5 ou composition pharmaceutique selon la revendication 6 pour une utilisation en tant que médicament.

**9.** Composé selon l'une quelconque des revendications 1 à 5 ou composition pharmaceutique selon la revendication 6 pour une utilisation dans la prévention ou le traitement d'un cancer.

**10.** Composé ou composition pharmaceutique pour une utilisation selon la revendication 9, dans lequel le cancer est choisi parmi cancer de la prostate, du poumon, du pancréas, du sein, de l'ovaire, du col de l'utérus, mélanome, leucémie lymphoïde chronique à lymphocytes B (LLC), leucémie myéloïde aiguë (LMA), et leucémie lymphoblastique aiguë (LLA).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019046150 A
- WO 2019173181 A
- WO 2016033486 A
- WO 2019036575 A
- WO 2017147410 A
- WO 2018183418 A
- WO 2019222112 A
- WO 2020097577 A
- WO 2021211922 A


### Non-patent literature cited in the description


- **CHENG et al.** *eLife*, 2016, vol. 5, e17755
- **JULIAN et al.** *Cell Death and Differentiation*, 2017, vol. 24, 1380-1389
- **HANAHAN** ; **WEINBERG**. *Cell*, 2011, 1-44
- **BEROUKHIM et al.** *Nature*, 2010, vol. 463 (7283), 899-905
- **YECIES et al.** *Blood*, 2010, vol. 115 (16), 3304-3313
- **ROBERTS et al.** *NEJM*, 2016, vol. 374, 311-322
- **KOTSCHY et al.** *Nature*, 2016, vol. 538, 477-486
- **MERINO et al.** *Sci. Transl. Med*, 2017, vol. 9
- **CHEN et al.** *JCI*, 2018, vol. 128 (1), 500-516
- **WANG et al.** *Genes and Dev*, 2013, vol. 27, 1351-1364
- **STEIMER et al.** *Blood*, 2009, vol. 113, 2805-2815
- **CHARRON, CARLIE L. et al.** *Tetrahedron Lett.*, 2016, vol. 57 (37), 4119-4127
- **AUSTIN R. et al.** *Cancer Letters*, 2016
- *CHEMICAL ABSTRACTS*, 1638587-10-6
- **T. W. GREENE** ; **P. G. M. WUTS**. Protective Groups in Organic Synthesis. Wiley, 2007
- Part 8 : Pharmaceutical preparations and their Manufacture. **GENNARO et al.** Remington's Pharmaceutical Sciences. Mack Publishing Company, 1990
- *CHEMICAL ABSTRACTS*, 87413-09-0
- *CHEMICAL ABSTRACTS*, 161511-85-9
- *CHEMICAL ABSTRACTS*, 125225-80-1
- *CHEMICAL ABSTRACTS*, 131242-36-9
- *CHEMICAL ABSTRACTS*, 10213-10-2
- *CHEMICAL ABSTRACTS*, 157171-33-1
- *CHEMICAL ABSTRACTS*, 557-20-0
- *CHEMICAL ABSTRACTS*, 353-54-8
- *CHEMICAL ABSTRACTS*, 603-35-0
- *CHEMICAL ABSTRACTS*, 73183-34-3
- *CHEMICAL ABSTRACTS*, 95464-05-4
- *CHEMICAL ABSTRACTS*, 1883726-85-9
- *CHEMICAL ABSTRACTS*, 1883726-74-6
- *CHEMICAL ABSTRACTS*, 72287-26-4
- *CHEMICAL ABSTRACTS*, 13269-77-7
- *CHEMICAL ABSTRACTS*, 39389-20-3
- *CHEMICAL ABSTRACTS*, 2081-44-9
- *CHEMICAL ABSTRACTS*, 111-95-5
- *CHEMICAL ABSTRACTS*, 533-50-6
- *CHEMICAL ABSTRACTS*, 18162-48-6
- *CHEMICAL ABSTRACTS*, 288-32-4
- *CHEMICAL ABSTRACTS*, 96-26-4
- *CHEMICAL ABSTRACTS*, 161265-03-8
- *CHEMICAL ABSTRACTS*, 125552-89-8
- *CHEMICAL ABSTRACTS*, 7646-69-7